# FASCICULE DE BREVET EUROPEEN

(11) **EP 2 235 016 B1**
(45) Date de publication et mention de la délivrance du brevet: **18.09.2013**
(21) Numéro de dépôt: 09720539.7
(22) Date de dépôt: 20.01.2009
(51) Int. Cl.: C07D 471/04, A61K 31/437, A61P 29/00, A61P 11/00, A61P 3/00

(54) **DÉRIVÉS DE CARBOXAMIDES AZABICYCLIQUES, LEUR PRÉPARATION ET LEUR APPLICATION EN THÉRAPEUTIQUE**
AZABIZYKLISCHE CARBOXAMID-DERIVATE SOWIE IHRE HERSTELLUNG UND THERAPEUTISCHE VERWENDUNG
AZABICYCLIC CARBOXAMIDE DERIVATIVES, PREPARATION THEREOF AND THERAPEUTIC USE THEREOF

(30) Priorité: 22.01.2008 FR 0800310
(43) Date de publication de la demande: 06.10.2010
(73) Titulaire: SANOFI, 75008 Paris (FR)
(72) Inventeur: DUBOIS. Laurent, F-75013 Paris (FR); EVANNO, Yannick, F-75013 Paris (FR); LECLERC, Odile, F-75013 Paris (FR); MALANDA, André, F-75013 Paris (FR)
(74) Mandataire: Le Coupanec, Pascale A.M.P.
(86) Numéro de dépôt international: PCT/FR2009/000053
(87) Numéro de publication internationale: WO 2009/112679

(56) Documents cités:
- WO-A-2007/088277

## Description

Les documents WO 2007/088277, WO2006/024776, WO2006/072736, WO2007/010144, WO2007/010138 décrivent des dérivés de carboxamides bicycliques, présentant une activité antagoniste ou agoniste *in vitro* et *in vivo* pour les récepteurs de type TRPV1 (ou VR1).

Il existe toujours un besoin de trouver de nouveaux ligands pour les récepteurs de type TRPV1, présentant des améliorations en termes d'activité fonctionnelle, de profil métabolique et / ou de profil de sécurité.

La présente invention répond à ce besoin en fournissant des dérivés de carboxamides azabicycliques qui présentent une activité antagoniste ou agoniste *in vitro* et *in vivo* pour les récepteurs de type TRPV1 (ou VR1).

Un premier objet de l'invention concerne les composés répondant à la formule générale (I) ci-après.

Un autre objet de l'invention concerne des procédés de préparation des composés de formule générale (I).

Un autre objet de l'invention concerne l'utilisation des composés de formule générale (I) notamment dans des médicaments ou dans des compositions pharmaceutiques.

Les composés de l'invention répondent à la formule générale (I) : 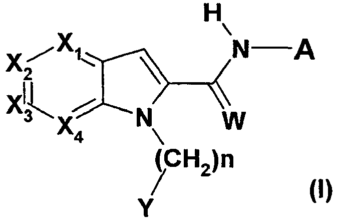 dans laquelle :
X₁, X₂, X₃, et X₄ représentent, indépendamment l'un de l'autre, un atome d'azote ou un groupe C-R₁ ;
   étant entendu que quand l'un des X₁, X₂, X₃, et X₄ représente un atome d'azote, les autres correspondent à un groupe C-R₁ ;
W représente un atome d'oxygène ou de soufre ;
n est égal à 0, 1, 2 ou 3 ;
Y représente un aryle ou un hétéroaryle éventuellement substitué par un ou plusieurs groupes choisis parmi un atome d'halogène, un groupe C₁-C₆-alkyle, C₃-C₇-cycloalkyle, C₃-C₇-cycloalkyl-C₁-C₃-alkylène, C₁-C₆-fluoroalkyle, hydroxy, C₁-C₆-alcoxy, C₃-C₇-cycloalkyloxy, C₃-C₇-cyloalkyl-C₁-C₆-alkylène-O-, C₁-C₆-fluoroalcoxy, cyano, C(O) NR₃R₄, nitro, NR₃R₄, C₁-C₆-thioalkyle, thiol, -S(O)-C₁-C₆-alkyle, -S(O)₂-C₁-C₆-alkyle, SO₂NR₃R₄, NR₅C(O)R₆, NR₅SO₂R₇, C(O)NR₃R₄, OC(O)NR₃R₄, -Si-(C₁-C₆-alkyl)₃, -SF₅, aryle-C₁-C₅-alkylène ou aryle, hétéroaryl-C₁-C₅-alkylène ou hétéroaryle ; les groupes C₁-C₆-alkyle, C₃-C₇-cycloalkyle, C₃-C₇-cycloalkyl-C₁-C₃-alkylène, C₁-C₆-fluoroalkyle, C₁-C₆-alcoxy, C₃-C₇-cycloalkyloxy, C₃-C₇-cycloalkyl-C₁-C₆-alkylène-O- pouvant être substitués par un groupe hydroxy, C₁-C₆-alcoxy ou NR₃R₄ ; les groupes aryle et hétéroaryle étant éventuellement substitués par un ou plusieurs substituants R₈ identiques ou différents l'un de l'autre ;
A représente un hétéroaryle bicyclique de formule : 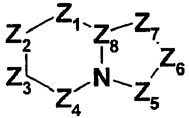 où
   Z₁, Z₂, Z₃, et Z₄ représentent, indépendamment l'un de l'autre, un atome de carbone, un atome d'azote ou un groupe C-R₂ₐ ;
   Z₅, Z₆ et Z₇ représentent, indépendamment l'un de l'autre, un atome d'azote ou un groupe C-R_{2b} ;
   Z₈ représente un atome de carbone ;
   trois, au maximum, des Z₁, Z₂, Z₃, Z₄, Z₅, Z₆ et Z₇ représentant un atome d'azote ;
   l'un des Z₁, Z₂, Z₃ et Z₄, correspondant à un atome de carbone, étant lié à l'atome d'azote de l'amide ou du thioamide de formule (I) ;
R₁ est choisi parmi un atome d'hydrogène, un atome d'halogène, C₁-C₆-alkyle, C₃-C₇-cycloalkyle, C₃-C₇-cycloalkyl-C₁-C₃-alkylène, C₁-C₆-fluoroalkyle, aryloxy-C₁-C₆-alkyle, hétéroaryloxy-C₁-C₆-alkyle, aryl-C₁-C₃-alkylènoxy-C₁-C₆-alkyle, hétéroaryl-C₁-C₃-alkylènoxy-C₁-C₆-alkyle, arylthio-C₁-C₆-alkyle, hétéroarylthio-C₁-C₆-alkyle, aryl-C₁-C₃-alkylène-thio-C₁-C₆-alkyle, hétéroaryl-C₁-C₃-alkylène-thio-C₁-C₆-alkyle, C₁-C₆-alcoxy, C₃-C₇-cycloalkyloxy, C₃-C₇-cycloalkyl-C₁-C₃-alkylènoxy, C₁-C₆-fluoroalcoxy, cyano, C(O)NR₃R₄, nitro, NR₃R₄, C₁-C₆-thioalkyle, C₃-C₇-cycloalkylthio, C₃-C₇-cycloalkyl-C₁-C₃-alkylène-thio, -S(O)-C₁-C₆-alkyle, -S(O)-C₃-C₇-cycloalkyle, -S(O)-C₁-C₃-alkylène-C₃-C₇-cycloalkyle, C₁-C₆-alkyle-S(O)₂-, C₁-C₆-fluoroalkyle-S(O)₂-, C₃-C₇-cycloalkyl-S(O)₂-, C₃-C₇-cycloalkyl-C₁-C₃-alkylène-S(O)₂-, SO₂NR₃R₄, (C₁-C₆-alkyl)₃-Si-, -SF₅, NR₅C(O)R₆, NR₅SO₂R₇, C(O)NR₃R₄, OC(O)NR₃R₄, aryle, hétéroaryle, aryl-C₁-C₅-alkylène, hétéroaryl-C₁-C₅-alkylène, aryloxy, arylthio, hétéroaryloxy ou hétéroarylthio ; les groupes hétéroaryle ou aryle étant éventuellement substitués par un ou plusieurs substituants R₈, identiques ou différents l'un de l'autre ;
R₂ₐ représente un atome d'hydrogène, un atome d'halogène, un groupe C₁-C₆-alkyle, C₃-C₇-cycloalkyle, C₃-C₇-cycloalkyl-C₁-C₃-alkylène, C₁-C₆-fluoroalkyle, C₁-C₆-alcoxy, C₃-C₇-cycloalkyloxy, C₃-C₇cycloalkyl-C₁-C₃-alkylène-O-, hydroxy, thiol, C₁-C₆-fluoroalcoxy ;
R_{2b} représente un atome d'hydrogène, un atome d'halogène, un groupe C₁-C₆-alkyle, C₃-C₇-cycloalkyle, C₃-C₇-cycloalkyl-C₁-C₃-alkylène, C₁-C₆-fluoroalkyle, C₁-C₆-alcoxy, hydroxy, thiol, oxo, thio, C₃-C₇-cycloalkyloxy, C₁-C₆-fluoroalcoxy, C₃-C₇-cycloalkyl-C₁-C₃-alkylénoxy, C₁-C₆-alcoxy-C₁-C₃-alkylène, C₃-C₇-cycloalkyloxy-C₁-C₃-alkylène, C₃-C₇-cycloalkyl-C₁-C₃-alkylénoxy-C₁-C₃-alkylène, C₁-C₆-alkyl-C(O)-O-C₁-C₃-alkylène, C₁-C₆-alkyl-C(O)-O-, C₃-C₇-cycloalkyl-C(O)-O-C₁-C₃-alkylène, C₃-C₇-cycloalkyl-C(O)-O-, C₁-C₆-fluoroalkyl-C(O)-O-C₁-C₃-alkylène, C₁-C₆-fluoroalkyl-C(O)-O-, C(O)NR₃R₄, C(O)O-C₁-C₆-alkyle, cyano, CHO, CO₂H, -C(O)-C₁-C₈-alkyle, -C(O)-C₃-C₇-cycloalkyle, phényle ou thiényle ; les groupes C₁-C₆-alkyle, C₃-C₇-cycloalkyle, C₃-C₇-cycloalkyl-C₁-C₃-alkylène, C₁-C₆-fluoroalkyle, C₁-C₆-alcoxy, C₃-C₇-cycloalkyloxy, C₁-C₆-fluoroalcoxy, C₃-C₇-cycloalkyl-C₁-C₃-alkylénoxy, C₁-C₆-alcoxy-C₁-C₃-alkylène, C₃-C₇-cycloalkyloxy-C₁-C₃-alkylène, C₃-C₇-cycloalkyl-C₁-C₃-alkylénoxy-C₁-C₃-alkylène pouvant être substitués par un groupe hydroxy, C₁-C₆-alcoxy ou NR₃R₄ ;
R₃ et R₄ représentent, indépendamment l'un de l'autre, un atome d'hydrogène ou un groupe C₁-C₆-alkyle, C₃-C₇-cycloalkyle, C₃-C₇-cycloalkyl-C₁-C₃-alkylène, aryle-C₁-C₅-alkylène ou aryle, ou R₃ et R₄ forment ensemble, avec l'atome d'azote qui les porte, un groupe azétidine, pyrrolidine, pipéridine, azépine, morpholine, thiomorpholine, pipérazine, homopipérazine; le groupe NR₃R₄ étant éventuellement substitué par un groupe C₁-C₆-alkyle, C₃-C₇-cycloalkyle, C₃-C₇-cycloalkyl-C₁-C₃-alkylène, aryle-C₁-C₆-alkylène, aryle, hétéroaryle, aryle-S(O)₂-, C₁-C₆-alkyle-S(O)₂-, C₁-C₆-fluoroalkyle-S(O)₂, C₃-C₇-cycloalkyl-S(O)₂-, C₃-C₇-cycloalkyl-C₁-C₃-alkylène-S(O)₂-, aryle-C(O)-, C₁-C₆-alkyle-C(O)-, C₃-C₇-cycloalkyl-C(O)-, C₃-C₇-cycloalkyl-C₁-C₃-alkyléne-C(O)-, C₁-C₆-fluoroalkyle-C(O)-, hydroxy, C₁-C₆-alkyloxy, C₃-C₇-cycloalkyloxy, C₃-C₇-cycloalkyl-C₁-C₃-alkylénoxy, C₁-C₆-fluoroalkyle, aryloxy-C₁-C₆-alkylène, aryloxy, hétéroaryloxy-C₁-C₆-alkylène, hétéroaryloxy ;
R₅ et R₆ représentent, indépendamment l'un de l'autre, un atome d'hydrogène, un groupe C₁-C₆-alkyle, C₃-C₇-cycloalkyle, C₃-C₇-cycloalkyl-C₁-C₃-alkylène, aryle-C₁-C₆-alkylène ou aryle ; le groupe aryle étant éventuellement substitué par un ou plusieurs substituants choisis parmi un atome d'halogène, un groupe C₁-C₆-alkyle, C₃-C₇-cycloalkyle, C₃-C₇-cycloalkyl-C₁-C₃-alkylène, C₁-C₆-fluoroalkyle, C₁-C₆-alcoxy, C₃-C₇-cycloalkyloxy, C₃-C₇-cycloalkyl-C₁-C₃-alkylénoxy, C₁-C₆-fluoroalcoxy, nitro ou cyano ; ou R₅ et R₆ forment ensemble un lactame de 4 à 7 chaînons comprenant l'atome d'azote et le groupe C(O) qui les portent ;
R₇ représente un groupe C₁-C₆-alkyle, C₃-C₇-cycloalkyle, C₃-C₇-cycloalkyl-C₁-C₃-alkylène, aryle-C₁-C₆-alkylène ou aryle ; le groupe aryle étant éventuellement substitué par un ou plusieurs substituants choisis parmi un atome d'halogène, un groupe C₁-C₆-alkyle, C₃-C₇-cycloalkyle, C₃-C₇-cycloalkyl-C₁-C₃-alkylène, C₁-C₆-fluoroalkyle, C₁-C₆-alcoxy, C₃-C₇-cycloalkyloxy, C₃-C₇-cycloalkyl-C₁-C₃-alkylénoxy, C₁-C₆-fluoroalcoxy, nitro ou cyano ;
   ou R₅ et R₇ forment ensemble un sultame de 4 à 7 chaînons comprenant l'atome d'azote et le groupe S(O)₂ qui les portent ;
R₈ représente un atome d'halogène, un groupe C₁-C₆-alkyle, C₃-C₇-cycloalkyle, C₃-C₇-cycloalkyl-C₁-C₃-alkylène, C₁-C₆-fluoroalkyle, C₁-C₆-alcoxy, C₃-C₇-cycloalkyloxy, C₃-C₇-cycloalkyl-C₁-C₃-alkylénoxy, C₁-C₆-fluoroalcoxy, nitro, cyano, NR₃R₄, -C(O)-C₁-C₆-alkyle, -C(O)-C₃-C₇-cycloalkyle ; les groupes C₁-C₆-alkyle, C₃-C₇-cycloalkyle, C₃-C₇-cycloalkyl-C₁-C₃-alkylène, C₁-C₆-fluoroalkyle, C₁-C₆-alcoxy, C₃-C₇-cycloalkyloxy, C₃-C₇-cycloalkyl-C₁-C₃-alkylénoxy, pouvant être substitués par un groupe OH, C₁-C₆-alcoxy ou NR₃R₄.

Dans les composés de formule générale (I) :
- le ou les atomes de soufre peuvent être sous forme oxydée (S(O) ou S(O)₂) ;
- le ou les atomes d'azote peuvent éventuellement être sous forme oxydée (N-oxyde).

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.
Ces sels peuvent être préparés avec des acides pharmaceutiquement acceptables, mais les sels d'autres acides utiles, par exemple, pour la purification ou l'isolement des composés de formule (I) font également partie de l'invention.

Les composés de formule (I) peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Dans le cadre de la présente invention, on entend par :
- un atome d'halogène : un fluor, un chlore, un brome ou un iode ;
- Cₜ-C_{z} : une chaîne carbonée pouvant avoir de t à z atomes de carbone où t et z peuvent prendre les valeurs de 1 à 7 ; par exemple C₁-C₃ est une chaîne carbonée qui peut avoir de 1 à 3 atomes de carbone ;
- un alkyle : un groupe aliphatique saturé linéaire ou ramifié. A titre d'exemples, on peut citer les groupes méthyle, éthyle, propyle, isopropyle, butyle, isobutyle, tertiobutyle, pentyle, etc ;
- un alkylène : un groupe alkyle divalent saturé, linéaire ou ramifié, par exemple un groupe C₁₋₃-alkylène représente une chaîne carbonée divalente de 1 à 3 atomes de carbone, linéaire ou ramifiée, plus particulièrement un méthylène, éthylène, 1-méthyléthylène, propylène ;

un cycloalkyle : un groupe alkyle cyclique, saturé ou partiellement insaturé. A titre d'exemples, on peut citer les groupes cyclopropyle, cyclobutyle, cyclopentyle, cyclohexyle, etc ;
- un cycloalkyloxy : un radical -O-cycloalkyle où le groupe cycloalkyle est tel que précédemment défini ;
- un fluoroalkyle : un groupe alkyle dont un ou plusieurs atomes d'hydrogène ont été substitués par un atome de fluor ;
- un alcoxy : un radical -O-alkyle où le groupe alkyle est tel que précédemment défini ;
- un fluoroalcoxy : un groupe alcoxy dont un ou plusieurs atomes d'hydrogène ont été substitués par un atome de fluor ;
- un thioalkyle ou alkyle-thio : un radical -S-alkyle où le groupe alkyle est tel que précédemment défini ;
- un aryle : un groupe aromatique mono- ou bicyclique comprenant entre 6 et 10 atomes de carbones. A titre d'exemples de groupe aryle, on peut citer les groupes phényle ou naphthyle ;
- un hétéroaryle : un groupe mono- ou bicyclique aromatique de 5 à 12 chaînons contenant de 1 à 5 hétéroatomes choisis parmi O, S ou N.
A titre d'exemples d'hétéroaryle monocyclique, on peut citer les groupes imidazolyle, pyrazolyle, thiazolyle, oxazolyle, isothiazolyle, isoxazolyle, furanyle, thiophényle, oxadiazolyle, thiadiazolyle, triazolyle, tétrazolyle, pyridinyle, pyrazinyle, pyrimidinyle, pyridazinyle, triazinyle.
A titre d'exemples d'hétéroaryle bicyclique, on peut citer les groupes indolyle, isoindolyle, benzofuranyle, benzothiophényle, benzoxazolyle, benzimidazolyle, indazolyle, benzothiazolyle, isobenzofuranyle, isobenzothiazolyle, pyrrolo[2,3-*c*]pyridinyle, pyrrolo[2,3-*b*]pyridinyle, pyrrolo[3,2-*b*]pyridinyle, pyrrolo[3,2-*c*]pyridinyle, pyrrolo[1,2-*a*]pyridinyle, quinoléinyle, isoquinoléinyle, cinnolinyle, quinazolinyle, quinoxalinyle, pyrrolo[1,2-*a*]imidazolyle, imidazo[1,2-*a*]pyridinyle, imidazo[1,5-*a*]pyridinyle, imidazo[1,2-*b*]pyridazinyle, imidazo[1,2-*c*]pyrimidinyle, imidazo[1,2-*a*]pyrimidinyle, imidazo[1,5-*a*]pyrimidinyle, imidazo[1,5-*c*]pyrimidinyle, imidazo[1,2-*a*]pyrazinyle, imidazo[1,5-*a*]pyrazinyle, imidazo[1,5-*b*]pyridazinyle, imidazo[4,5-*b*]pyrazinyle, imidazo[4,5-*b*]pyridinyle, imidazo[4,5-*c*]pyridinyle, pyrazolo[1,5-*a*]pyridinyle, pyrazolo[1,5-*a*]pyrimidinyle, pyrazolo[1,5-*c*]pyrimidinyle, pyrazolo[1,5-*a*]pyrazinyle, pyrazolo[1,5-*b*]pyridazinyle, indolizinyle, pyrrolo[1,2-*b*]pyridazinyle, pyrrolo[1,2-*c*]pyrimidinyle, pyrrolo[1,2-*a*]pyrazinyle, pyrrolo[1,2-*a*]pyrimidinyle, [1,2,4]triazolo[4,3-*a*]pyridinyle, [1,2,4]triazolo[2,3-*a*]pyridinyle, [1,2,4]triazolo[1,5-*a*]pyridinyle, [1,2,3]triazolo[1,5-*a*]pyridinyle, [1,2,4]triazolo[1,5-*b*]pyridazinyle, triazolo[1,2-*a*]pyrimidinyle, triazolo[1,2-*c*]pyrimidinyle, triazolo[1,2-*a*]pyrazinyle, triazolo[1,2-*b*]pyridazinyle, [1,2,4]triazolo[1,5-*b*]pyridazinyle, [1,2,4]triazolo[1,5-*c*]pyrimidinyle, [1,2,4]triazolo[1,2-*a*]pyrazinyle, [1,2,4]triazolo[1,5-*a*]pyrimidinyle, [1,2,3]triazolo[1,5*-b*]pyridazinyle, [1,2,3]triazolo[1,5-*c*]pyrimidinyle, [1,2,3]triazolo[1,2-*a*]pyrazinyle, [1,2,3]triazolo[1,5-*a*]pyrimidinyle, imidazo[1,2-*d*][1,2,4]triazinyle, imidazo[2,1-*f*][1,2,4]triazinyle, imidazo[1,2-*b*][1,2,4]triazinyle, imidazo[1,2-*a*][1,3,5]triazinyle, imidazo[2,1-*c*][1,2,4]triazinyle, imidazo[5,1-*c*][1,2,4]triazinyle, imidazo[1,5-*a*][1,3,5]triazinyle, imidazo[1,5-*b*][1,2,4]triazinyle, imidazo[5,1-*f*][1,2,4]triazinyle, imidazo[1,5-*d*][1,2,4]triazinyle, imidazo[1,5-*c*][1,2,3]triazinyle, pyrazolo[5,1-*c*][1,2,4]triazinyle, pyrazolo[1,5-*a*][1,3,5]triazinyle, pyrazolo[1,5-*b*][1,2,4]triazinyle, pyrazolo[5,1-*f*][1,2,4]triazinyle, pyrazolo[1,5-*d*][1,2,4]triazinyle, pyrazolo[1,5-*c*][1,2,3]triazinyle ;
- « oxo » signifie « =O »;
- « thio » signifie « =S ».

Parmi les composés de formule générale (I) objets de l'invention, un premier sous-groupe de composés est constitué par les composés pour lesquels
X₁, X₂, X₃, et X₄ représentent, indépendamment l'un de l'autre un groupe C-R₁ ;
R₁ étant tel que défini dans la formule générale (I).

Parmi les composés de formule générale (I) objets de l'invention, un second sous-groupe de composés est constitué par les composés pour lesquels
X₁, X₂, X₃, et X₄ représentent, indépendamment l'un de l'autre, un atome d'azote ou un groupe C-R₁ ;
Etant entendu que l'un des X₁, X₂, X₃, et X₄ représente un atome d'azote, les autres correspondent à un groupe C-R₁ ;
R₁ étant tel que défini dans la formule générale (I).

Parmi les composés de formule générale (I) objets de l'invention, un troisième sous-groupe de composés est constitué par les composés pour lesquels
X₁, X₂, X₃ représentent un groupe C-R₁ ; X₄ représente un atome d'azote ;
R₁ étant tel que défini dans la formule générale (I).

Parmi les composés de formule générale (I) objets de l'invention, un quatrième sous-groupe de composés est constitué par les composés pour lesquels
R₁ est choisi parmi un atome d'hydrogène, un atome d'halogène, plus particulièrement un atome de fluor ou un groupe C₁-C₆-fluoroalkyle, plus particulièrement un groupe trifluorométhyle.

Parmi les composés de formule générale (I) objets de l'invention, un cinquième sous-groupe de composés est constitué par les composés pour lesquels n est égal à 1.

Parmi les composés de formule générale (I) objets de l'invention, un sixième sous-groupe Y représente un aryle, plus particulièrement un phényle, éventuellement substitué par un ou plusieurs atomes d'halogène, plus particulièrement de fluor.

Parmi les composés de formule générale (I) objets de l'invention, un septième sous-groupe de composés est constitué par les composés pour lesquels W représente un atome d'oxygène.

Parmi les composés de formule générale (I) objets de l'invention, un huitième sous-groupe de composés est constitué par les composés pour lesquels
A représente un hétéroaryle bicyclique de formule : 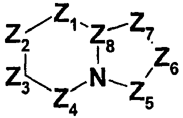 où
Z₁, Z₂, Z₃, et Z₄ représentent, indépendamment l'un de l'autre, un atome de carbone, un atome d'azote ;
Z₅, Z₆ et Z₇ représentent, indépendamment l'un de l'autre, un atome d'azote ou un groupe C-R_{2b} ;
Z₈ représente un atome de carbone ;
deux, au maximum, des Z₁, Z₂, Z₃, Z₄, Z₅, Z₆ et Z₇ représentant un atome d'azote ;
l'un des Z₁, Z₂, Z₃ et Z₄, correspondant à un atome de carbone, étant lié à l'atome d'azote de l'amide ou du thioamide de formule (I) ;
R_{2b} étant tel que défini dans la formule générale (I).

Parmi les composés de formule générale (I) objets de l'invention, neuvième sous-groupe de composés est constitué par les composés pour lesquels
A représente un hétéroaryle bicyclique de formule : 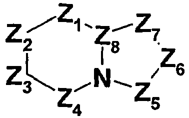 où
Z₁, Z₂, Z₃, et Z₄ représentent, indépendamment l'un de l'autre, un atome de carbone, un atome d'azote ;
Z₅, Z₆ et Z₇ représentent, indépendamment l'un de l'autre, un atome d'azote ou un groupe C-R_{2b} ;
Z₈ représente un atome de carbone ;
deux, au maximum, des Z₁, Z₂, Z₃, Z₄, Z₅, Z₆, et Z₇ représentant un atome d'azote ;
l'un des Z₁, Z₂, Z₃ et Z₄, correspondant à un atome de carbone, étant lié à l'atome d'azote de l'amide ou du thioamide de formule (I) ;
R_{2b} représente un atome d'hydrogène, un groupe C₁-C₆-alkyle, C(O)O-C₁-C₆-alkyle, phényle ou thiényle ; les groupes C₁-C₆-alkyle pouvant être substitués par un groupe hydroxy ou C₁-C₆-alcoxy.

Parmi les composés de formule générale (I) objets de l'invention, dixième sous-groupe de composés est constitué par les composés pour lesquels
A représente un hétéroaryle bicyclique de formule : 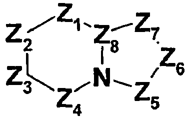 où
Z₁, Z₂, Z₃, et Z₄ représentent, indépendamment l'un de l'autre, un atome de carbone, un atome d'azote ;
Z₅, Z₆ et Z₇ représentent, indépendamment l'un de l'autre, un atome d'azote ou un groupe C-R_{2b} ;
Z₈ représente un atome de carbone ;
deux, au maximum, des Z₁, Z₂, Z₃, Z₄, Z₅, Z₆ et Z₇ représentant un atome d'azote ;
l'un des Z₁, Z₂, Z₃ et Z₄, correspondant à un atome de carbone, étant lié à l'atome d'azote de l'amide ou du thioamide de formule (I) ;
R_{2b} représente un atome d'hydrogène, un groupe méthyle, éthyle, tert-butyle, C(O)O-éthyle, phényle ou thiényle ; les groupes méthyle pouvant être substitués par un groupe hydroxy ou méthoxy.

Parmi les composés de formule générale (I) objets de l'invention, onzième sous-groupe de composés est constitué par les composés pour lesquels
A représente le groupe 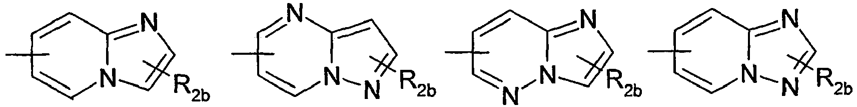
R_{2b} étant tel que défini dans la formule générale (I).

Parmi les composés de formule générale (I) objets de l'invention, douzième sous-groupe de composés est constitué par les composés pour lesquels
A représente le groupe 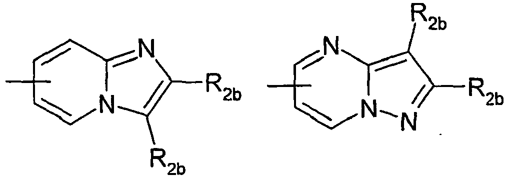 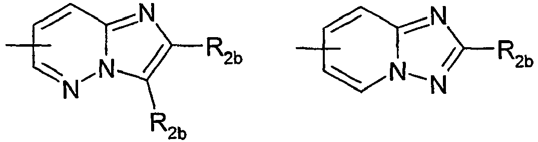
R_{2b} représente un atome d'hydrogène, un groupe C₁-C₆-alkyle, C(O)O-C₁-C₆-alkyle, phényle ou thiényle ; les groupes C₁-C₆-alkyle pouvant être substitués par un groupe hydroxy ou C₁-C₆-alcoxy.

Parmi les composés de formule générale (I) objets de l'invention, treizième sous-groupe de composés est constitué par les composés pour lesquels
A représente le groupe 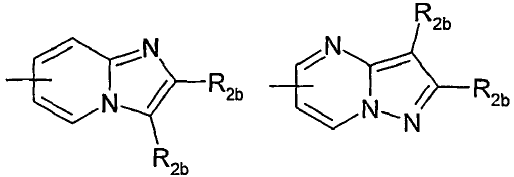 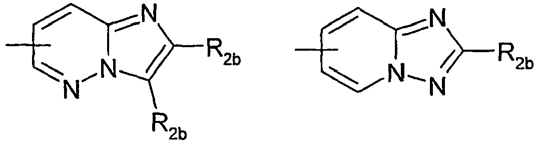
R_{2b} représente un atome d'hydrogène, un groupe méthyle, éthyle, tert-butyle, C(O)O-éthyle, phényle ou thiényle ; les groupes méthyle pouvant être substitués par un groupe hydroxy ou méthoxy.

Parmi les composés de formule générale (I) objets de l'invention, quatorzième sous-groupe de composés est constitué par les composés pour lesquels
A représente le groupe 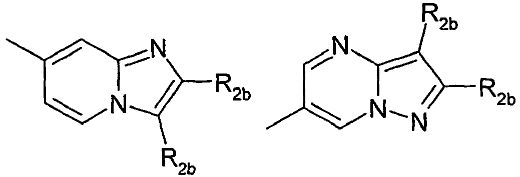
R_{2b} étant tel que défini dans la formule générale (I).

Parmi les composés de formule générale (I) objets de l'invention, quinzième sous-groupe de composés est constitué par les composés pour lesquels
A représente le groupe 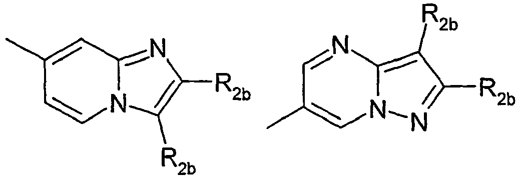
R_{2b} représente un atome d'hydrogène, un groupe C₁-C₆-alkyle, phényle ou thiényle ; les groupes C₁-C₆-alkyle pouvant être substitués par un groupe hydroxy ou C₁-C₆-alcoxy.

Parmi les composés de formule générale (I) objets de l'invention, seizième sous-groupe de composés est constitué par les composés pour lesquels
A représente le groupe 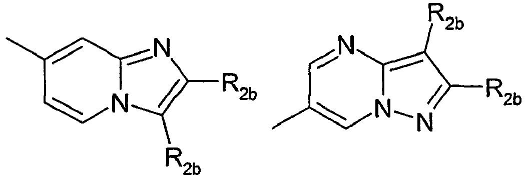
R_{2b} représente un atome d'hydrogène, un groupe méthyle, éthyle, tert-butyle, phényle ou thiényle ; les groupes méthyle pouvant être substitués par un groupe hydroxy ou méthoxy.

Parmi les composés de formule générale (I) objets de l'invention, dix-septième sous-groupe de composés est constitué par les composés pour lesquels les définitions de X₁, X₂, X₃, et X₄, n, Y, W et A données ci-dessus sont combinées.

Parmi les composés de formule générale (I) objets de l'invention, dix-huitième sous-groupe de composés est constitué par les composés pour lesquels
X₁, X₂, X₃, et X₄ représentent, indépendamment l'un de l'autre un groupe C-R₁ ; ou bien X₁, X₂, X₃ représentent un groupe C-R₁ ; X₄ représente un atome d'azote ;
R₁ est choisi parmi un atome d'hydrogène, un atome d'halogène, plus particulièrement un atome de fluor ou un groupe C₁-C₆-fluoroalkyle, plus particulièrement un groupe trifluorométhyle ;
n est égal à 1 ;
Y représente un aryle, plus particulièrement un phényle, éventuellement substitué par un ou plusieurs atomes d'halogène, plus particulièrement de fluor ;
W représente un atome d'oxygène ;
A représente un hétéroaryle bicyclique de formule : 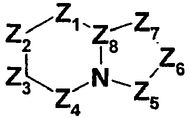 où
Z₁, Z₂, Z₃, et Z₄ représentent, indépendamment l'un de l'autre, un atome de carbone, un atome d'azote ;
Z₅, Z₆ et Z₇ représentent, indépendamment l'un de l'autre, un atome d'azote ou un groupe C-R_{2b} ;
Z₈ représente un atome de carbone ;
deux, au maximum, des Z₁, Z₂, Z₃, Z₄, Z₅, Z₆ et Z₇ représentant un atome d'azote ;
l'un des Z₁, Z₂, Z₃ et Z₄, correspondant à un atome de carbone, étant lié à l'atome d'azote de l'amide ou du thioamide de formule (I) ;
R_{2b} représente un atome d'hydrogène, un groupe C₁-C₆-alkyle, C(O)O-C₁-C₆-alkyle, phényle ou thiényle ; les groupes C₁-C₆-alkyle pouvant être substitués par un groupe hydroxy ou C₁-C₆-alcoxy.

Parmi les composés de formule générale (I) objets de l'invention, on peut notamment citer les composés suivants :
1• *N*-(2,3-Diméthyl-imidazo[1,2-a]pyridin-7-yl)-5-fluoro-1-[(3-fluorophényl)méthyl]-1*H*-indole-2- carboxamide ;
2• *N*-(2,3-Diméthyl-imidazo[1,2-a]pyridin-6-yl)-5-fluoro-1-[(3-fluorophényl)méthyl]-1*H*-indole-2-carboxamide
3• *N*-[2-(Hydroxyméthyl)-imidazo[1,2-a]pyridin-6-yl]-5-fluoro-1-[(3-fluorophényl)méthyl]-1*H*-indole-2-carboxamide
4• *N*-(3-Méthyl-2-phényl-imidazo[1,2-a]pyridin-6-yl)-5-fluoro-1-[(3-fluorophényl)méthyl]-1*H*-indole-2-carboxamide
5• *N*-(2-Ethyl-imidazo[1,2-*a*]pyridin-6-yl)-5-fluoro-1-[(3-fluorophényl)méthyl-1*H-*indole-2-carboxamide.
6• *N*-[2-(Thién-2-yl)-imidazo[1,2-a]pyridin-6-yl]-5-fluoro-1-[(3-fluorophényl)méthyl]-1*H*-indole-2-carboxamide.
7• *N*-(2-*tert*-Butyl-imidazo[1,2-a]pyridin-6-yl)-5-fluoro-1-[(3-fluorophényl)méthyl]-1*H*-indole-2-carboxamide
8• *N*-(2-Méthoxyméthyl-imidazo[1,2-a]pyridin-7-yl)-5-fluoro-1-[(3-fluorophényl)méthyl]-1*H*-indole-2- carboxamide
9• *N*-[2-(Hydroxyméthyl)-imidazo[1,2-a]pyridin-7-yl]-5-fluoro-1-[(3-fluorophényl)méthyl]-1*H*-indole-2- carboxamide
10• *N*-(2-Méthyl-3-phényl-imidazo[1,2-a]pyridin-7-yl)-5-fluoro-1-[(3-fluorophényl)méthyl]-1*H*-indole-2- carboxamide.
11• *N*-[2-(Thién-2-yl)-imidazo[1,2-*a*]pyridin-7-yl]-5-fluoro-1-[(3-fluorophényl)méthyl]-1*H*-indole-2- carboxamide.
12• *N*-(2-Ethyl-imidazo[1,2-*a*]pyridin-7-yl)-5-fluoro-1-[(3-fluorophényl)méthyl-1*H-*indole-2- carboxamide.
13• *N*-(2-*tert*-Butyl-imidazo[1,2-*a*]pyridin-7-yl)-5-fluoro-1-[(3-fluorophényl)méthyl]-1*H*-indole-2- carboxamide
14• *N*-(2,3-Diméthyl-imidazo[1,2-*a*]pyridin-7-yl)-5-trifluorométhyl-1-[(3-fluorophényl)méthyl]-1*H*-indole-2- carboxamide.
15• *N*-(2-Ethyl-imidazo[1,2-*a*]pyridin-7-yl)-5-trifluorométhyl-1-[(3-fluorophényl)méthyl]-1*H*-pyrrolo[2,3-*b*]pyridine-2-carboxamide
16• *N*-(2,3-Diméthyl-imidazo[1,2-*a*]pyridin-7-yl)-5-trifluorométhyl-1-[(3-fluorophényl)méthyl]-1*H*-pyrrolo[2,3-*b*]pyridine-2- carboxamide.
17• *N*-[2-(Hydroxyméthyl)-imidazo[1,2-*a*]pyridin-7-yl]-5-trifluorométhyl-1-[(3-fluorophényl)méthyl]-1*H*-indole-2- carboxamide.
18• *N*-[2-(Hydroxyméthyl)-imidazo[1,2-a]pyridin-7-yl]-5-trifluorométhyl-1-[(3-fluorophényl)méthyl]-1*H*-pyrrolo[2,3-b]pyridine-2-carboxamide
19• *N*-(2-Méthyl-imidazo[1,2-*a*]pyridin-7-yl)-5-fluoro-1-[(3-fluorophényl)méthyl]-1*H-*indole-2- carboxamide
20• *N*-(2-Méthyl-pyrazolo[1,5-*a*]pyrimidin-6-yl)-5-fluoro-1-[(3-fluorophényl)méthyl]-1*H*-indole-2- carboxamide
21• *N*-[2-(Ethyloxycarbonyl)-imidazo[1,2-*b*]pyridazin-6-yl]-5-fluoro-1-[(3-fluorophényl)méthyl]-1*H*-indole-2- carboxamide
22• *N*-[(2-(Ethyloxycarbonyl)-imidazo[1,2-*a*]pyridin-6-yl)-5-fluoro-1-[(3-fluorophényl)méthyl]-1*H*-indole-2- carboxamide
23• *N*-(2-Méthyl-imidazo[1,2-*a*]pyridin-6-yl)-5-fluoro-1-[(3-fluorophényl)méthyl]-1*H-*indole-2- carboxamide
24• *N*-([1,2,4]triazolo[1,5-*a*]pyridin-6-yl)-5-fluoro-1-[(3-fluorophényl)méthyl]-1H-indole-2- carboxamide

Parmi les composés de formule générale (I) objets de l'invention, une sous-famille est représentée par les composés de formule générale (I') pour lesquels :
R_{2b} représente un atome d'hydrogène, un atome d'halogène, un groupe C₁-C₆-alkyle, C₃-C₇-cycloalkyle, C₃-C₇-cycloalkyl-C₁-C₃-alkylène, C₁-C₆-fluoroalkyle, C₁-C₆-alcoxy, hydroxy, thiol, oxo, thio, C₃-C₇-cycloalkyloxy, C₁-C₆-fluoroalcoxy, C₃-C₇-cycloalkyl-C₁-C₃-alkylénoxy, C₁-C₆-alcoxy-C₁-C₃-alkylène, C₃-C₇-cycloalkyloxy-C₁-C₃-alkylène, C₃-C₇-cycloalkyl-C₁-C₃-alkylénoxy-C₁-C₃-alkylène, C₁-C₆-alkyl-C(O)-O-C₁-C₃-alkylène, C₁-C₆-alkyl-C(O)-O-, C₃-C₇-cycloalkyl-C(O)-O-C₁-C₃-alkylène, C₃-C₇-cycloalkyl-C(O)-O-, C₁-C₆-fluoroalkyl-C(O)-O-C₁-C₃-alkylène, C₁-C₆-fluoroalkyl-C(O)-O-, C(O)NR₃R₄, C(O)O-C₁-C₆-alkyle, cyano, CHO, CO₂H, -C(O)-C₁-C₆-alkyle, -C(O)-C₃-C₇-cycloalkyle ; les groupes C₁-C₆-alkyle, C₃-C₇-cycloalkyle, C₃-C₇-cycloalkyl-C₁-C₃-alkylène, C₁-C₆-fluoroalkyle, C₁-C₆-alcoxy, C₃-C₇-cycloalkyloxy, C₁-C₆-fluoroalcoxy, C₃-C₇-cycloalkyl-C₁-C₃-alkylénoxy, C₁-C₆-alcoxy-C₁-C₃-alkylène, C₃-C₇-cycloalkyloxy-C₁-C₃-alkylène, C₃-C₇-cycloalkyl-C₁-C₃-alkylénoxy-C₁-C₃-alkylène pouvant être substitués par un groupe hydroxy, C₁-C₆-alcoxy ou NR₃R₄ ;
R₃ et R₄ étant tels que définis dans la formule générale (I).

Parmi les composés de formule générale (I') objets de l'invention, un premier sous-groupe de composés est constitué par les composés pour lesquels X₁, X₂, X₃, et X₄ représentent, indépendamment l'un de l'autre un groupe C-R₁ ; et R₁ est choisi parmi un atome d'hydrogène, un atome d'halogène, plus particulièrement un atome de fluor ou un groupe C₁-C₆-fluoroalkyle, plus particulièrement un groupe trifluorométhyle.

Parmi les composés de formule générale (I') objets de l'invention, un second sous-groupe de composés est constitué par les composés pour lesquels n est égal à 1 et Y représente un aryle, plus particulièrement un phényle, éventuellement substitué par un ou plusieurs atomes d'halogène, plus particulièrement de fluor.

Parmi les composés de formule générale (I') objets de l'invention, un troisième sous-groupe de composés est constitué par les composés pour lesquels W représente un atome d'oxygène.

Parmi les composés de formule générale (I') objets de l'invention, un quatrième sous-groupe de composés est constitué par les composés pour lesquels A représente le groupe 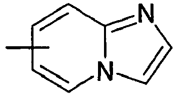 ce groupe étant éventuellement substitué comme défini dans la formule générale (I) ci-dessus.

Parmi les composés de formule générale (I') objets de l'invention, un cinquième sous-groupe de composés est constitué par les composés pour lesquels
X₁, X₂, X₃, et X₄ représentent, indépendamment l'un de l'autre, un groupe C-R₁ ; et R₁ est choisi parmi un atome d'hydrogène, un atome d'halogène, plus particulièrement un atome de fluor ou un groupe C₁-C₆-fluoroalkyle, plus particulièrement un groupe trifluorométhyle ;
n est égal à 1 ;
Y représente un aryle, plus particulièrement un phényle, éventuellement substitué par un ou plusieurs atomes d'halogène, plus particulièrement de fluor ;
W représente un atome d'oxygène ;
A représente le groupe 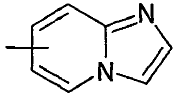 ce groupe étant éventuellement substitué comme défini dans la formule générale (I) ci-dessus.

On entend par groupe partant, dans ce qui suit, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un méthanesulfonate, benzènesulfonate, p-toluènesulfonate, trifluorométhanesulfonate, acétate, etc. Des exemples de groupes partants ainsi que des références pour leur préparation sont donnés dans « Advances in Organic Chemistry », J. March, 5th Edition, Wiley Interscience, 2001.
On entend par groupe protecteur, dans ce qui suit, un groupe pouvant être momentanément incorporé à une structure chimique dans le but d'inactiver temporairement une partie de la molécule lors d'une réaction et qui peut être facilement être enlevé à une étape ultérieure de la synthèse. Des exemples de groupes protecteurs ainsi que des références concernant leurs propriétés sont donnés dans T.W. Greene, P.G.M. Wutz, 3rd Edition, Wiley Interscience 1999.

Conformément à l'invention, on peut préparer les composés de formule générale (I) selon le procédé illustré par les schémas généraux 1 ou 2 qui suivent :

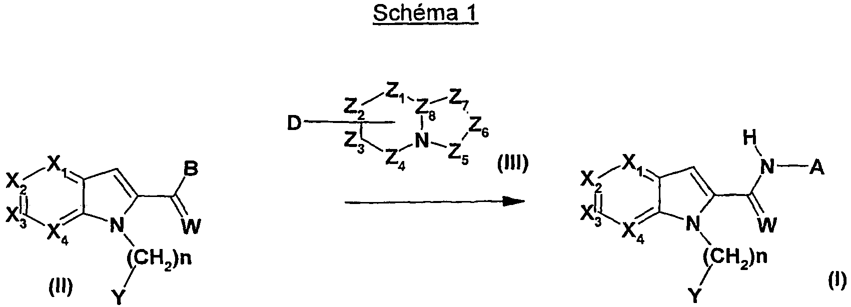

Selon le schéma 1, les composés (I) peuvent être obtenus par réaction d'un composé de formule générale (II) dans laquelle B représente un groupe NH₂ et X₁, X₂, X₃, X₄, n, Y et W sont tels que définis dans la formule générale (I) ci-dessus avec un composé de formule générale (III), dans laquelle Z₁, Z₂, Z₃, Z₄, Z₅, Z₆, Z₇ et Z₈ sont tels que définis dans la formule générale (I) ci-dessus et D représente un groupe partant, tel que défini ci-dessus, comme, par exemple, un atome de brome, d'iode ou un groupe trifluorométhanesulfonate,
- par exemple selon une méthode analogue à celle décrite dans J.Am. Chem. Soc. 2001, 123 (31), 7727, ou selon des méthodes décrites dans la littérature ou connues de l'homme du métier, en présence d'un sel de cuivre en quantité catalytique, en présence d'une quantité catalytique d'un ligand du cuivre, tel qu'une diamine, le tout en présence d'une base comme le carbonate de potassium, dans un solvant tel que le dioxane ;
- par exemple selon une méthode analogue à celle décrite dans J.Am.Chem.Soc. 2002, 124 (21), 6043, ou selon des méthodes décrites dans la littérature ou connues de l'homme du métier, en présence d'une quantité catalytique d'un dérivé du palladium, tel que le palladium diacétate, en présence d'une quantité catalytique d'un ligand du palladium, tel qu'une diphosphine, le tout en présence d'une base comme le carbonate de césium, au reflux d'un solvant tel que le dioxane.

Les composés de formule générale (I) peuvent également être obtenus par réaction d'un composé de formule générale (II) dans laquelle B représente un groupe hydroxyle et X₁, X₂, X₃, X₄, n, Y et W sont tels que définis dans la formule générale (I1) ci-dessus avec un composé de formule générale (III), dans laquelle Z₁, Z₂, Z₃, Z₄, Z₅, Z₆, Z₇ et Z₈ sont tels que définis dans la formule générale (I) ci-dessus et D représente un groupe NH₂, en présence d'un agent de couplage tel que le cyanophosphonate de diéthyle ou le *N*-(3-diméthylaminopropyl)-*N'*-éthylcarbodiimide, éventuellement en présence d'une base telle que la triéthylamine, dans un solvant tel que par exemple le diméthylformamide.

Les composés de formule générale (I) peuvent également être obtenus par réaction d'un composé de formule générale (II) où B est un atome de chlore et X₁, X₂, X₃, X₄, n, Y et W sont tels que définis dans la formule générale (I) ci-dessus avec un composé de formule générale (III), dans laquelle Z₁, Z₂, Z₃, Z₄, Z₅, Z₆, Z₇ et Z₈ sont tels que définis dans la formule générale (1) ci-dessus et D représente un groupe NH₂, dans un solvant tel que le dichloroéthane, le toluène ou le tétrahydrofuranne.

Les composés de formule générale (II), pour lesquels B représente un groupe C₁-C₆-alcoxyle, peuvent être transformés en composés de formule générale (II), où B représente un groupe hydroxyle, par l'action d'une base telle que la soude ou la potasse en solution dans un solvant tel que l'éthanol. Les composés de formule générale (II), où B représente un groupe hydroxyle peuvent, par la suite, être transformés en composés de formule générale (II), où B représente un atome de chlore, par l'action d'un agent chlorant tel que le chlorure de thionyle dans un solvant tel que le dichlorométhane.

Alternativement, selon le schéma 2, les composés de formule générale (I), dans laquelle W représente un atome d'oxygène et A représente un groupe : 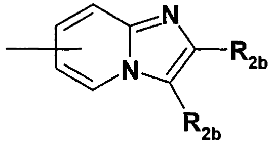 peuvent être obtenus par réaction d'un composé de formule générale (V) dans laquelle X₁, X₂, X₃, X₄, n et Y sont tels que définis dans la formule générale (I) ci-dessus et GP représente un atome d'hydrogène, avec un composé de formule générale (IV), dans laquelle X représente un groupe partant, tel que défini ci-dessus, comme, par exemple, un atome de brome ou de chlore et R_{2b} est tel que défini dans la formule générale (I) ci-dessus, par exemple selon une méthode analogue à celles décrites dans J.Org. Chem. 1965, 30 (7), 2403 et Organometallics 2008, 27(8), 1936 ou selon des méthodes décrites dans la littérature ou connues de l'homme du métier.

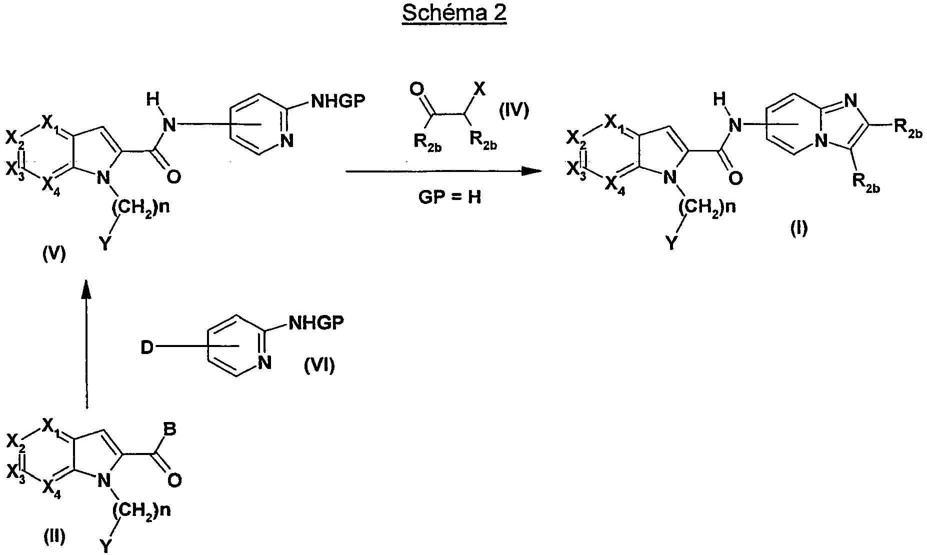

Les composés de formule générale (V) dans lesquels GP représente un atome d'hydrogène, peuvent être obtenus à partir des composés de formule générale (V) dans laquelle GP représente un groupe protecteur, par exemple un groupe acétyle, selon des méthodes de déprotection décrites dans la littérature ou connues de l'homme du métier.

Les composés de formule générale (V) dans laquelle GP représente un groupe protecteur, par exemple un groupe acétyle, peuvent être obtenus par réaction d'un composé de formule générale (II) dans laquelle B représente un groupe NH₂ et X₁, X₂, X₃, X₄, n et Y sont tels que définis dans la formule générale (I) ci-dessus avec un composé de formule générale (VI), dans laquelle Gp représente un groupe protecteur, par exemple un groupe acétyle, et D représente un groupe partant, tel que défini ci-dessus, comme, par exemple, un atome de brome ou un groupe trifluorométhanesulfonate, par exemple selon une méthode analogue à celle décrite dans J.Am.Chem.Soc. 2001, 123 (31), 7727, ou selon des méthodes décrites dans la littérature ou connues de l'homme du métier, en présence d'un sel de cuivre en quantité catalytique, en présence d'une quantité catalytique d'un ligand du cuivre, tel qu'une diamine, le tout en présence d'une base comme le carbonate de potassium, dans un solvant tel que le dioxane.
Les composés de formule générale (V) peuvent également être obtenus par réaction d'un composé de formule générale (II) dans laquelle B représente un groupe hydroxyle et X₁, X₂, X₃, X₄, n et Y sont tels que définis dans la formule générale (I) ci-dessus avec un composé de formule générale (VI), dans laquelle GP représente un groupe protecteur, par exemple un groupe acétyle, et D représente un groupe amino, selon toutes méthodes de couplage connues de l'homme de métier.

L'invention, selon un autre de ses aspects, a également pour objet les composés de formules (IIIa), (IIIb), (IIIc), (IIId), (IIIe) et (IIIf). Ces composés sont utiles comme intermédiaires de synthèse pour la préparation des composés de formule (I).

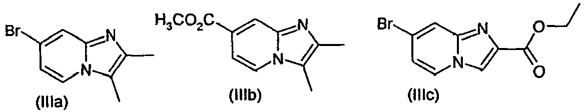

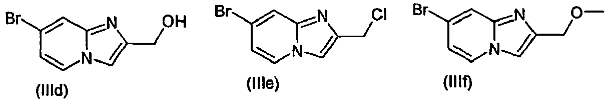

Les composés de formules (IIIa), (IIIb), (IIIc), (IIId), (IIIe) et (IIIf) peuvent être préparés, par exemple selon les procédés décrits dans les exemples 1, 8 et 14.

Dans les schémas 1 et 2, les composés de formule générale (II), (III), (IV), (VI) et les autres réactifs, quand leur mode de préparation n'est pas décrit, sont disponibles dans le commerce, décrits dans la littérature ou préparés par analogie à des procédés décrits dans la littérature (US6,737,435 ; US6,673,797 ; WO2006/101455 ; Synthesis 1985, 2, 186 ; J.G. Lombardino J. Org. Chem. 1965, 30 (7), 2403 ; T.M. Williams J.Med.Chem. 1993, 36 (9), 1291; J.Med.Chem. 2008, 51 (19), 6044; JP2001151771A2 ; WO2006/024776; WO2006/072736 ; WO2007/010144 ; WO2007/010138 par exemple).

Les composés de formule générale (I), pour lesquels l'un des X₁, X₂, X₃ ou X₄, correspond à un atome de carbone substitué par un groupe alkyle, peuvent être obtenus par une réaction de couplage, catalysée par un métal tel que le palladium ou le fer, réalisée sur les composés de formules générales (I) correspondants, substitués par un atome d'halogène, tel qu'un chlore, en présence par exemple d'un halogénure d'alkylmagnésium ou d'un halogénure d'alkylzinc, selon les méthodes décrites dans la littérature (A. Furstner et al J Am Chem Soc 2002, 124(46), 13856 ; G. Quéguiner et al J Org Chem 1998, 63(9), 2892) par exemple, ou connues de l'homme du métier.
Les composés de formule générale (I), pour lesquels l'un des X₁, X₂, X₃ ou X₄, correspond à un atome de carbone substitué par un groupe cyano, aryle ou hétéroaryle, peuvent être obtenus par une réaction de couplage, catalysée par un métal tel que le palladium, réalisée sur les composés de formule générale (I) correspondants, substitués, par exemple, par un atome de brome, en présence de cyanure de triméthylsilyle, d'acide arylboronique ou d'acide hétéroarylboronique, ou par toute autre méthode décrite dans la littérature ou connue de l'homme du métier. Les composés de formule générale (I) substitués par un groupe C(O)NR₃R₄, peuvent être obtenus à partir des composés de formule générale (I) correspondants substitués par un groupe cyano, selon des méthodes décrites dans la littérature ou connues de l'homme du métier.
Les composés de formule générale (I) substitués par un groupe -S(O)-alkyle ou -S(O)₂-alkyle, peuvent être obtenus par oxydation des composés de formule générale (I) correspondants, substitués par un groupe thioalkyle, selon des méthodes décrites dans la littérature ou connues de l'homme du métier.
Les composés de formule générale (I), substitués par un groupe NR₃R₄, NR₅COR₆ ou NR₅SO₂R₇, peuvent être obtenus à partir des composés de formule générale (I) correspondants, substitués par un groupe nitro, par exemple par réduction, puis acylation ou sulfonylation, selon des méthodes décrites dans la littérature ou connues de l'homme du métier.
Les composés de formule générale (I) dans laquelle W représente un atome de soufre peuvent, par exemple, être obtenus par réaction des composés de formule générale (I) correspondants dans lesquels W représente un atome d'oxygène, avec un réactif tel que le réactif de Lawesson.
Les composés de formule générale (I) pour lesquelles R_{2b} correspond à un groupe hydroxyalkyle peuvent être obtenus à partir des composés de formule générale (I) pour lesquelles R_{2b} correspond, par exemple, à un groupe acétoxyalkyle ou pivaloyloxyalkyle selon des méthodes chimiques connues de l'homme de l'art, telle que la réaction avec une base comme une solution aqueuse de soude, ou la réaction avec un alcoolate, par exemple le méthanolate, d'un sel tel que le lithium ou de sodium, dans un solvant alcoolique tel que le méthanol ou l'éthanol.
Les composés de formule générale (I) pour lesquelles R_{2b} correspond à un groupe hydroxyméthyle peuvent également être obtenus à partir des composés de formule générale (I) pour lesquelles R_{2b} correspond, par exemple, à un groupe carboxylate d'éthyle par réaction avec un agent réducteur tel que le borohydrure de sodium, dans un solvant tel que le tétrahydrofurane.
Les composés de formule générale (II), pour lesquels l'un des X₁, X₂, X₃ ou X₄, correspond à un atome de carbone substitué par un groupe NR₃R₄, NR₅COR₆ ou NR₅SO₂R₇ et B représente un groupe C₁-C₆-alcoxy, C₃-C₇-cycloalkyloxy, C₃-C₇-cycloalkyle-C₁-C₃-alkylénoxy ou aryle-C₁-C₃-alkylénoxy, peuvent être obtenus à partir des composés de formule générale (II) correspondants, substitués, par exemple, par un atome de brome, par réaction de couplage respectivement avec une amine, un amide ou une sulfonamide en présence d'une base, d'une phosphine et d'un catalyseur à base de palladium, selon des méthodes décrites dans la littérature ou connues de l'homme du métier.
Les composés de formule générale (II), pour lesquels l'un des X₁, X₂, X₃ ou X₄, correspond à un atome de carbone substitué par un groupe SO₂NR₃R₄ et B représente un groupe C₁-C₆-alcoxy, C₃-C₇-cycloalkyloxy, C₃-C₇-cycloalkyle-C₁-C₃-alkylénoxy ou aryle-C₁-C₃-alkylénoxy peuvent être obtenus par une méthode analogue à celle décrite dans Pharmazie 1990, 45, 346, ou selon des méthodes décrites dans la littérature ou connues de l'homme du métier.

Les exemples suivants décrivent la préparation de certains composés conformes à l'invention. Ces exemples ne sont pas limitatifs et ne font qu'illustrer la présente invention. Les numéros des composés exemplifiés renvoient à ceux du tableau 1. Les microanalyses élémentaires, les analyses LC-MS (chromatographie liquide couplée à la spectrométrie de masse) et les spectres I.R. ou R.M.N. confirment les structures des composés obtenus.

### Exemple 1 (Composé N°1)

### N-(2,3-Diméthyl-imidazo[1,2-a]pyridin-7-yl)-5-fluoro-1-[(3-fluorophényl)méthyl]-1H-indole-2-carboxamide

### 1.1 Acide 5-fluoro-1-[(3-fluorophényl)méthyl]-1H-indole-2-carboxylique

On ajoute une solution aqueuse de soude, préparée à partir de 1,15 g (28,92 mmoles) de pastille de soude dans 50 mL d'eau, à une solution de 7,6 g (24,10 mmoles) de 5-fluoro-1-[(3-fluorophényl)méthyl]-1*H*-indole-2-carboxylate d'éthyle (WO2006/024776) dans 241 mL d'éthanol. Le mélange est chauffé durant deux heures puis concentré sous pression réduite. Le solide résultant est repris dans 200 mL d'eau. La solution est lavée avec deux fois 100 mL d'éther éthylique, acidifiée par ajouts successifs de petites quantités d'acide chlorhydrique concentré puis extraite avec 200 mL d'acétate d'éthyle. La phase organique est finalement lavée deux fois avec 100 mL d'eau, une fois avec 50 ml d'une solution saturée en chlorure de sodium, séchée sur sulfate de magnésium et concentrée sous pression réduite. On obtient, après séchage à 50°C sous pression réduite, 6,4 g du produit attendu sous la forme d'un solide qui sera utilisé tel quel dans la suite de la synthèse.

### 1.2 5-fluoro-1-[(3-fluorophényl)méthyl]-1H-indole-2-carboxamide

A une suspension, agitée à 20°C, de 2 g (6,96 mmoles) d'acide 5-fluoro-1-[(3-fluorophényl)méthyl]-1*H*-indole-2-carboxylique préparé à l'étape 1.1, dans 80 mL de toluène sec, sont ajoutés 5,08 mL (69,62 mmoles) de chlorure de thionyle. Le mélange réactionnel est agité 2 heures à reflux puis est concentré sous pression réduite. Le produit résultant est repris par 10 mL de dichlorométhane et on verse cette solution, goutte à goutte, sur une solution de 9,12 mL (69,62 mmoles) d'ammoniaque à 30% dans l'eau. Le mélange réactionnel est agité 14 heures à 20°C. Après ce temps, on recueille, par filtration, un solide que l'on triture dans 50 mL d'éther diisopropylique. On recueille après filtration et séchage sous pression réduite 0,58 g de produit attendu. R.M.N. ¹H (DMSO-D₆), δ ppm : 8,11 (pic élargi, 1H) ; 7,5 (m, 3H) ; 7,32 (m, 1H) ; 7,25 (s, 1H) ; 7,09 (m, 2H) ; 6,89 (m, 2H) ; 5,91 (s, 2H).

### 1.3 7-Bromo-2,3-diméthyl-imidazo[1,2-a]pyridirie (Composé N° IIIa)

On ajoute 3 mL (19,86 mmoles) de 3-bromobutan-2-one à un mélange, agité à 20°C, de 1,5 g (8,67 mmoles) de 2-amino-4-bromopyridine et de 1,45 g (17,34 mmoles) d'hydrogénocarbonate de sodium dans 50 mL d'éthanol. Le mélange est agité au reflux durant 12 heures puis concentré sous pression réduite. On reprend le mélange obtenu avec 20 mL d'eau. On basifie le pH de la solution par ajouts successifs de carbonate de potassium. On recueille par filtration un précipité que l'on lave à l'eau puis que l'on sèche sous pression réduite. On isole ainsi 0,79 g du produit attendu que l'on utilise, tel quel, dans la suite de la synthèse.
R.M.N. ¹H (DMSO-D₆), δ ppm : 8,15 (d, 1H) ; 7,71 (s, 1H) ; 7,01 (d, 1H) ; 2,39 (s, 3H) ; 2,31 (s, 3H).

### 1.4 N-(2,3-Diméthyl-imidazo[1,2-a]pyridin-7-yl)-5-fluoro-1-[(3-fluorophényl)méthyl]-1H-indole-2- carboxamide (composé n°1)

On mélange, dans un tube à pression sous atmosphère inerte à 20°C, 0,0134 g (0,07 mmole) d'iodure de cuivre, 0,2 g (0,7 mmole) de 5-fluoro-1-[(3-fluorophényl)méthyl]-1*H*-indole-2-carboxamide, obtenu à l'étape 1.2, 132 mg (0,56 mmole) de 7-bromo-2,3-diméthyl-imidazo[1,2-a]pyridine, obtenu à l'étape 1.3, 193 mg (1,4 mmole) de carbonate de potassium et 5 mL de dioxane. La suspension est dégazée quelques minutes puis on ajoute 0,01 mL (0,08 mmole) de trans-1,2-cyclohexanediamine et on ferme le tube. Le mélange réactionnel est agité 20 heures à 110 °C. Après ce temps, la suspension refroidie est filtrée sur un tampon de silice en éluant avec un mélange de dichlorométhane et de méthanol. Le filtrat est concentré sous pression réduite. Le produit résultant est trituré dans un mélange de 90 mL d'isopropanol et de 10 mL d'éther isopropylique. On recueille par filtration un solide que l'on purifie par chromatographie sur colonne de silice en éluant avec un mélange de dichlorométhane et de méthanol. On obtient ainsi 27 mg du produit attendu.
PF : 251 - 253 °C
R.M.N. ¹H (DMSO-D₆), δ ppm : 10,82 (s, 1H) ; 8,41 (pic élargi, 1H) ; 8,19 (pic élargi, 1 H) ; 7,62 (dxd, 1 H) ; 7,58 (dxd, 1 H) ; 7,51 (s, 1 H) ; 7,45 (d, 1 H) ; 7,32 (m, 1 H) ; 7,19 (txd, 1 H) ; 7,4 (txd, 1 H) ; 6,5 (m, 2H) ; 5,89 (s, 2H) ; 2,5 (s, 6H).

### Exemple 2 (Composé N°2)

### N-(2,3-Diméthyl-imidazo[1,2-a]pyridin-6-yl)-5-fluoro-1-[(3-fluorophényl)méthyl]-1H-indole-2- carboxamide

### 2.1 6-Bromo-2,3-diméthyl-imidazo[1,2-a]pyridine

On ajoute 13,53 mL (130,06 mmoles) de 3-bromobutan-2-one à un mélange, agité à 20°C, de 5 g (28,90 mmoles) de 2-amino-5-bromopyridine et de 4,85 g (57,80 mmoles) d'hydrogénocarbonate de sodium dans 100 mL d'éthanol. Le mélange est agité au reflux durant 12 heures puis concentré sous pression réduite. On reprend le mélange obtenu avec 100 mL d'eau. On basifie le pH de la solution par ajouts successifs de carbonate de potassium. On extrait ensuite le mélange avec 3x50 mL d'acétate d'éthyle. Les phases organiques rassemblées sont lavées à l'eau (50 mL), séchées sur sulfate de sodium puis concentrées sous pression réduite. On obtient une huile que l'on purifie par chromatographie sur colonne de silice en éluant avec un mélange de dichlorométhane et de méthanol. On isole ainsi 1,1 g du produit attendu.
R.M.N. ¹H (DMSO-D₆), δ ppm : 8,5 (s, 1 H) ; 7,4 (d, 1 H) ; 7,25 (d, 1H) ; 2,4 (s, 3H) ; 2,31 (s, 3H).
LC-MS: 225 ([M+H]+, *R*t= 0,52 min.).

### 2.2 N-(2,3-Diméthyl-imidazo[1,2-a]pyridin-6-yl)-5-fluoro-1-[(3-fluorophényl)méthyl]-1H-indole-2-carboxamide (composé n°2)

On mélange, dans un tube à pression sous atmosphère inerte à 20°C, 0,054 g (0,28 mmole) d'iodure de cuivre, 0,8 g (2,79 mmole) de 5-fluoro-1-[(3-fluorophényl)méthyl]-1*H*-indole-2-carboxamide, obtenu à l'étape 1.2, 0,5 g (2,24 mmoles) de 6-bromo-2,3-diméthyl-imidazo[1,2-*a*]pyridine, obtenu à l'étape 2.1, 0,77 g (5,59 mmoles) de carbonate de potassium et 25 mL de dioxane. La suspension est dégazée quelques minutes puis on ajoute 0,04 mL (0,31 mmole) de trans-1,2-cyclohexanediamine et on ferme le tube. Le mélange réactionnel est agité 20 heures à 110 °C. Après ce temps, le mélange réactionnel est dilué avec 50 mL d'eau et 50 mL d'acétate d'éthyle. La phase aqueuse est extraite avec 2x50 mL d'acétate d'éthyle. Les phases organiques rassemblées sont lavées à l'eau (50 mL), séchées sur sulfate de sodium puis concentrées sous pression réduite. Le produit résultant est trituré dans un mélange bouillant de 30 mL d'éther isopropylique et de 10 mL d'eau. On recueille par filtration à chaud 0,63 g du produit attendu sous la forme d'un solide blanc.
PF : 220 - 242 °C
R.M.N. ¹H (DMSO-D₆), δ ppm : 11,0 (s, 1 H) ; 9,2 (s, 1 H) ; 8,1 (m, 1 H) ; 7,95 (m, 1H) ; 7,6 (m, 2H) ; 7,55 (s, 1H) ; 7,35 (m, 1H) ; 7,2 (m, 1 H) ; 7,15 (m, 1H) ; 6,9 (m, 2H) ; 5,9 (s, 2H) ; 2,5 (s, 3H) ; 2,45 (s, 3H).

### Exemple 3 (Composé N°3)

### N-[2-(hydroxyméthyl-imidazo[1,2-a]pyridin-6-yl]-5-fluoro-1-[(3-fluorophényl)méthyl]-1H-indole-2- carboxamide

On mélange, dans un tube à pression sous atmosphère inerte à 20°C, 20 mg (0,1 mmole) d'iodure de cuivre, 0,3 g (1,05 mmole) de 5-fluoro-1-[(3-fluorophényl)méthyl]-1*H*-indole-2-carboxamide, obtenu à l'étape 1.2, 0,23 g (0,84 mmole) de 6-iodo imidazo[1,2-*a*]pyridin-2-ylméthanol, 0,29 g (2,1 mmoles) de carbonate de potassium et 10 mL de dioxane. La suspension est dégazée quelques minutes puis on ajoute 0,01 mL (0,12 mmole) de trans-1,2-cyclohexanediamine et on ferme le tube. Le mélange réactionnel est agité 15 heures à 110 °C. Après ce temps, la suspension refroidie à température ambiante est versée dans 20 mL d'eau et 20 mL d'acétate d'éthyle. On extrait la phase aqueuse avec 2x10 mL d'acétate d'éthyle. Les phases organiques rassemblées sont séchées sur sulfate de magnésium et concentrées sous pression réduite. Le produit résultant est trituré dans un mélange bouillant de 10 mL de dichlorométhane et de 30 mL d'éther isopropylique puis filtré à chaud. On recueille après séchage 0,31 g du produit attendu sous la forme d'un solide beige.
PF : 247 - 249 °C
R.M.N. ¹H (DMSO-D₆), δ ppm : 10,6 (s, 1H) ; 9,3 (s, 1H) ; 7,9 (s, 1H) ; 7,6 (m, 2H) ; 7,5 (m, 2H) ; 7,35 (m, 2H) ; 7,2 (m, 1 H) ; 7,1 (m, 1 H) ; 6,9 (m, 2H) ; 5,9 (s, 2H) ; 5,1 (t, 1H); 4,6 (d, 2H).
LC-MS: 433 ([M+H]+, *R*t= 4,12 min.).

### Exemple 4 (Composé N°4)

### N-(3-méthyl-2-phényl-imidazo[1,2-a]pyridin-6-yl)-5-fluoro-1-[(3-fluorophényl)méthyl]-1H-indole-2- carboxamide

### 4.1 N-[6-(Acétylamino)pyridin-3-yl]-5-fluoro-1-[(3-fluorophényl)méthyl]-1H-indole-2-carboxamide

A une solution, agitée à 20°C sous atmosphère inerte, de 2 g (6,96 mmoles) de l'acide 5-fluoro-1-[(3-fluorophényl)méthyl]-1*H*-indole-2-carboxylique, préparé selon le protocole décrit à l'étape 1.1, dans 40 mL de DMF, sont ajoutés, 1,72 g (8,35 mmoles) de dicyclohexylcarbodiimide (DCC) et 1,13 g (8,35 mmoles) de 1-hydroxy-benzotriazole hydrate (HOBT). Le mélange réactionnel est agité à température ambiante pendant 30 minutes. On ajoute ensuite au mélange réactionnel, 2,1 g (13,92 mmoles) de 2-acétamido-5-aminopyridine et 20 mg de diméthylaminopyridine (DMAP). Après 24 heures d'agitation à 20°C, le mélange réactionnel est concentré à sec. Le résidu obtenu est repris avec un mélange de 200 mL d'une solution aqueuse saturée en NaHCO₃ et de 200 mL d'acétate d'éthyle. On décante et on extrait la phase aqueuse par 200 mL d'acétate d'éthyle. Les phases organiques rassemblées sont séchées sur sulfate de sodium puis concentrées sous pression réduite. On recueille un solide que l'on purifie par chromatographie sur colonne de silice en éluant avec un mélange de dichlorométhane et de méthanol. On obtient ainsi 2,6 g du produit attendu. R.M.N. ¹H (DMSO-D₆), δ ppm : 10,6 (s, 1H) ; 10,5 (s, 1H) ; 8,7 (s, 1H) ; 8,1 (s, 2H) ; 7,6 (m, 2H) ; 7,45 (s, 1 H) ; 7,35 (m, 1 H) ; 7,2 (m, 1 H) ; 7,1 (m, 1 H) ; 6,9 (m, 2H) ; 5,9 (s, 2H) ; 2,1 (s, 3H).
LC-MS: 421 ([M+H]+, *R*t= 1,10 min.).

### 4.2 N-[6-Aminopyridin-3-yl]-5-fluoro-1-[(3-fluorophényl)méthyl]-1H-indole-2-carboxamide

A une suspension de 0,6 g (1,43 mmoles) de *N*-[6-(acétylamino)pyridin-3-yl]-5-fluoro-1-[(3-fluorophényl)méthyl]-1*H*-indole-2-carboxamide préparé selon le protocole décrit à l'étape 4.1, dans 9 mL de méthanol anhydre, agitée à 0°C sous atmosphère inerte, sont ajoutés goutte à goutte, 2,03 mL (28,54 mmoles) de chlorure d'acétyle. Le mélange réactionnel est agité à 0°C pendant 30 minutes puis progressivement porté à 70°C et agité pendant 48 heures. Le mélange réactionnel est concentré à sec. Le résidu obtenu est repris avec un mélange de 200 mL d'une solution aqueuse saturée en NaHCO₃ et de 200 mL d'acétate d'éthyle. On décante et on extrait la phase aqueuse par 200 mL d'acétate d'éthyle. Les phases organiques rassemblées sont séchées sur sulfate de sodium puis concentrées sous pression réduite. On obtient ainsi 0,46 g du produit attendu.
R.M.N. ¹H (DMSO-D₆), δ ppm : 8,2 (s, 1H) ; 7,7 (m, 1H) ; 7,6 (m, 2H) ; 7,35 (m, 2H) ; 7,2 (m, 1 H) ; 7,1 (m, 1 H) ; 6,9 (m, 2H) ; 6,5 (d, 1 H) ; 5,9 (s, 2H) ; 5,8 (s, 2H).

### 4.3 N-(3-méthyl-2-phényl-imidazo[1,2-a]pyridin-6-yl)-5-fluoro-1-[(3-fluorophényl)méthyl]-1H-indole-2-carboxamide (composé N°4)

Dans un tube à vis de 10 mL muni d'un barreau magnétique, sont introduits 0,05 g (0,13 mmole) de *N*-[6-aminopyridin-3-yl]-5-fluoro-1-[(3-fluorophényl)méthyl]-1*H*-indole-2-carboxamide préparé selon le protocole décrit à l'étape 4.2, 0,056 g (0,26 mmole) de 2-bromo-1-phényl-propan-1-one et 4 mL d'acétonitrile. Le tube est ensuite vissé et mis à chauffer à 80°C sous agitation pendant 24 heures. Après ce temps et retour à température ambiante, le précipité obtenu est filtré, rincé successivement avec 2 mL d'acétonitrile et 2 mL d'éther isopropylique. Le solide obtenu est séché sous pression réduite. On obtient ainsi 0,024 g du produit attendu.
PF : 258 - 259 °C
R.M.N. ¹H (DMSO-D₆), δ ppm : 10,65 (s, 1H) ; 9,0 (s, 1H) ; 7,85 (d, 2H) ; 7,6 (m, 3H) ; 7,5 (m, 4H) ; 7,35 (m, 2H) ; 7,2 (m, 1 H) ; 7,1 (m, 1 H) ; 6,9 (m, 2H) ; 5,9 (s, 2H) ; 2,65 (s, 3H).

### Exemple 5 (Composé N°5)

### N-(2-Ethyl-imidazo[1,2-a]pyridin-6-yl)-5-fluoro-1-[(3-fluorophényl)méthyl]-1H-indole-2-carboxamide

Le composé N° 5 a été préparé selon un procédé similaire à celui décrit à l'étape 4.3 en faisant réagir 0,1 g (0,26 mmole) de *N*-[6-aminopyridin-3-yl]-5-fluoro-1-[(3-fluorophényl)méthyl]-1*H*-indole-2-carboxamide préparé selon le protocole décrit à l'étape 4.2 avec 0,08 g (0,53 mmole) de 1-bromo-butan-2-one dans 4 mL d'acétonitrile. On obtient 0,112 g du produit attendu.
PF = 278 - 279 °C
R.M.N. ¹H (DMSO D₆), δ (ppm) : 9,65 (s, 1H) ; 8,25 (s, 1 H) ; 8,05 (d, 1H) ; 7,95 (d, 1H) ; 7,65 (m, 2H) ; 7,55 (s, 1H) ; 7,35 (m, 1H) ; 7,25 (m, 1 H) ; 7,1 (m, 1H) ; 6,9 (m, 2H) ; 5,9 (s, 2H) ; 2,9 (q, 2H) ; 1,3 (t, 3H).
LC-MS: 431 ([M+H]+, *R*t= 1,09 min.).

### Exemple 6 (Composé N°6)

### N-[2-(Thién-2-yl)-imidazo[1,2-a]pyridin-6-yl]-5-fluoro-1-[(3-fluorophényl)méthyl]-1H-indole-2- carboxamide

Le composé N° 6 a été préparé selon un procédé similaire à celui décrit à l'étape 4.3 en faisant réagir 0,1 g (0,26 mmole) de *N*-[6-aminopyridin-3-yl)-5-fluoro-1-[(3-fluorophényl)méthyl]-1*H*-indole-2-carboxamide préparé selon le protocole décrit à l'étape 4.2 avec 0,11 g (0,53 mmole) de 2-bromo-1-(thién-2-yl)-éthanone dans 4 mL d'acétonitrile. On obtient 0,126 g du produit attendu sous la forme d'un solide blanc.
PF=316-318°C
R.M.N. ¹H (DMSO D₆), δ (ppm) : 10,8 (s, 1H); 9,5 (s, 1 H) ; 8,6 (s, 1 H) ; 7,8 (s, 2H) ; 7,7 (d, 1 H) ; 7,6 (m, 3H) ; 7,55 (s, 1 H) ; 7,35 (m, 1 H) ; 7,2 (m, 2H) ; 7,1 (m, 1 H) ; 6,95 (m, 2H) ; 5,9 (s, 2H).
LC-MS: 485 ([M+H]+, *R*t= 1,16 min.).

### Exemple 7 (Composé N°7)

### N-(2-tert-Butyl-imidazo[1,2-a]pyridin-6-yl)-5-fluoro-1-[(3-fluorophényl)méthyl]-1H-indole-2- carboxamide

Le composé N° 7 a été préparé selon un procédé similaire à celui décrit à l'étape 4.3 en faisant réagir 0,1 g (0,26 mmole) de *N*-[6-aminopyridin-3-yl]-5-fluoro-1-[(3-fluorophényl)méthyl]-1*H*-indole-2-carboxamide préparé selon le protocole décrit à l'étape 4.2 avec 0,096 g (0,53 mmole) de 1-bromo-3,3-diméthyl-butan-2-one dans 4 mL d'acétonitrile. On obtient 0,119 g du produit attendu sous la forme d'un solide blanc.
PF = 190 - 192 °C
R.M.N. ¹H (DMSO D₆), δ (ppm) : 10,9 (s, 1H) ; 9,6 (s, 1H) ; 8,3 (s, 1H) ; 8,0 (m, 2H) ; 7,65 (m, 2H) ; 7,55 (s, 1 H) ; 7,35 (m, 1 H) ; 7,25 (m, 1 H) ; 7,1 (m, 1 H) ; 6,95 (m, 2H) ; 5,9 (s, 2H) ; 1,4 (s, 9H).
LC-MS: 459 ([M+H]+, *R*t= 1,17 min.).

### Exemple 8 (Composé N°8)

### N-(2-Méthoxyméthyl-imidazo[1,2-a]pyridin-7-yl)-5-fluoro-1-[(3-fluorophényl)méthyl]-1H-indole-2- carboxamide

### 8.1 7-Bromo-imidazo[1,2-a]pyridine-2-carboxylate d'éthyle (Composé N° IIIc)

Dans un tube à vis de 20 mL muni d'un barreau magnétique, sont introduits 0,6 g (3,47 mmole) de 2-amino-4-bromopyridine et 1,55 g (7,15 mmoles) d'éthyl bromopyruvate et 4 mL d'acétonitrile. Le tube est ensuite vissé et agité à température ambiante pendant 1 heure puis mis à chauffer à 150°C sous agitation pendant 30 minutes. Après ce temps et retour à température ambiante, le précipité obtenu est filtré, rincé à l'acétonitrile et séché. Le filtrat est concentré à sec puis purifié par chromatographie sur colonne de gel de silice en éluant avec un mélange de dichlorométhane et de méthanol. Le solide obtenu après évaporation du solvant est séché sous pression réduite. On obtient en tout 530 mg du produit attendu.
R.M.N. ¹H (DMSO-D₆), δ ppm : 8,65 (s, 1 H) ; 8,6 (d, 1 H) ; 8,05 (s, 1 H) ; 7,3 (m, 1H) ; 4,3 (q, 2H) ; 1,3 (t, 3H).

### 8.2 (7-Bromo-imidazo[1,2-a]pyridin-2yl)méthanol (Composé N° IIId)

A une suspension de 0,25 g (0,93 mmole) de 7-bromo-imidazo[1,2-a]pyridine-2-carboxylate d'éthyle préparé selon le protocole décrit à l'étape 8.1, dans 4 mL de dichlorométhane anhydre, agitée à -19°C sous atmosphère inerte, sont ajoutés goutte à goutte, 2,09 mL (2,09 mmoles) d'une solution molaire de DIBAL-H dans le toluène. Le mélange réactionnel est agité à -19°C pendant 3 heures puis hydrolysé à -40°C par ajouts successifs de 0,1 mL de méthanol, 0,1 mL d'eau et 10 mL de HCl 5N. Le mélange réactionnel est ensuite basifié avec une solution aqueuse de soude (24%) puis extrait avec trois fois 100 mL de dichlorométhane. Les phases organiques rassemblées sont séchées sur sulfate de sodium puis concentrées sous pression réduite. Le brut réactionnel est ensuite purifié par chromatographie sur colonne de gel de silice en éluant avec un mélange de dichlorométhane et de méthanol. On obtient ainsi 110 mg du produit attendu.
R.M.N. ¹H (DMSO-D₆), δ ppm : 8,0 (d, 1H) ; 7,8 (s, 1H) ; 7,6 (s, 1H) ; 6,9 (m, 1H) ; 4,9 (s, 2H).

### 8.3 7-Bromo-2-chlorométhyl-imidazo[1,2-a]pyridine (Composé N° (IIIe)

A une solution de 110 mg (0,48 mmole) de (7-bromo-imidazo[1,2-a]pyridin-2yl)méthanol préparé selon le protocole décrit à l'étape 8.2, dans 8 mL de dichlorométhane anhydre, agitée à température ambiante sous atmosphère inerte, est ajouté 0,03 mL (0,53 mmole) de chlorure de thionyle. Le mélange réactionnel est agité à température ambiante pendant 4 heures, hydrolysé à 0°C par ajouts successifs de 3 mL d'eau froide et de 5 mL d'une solution aqueuse saturée en NaHCO₃ puis extrait avec trois fois 10 mL de dichlorométhane. Les phases organiques rassemblées sont séchées sur sulfate de sodium puis concentrées sous pression réduite. On obtient 80 mg du produit attendu.
R.M.N. ¹H (CDCl₃), δ ppm : 7,9 (d, 1H) ; 7,7 (s, 1H) ; 7,6 (s, 1 H) ; 6,85 (m, 1 H) ; 4,7 (s, 2H).

### 8.4 7-Bromo-2-méthoxyméthyl-imidazo[1,2-a]pyridine (Composé N° (IIIf)

A une solution de 81 mg (0,33 mmole) de 7-bromo-2-chlorométhyl-imidazo[1,2-*a*]pyridine préparé selon le protocole décrit à l'étape 8.3, dans 3 mL de méthanol anhydre, agitée à température ambiante sous atmosphère inerte, est ajouté 35 mg (0,99 mmole) de méthoxide de sodium. Le mélange réactionnel est agité à 100°C pendant 4 heures. Après ce temps, le milieu réactionnel, refroidi à température ambiante est concentré sous pression réduite, dilué avec 10 mL de chloroforme puis lavé avec 10mL d'une solution aqueuse saturée en NaCl. Le brut réactionnel obtenu après évaporation du solvant sous pression réduite est purifié par chromatographie sur colonne de gel de silice en éluant avec un mélange de dichlorométhane et de méthanol. On obtient ainsi 27 mg du produit attendu.
R.M.N. ¹H (CDCl₃), δ ppm: 7,9 (d, 1 H) ; 7,75 (s, 1H) ; 7,5 (s, 1 H) ; 6,85 (m, 1 H) ; 4,6 (s, 2H) ; 3,4 (s, 3H).

### 8.5 N-(2-Méthoxyméthyl-imidazo[1,2-a]pyridin-7-yl)-5-fluoro-1-[(3-fluorophényl)méthyl]-1H-indole-2-carboxamide (composé N° 8)

Dans un tube à pression de 10 mL spécifique pour microonde, sont introduits 20 mg (0,09 mmole) d'iodure de cuivre, 40 mg (0,14 mmole) de 5-fluoro-1-[(3-fluorophényl)méthyl]-1*H*-indole-2-carboxamide préparé selon le protocole décrit à l'étape 1.2, 27 mg (0,11 mmole) de 7-Bromo-2-méthoxyméthyl-imidazo[1,2-a]pyridine, préparé selon le protocole décrit à l'étape 8.3, 50 mg (0,36 mmole) de carbonate de potassium et 3 mL de dioxane anhydre. La suspension est dégazée quelques minutes puis on ajoute 0,01 mL (0,04 mmole) de trans-1,2-cyclohexanediamine. Le tube est ensuite scellé et mis à chauffer au micro-onde à 150°C pendant deux cycles de 1 h. Après ce temps, la suspension refroidie à température ambiante est versée dans 20 mL d'eau puis extrait avec 2x30 mL de dichlororométhane. Les phases organiques rassemblées sont séchées sur sulfate de magnésium et concentrées sous pression réduite. Le brut réactionnel est ensuite purifié par chromatographie sur colonne de gel de silice en éluant avec un mélange de dichlorométhane et de méthanol. On obtient ainsi 45 mg du produit attendu.
PF : 215-216 °C
R.M.N. ¹H (DMSO-D₆), δ ppm : 10,7 (s, 1 H) ; 8,5 (d, 1 H) ; 8,0 (s, 1 H) ; 7,8 (s, 1 H) ; 7,6 (m, 2H) ; 7,5 (s, 1 H) ; 7,3 (m, 1 H) ; 7,2 (m, 2H) ; 7,05 (m, 1 H) ; 6,9 (m, 2H) ; 5,9 (s, 2H) ; 4,5 (s, 2H) ; 3,3 (s, 3H).
LC-MS: 445 ([M-H]-, *R*t= 4,3 min.).

### Exemple 9 (Composé N°9)

### N-[2-(Hydroxyméthyl)-imidazo[1,2-a]pyridin-7-yl]-5-fluoro-1-[(3-fluorophényl)méthyl]-1H-indole-2-carboxamide

### 9.1 N-[2-(Ethyloxycarbonyl)-imidazo[1,2-a]pyridin-7-yl]-5-fluoro-1-[(3-fluorophényl)méthyl]-1H-indole-2-carboxamide

Le composé est préparé selon un procédé similaire à celui décrit à l'étape 4.3 en faisant réagir 0,2 g (0,53 mmole) de *N*-[2-amino-pyridin-4-yl]-5-fluoro-1-[(3-fluorophényl)méthyl]-1*H*-indole-2-carboxamide préparé selon le protocole décrit à l'étape 10.1 avec 0,21 g (1,06 mmole) de 3-bromo-2-oxo-propionate d'éthyle dans 8 mL d'acétonitrile. On obtient 70 mg du produit attendu.
LC-MS: 475 ([M+H]+, *R*t= 1,09 min.).
R.M.N. ¹H (DMSO D₆), δ (ppm) : 10,75 (s, 1H) ; 8,5 (m, 2H) ; 7,9 (s, 1H) ; 7,65 (m, 2H) ; 7,5 (s, 1 H) ; 7,35 (m, 2H) ; 7,2 (m, 1 H) ; 7,1 (m, 1 H) ; 6,9 (m, 2H) ; 5,9 (s, 2H) ; 4,3 (q, 2H) ; 1,3 (t, 3H).

### 9.2 N-[2-(Hydroxyméthyl)-imidazo[1,2-a]pyridin-7-yl]-5-fluoro-1-[(3-fluorophényl)méthyl]-1H-indole-2- carboxamide (composé N° 9)

Le composé N° 9 a été préparé selon un procédé similaire à celui décrit à l'étape 8.2 en faisant réagir 0,465 g (0,98 mmole) de *N*-[2-(éthyloxycarbonyl)-imidazo[1,2-*a*]pyridin-7-yl]-5-fluoro-1-[(3-fluorophényl)méthyl]-1*H*-indole-2-carboxamide préparé selon le protocole décrit à l'étape 9.1 avec 2,21 mL (2,21 mmoles) d'une solution molaire de DIBAL-H dans le toluène dans 4 mL de dichlorométhane. On obtient 80 mg du produit attendu.
PF = 244 - 245 °C
R.M.N. ¹H (DMSO D₆), δ (ppm) : 10,6 (s, 1H) ; 8,48 (d, 1H) ; 8,0 (s, 1H) ; 7,7 (s, 1H) ; 7,6 (m, 2H) ; 7,5 (s, 1 H) ; 7,35 (m, 1 H) ; 7,2 (m, 2H) ; 7,05 (m, 1 H) ; 6,9 (m, 2H) ; 5,9 (s, 2H) ; 5,1 (t, 1 H) ; 4,6 (d, 2H).
LC-MS: 433 ([M+H]+, *R*t= 1,06 min.).

### Exemple 10 (Composé N°10)

### N-(2-Méthyl-3-phényl-imidazo[1,2-a]pyridin-7-yl)-5-fluoro-1-[(3-fluorophényl)méthyl]-1H-indole-2- carboxamide

### 10.1 N-[2-amino-pyridin-4-yl]-5-fluoro-1-[(3-fluorophényl)méthyl]-1H-indole-2-carboxamide

Dans un tube à pression de 10 mL spécifique pour microonde, sont introduits 0,2 g (1,05 mmole) d'iodure de cuivre, 0,6 g (2,10 mmoles) de 5-fluoro-1-[(3-fluorophényl)méthyl]-1*H*-indole-2-carboxamide préparé selon le protocole décrit à l'étape 1.2, 0,4 g (2,31 mmoles) de 2-amino-4-bromopyridine, 1,16 g (8,40 mmoles) de carbonate de potassium et 15 mL de dioxane anhydre. La suspension est dégazée quelques minutes puis on ajoute 0,14 mL (1,15 mmole) de trans-1,2-cyclohexanediamine. Le tube est ensuite scellé et mis à chauffer au micro-onde à 170°C pendant deux cycles de 1 h. Après ce temps, la suspension refroidie à température ambiante est versée dans 50 mL d'eau puis extrait avec 2x75 mL de dichlororométhane. Les phases organiques rassemblées sont séchées sur sulfate de magnésium et concentrées sous pression réduite. Le brut réactionnel est ensuite purifié par chromatographie sur colonne de gel de silice en éluant avec un mélange de dichlorométhane et de méthanol. On obtient ainsi 0,425 g du produit attendu sous la forme d'un solide beige.
PF:217-218 °C
R.M.N. ¹H (DMSO-D₆), δ ppm : 10,4 (s, 1H) ; 7,8 (d, 1H) ; 7,6 (m, 2H) ; 7,45 (s, 1H) ; 7,35 (m, 1 H) ; 7,2 (m, 1 H) ; 7,1 (m, 2H) ; 6,9 (m, 2H) ; 6,8 (d, 1 H) ; 5,9 (m, 4H). LC-MS: 379 ([M+H]+, *R*t= 1,03 min.).

### 10.2 N-(2-Méthyl-3-phényl-imidazo[1,2-a]pyridin-7-yl)-5-fluoro-1-[3-fluorophényl)méthyl]-1H-indole-2- carboxamide (composé N°10)

Dans un tube à pression de 10 mL spécifique pour microonde, sont introduits 0,1 g (0,26 mmole) de *N*-2-amino-pyridin-4-yl]-5-fluoro-1-[(3-fluorophényl)méthyl]-1*H*-indole-2-carboxamide préparé selon le protocole décrit à l'étape 10.1, 0,11 g (0,53 mmole) de 2-bromo-1-phényl-propan-1-one et 4 mL d'acétonitrile. Le tube est ensuite scellé et mis à chauffer au micro-onde à 150°C pendant deux cycles de 1 h. Après ce temps, le mélange réactionnel est concentré sous pression réduite. Le brut réactionnel est ensuite purifié par chromatographie sur colonne de gel de silice en éluant avec un mélange de dichlorométhane et de méthanol. On obtient ainsi 0,03 g du produit attendu sous la forme d'un solide beige.
PF : 305 - 307 °C
R.M.N. ¹H (DMSO-D₆), δ ppm : 10,7 (s, 1H) ; 8,35 (d, 1H) ; 8,1 (s, 1H) ; 7,85 (d, 2H) ; 7,6 (m, 2H) ; 7,5 (m, 3H) ; 7,35 (m, 3H) ; 7,2 (m, 1 H) ; 7,1 (m, 1 H) ; 6,9 (m, 2H) ; 5,9 (s, 2H) ; 2,7 (s, 3H).
LC-MS: 493 ([M+H]+, *R*t= 1,17 min.).

### Exemple 11 (Composé N°11)

### N-[2-(Thién-2-yl)-imidazo[1,2-a]pyridin-7-yl]-5-fluoro-1-[(3-fluorophényl)méthyl]-1H-indole-2- carboxamide

Le composé N° 11 a été préparé selon un procédé similaire à celui décrit à l'étape 10.2 en faisant réagir 0,1 g (0,26 mmole) de *N*-[2-amino-pyridin-4-yl]-5-fluoro-1-[(3-fluorophényl)méthyl]-1*H*-indole-2-carboxamide préparé selon le protocole décrit à l'étape 10.1 avec 0,11 g (0,53 mmole) de 2-bromo-1-(thién-2-yl)-éthanone dans 4 mL d'acétonitrile. On obtient ainsi 0,026 g du produit attendu sous la forme d'un solide beige.
PF : 259 - 260 °C
R.M.N. ¹H (DMSO-D₆), δ ppm : 10,7 (s, 1 H) ; 8,5 (d, 1 H) ; 8,2 (s, 1 H) ; 8,0 (s, 1 H) ; 7,6 (m, 2H) ; 7,5 (m, 3H) ; 7,3 (m, 2H) ; 7,2 (m, 1H) ; 7,15 (m, 1 H) ; 7,05 (m, 1H) ; 6,95 (m, 2H) ; 5,9 (s, 2H).
LC-MS: 485 ([M+H]+, *R*t= 1,13 min).

### Exemple 12 (Composé N°12)

### N-(2-éthyl-imidazo[1,2-a]pyridin-7-yl)-5-fluoro-1-[(3-fluorophényl)méthyl-1H-indole-2-carboxamide

Le composé N° 12 a été préparé selon un procédé similaire à celui décrit à l'étape 10.2 en faisant réagir 0,1 g (0,26 mmole) de *N*-[2-amino-pyridin-4-yl]-5-fluoro-1-[(3-fluorophényl)méthyl]-1*H*-indole-2-carboxamide préparé selon le protocole décrit à l'étape 10.1 avec 0,08 g (0,53 mmole) de 1-bromo-butan-2-one dans 4 mL d'acétonitrile. On obtient ainsi 66 mg du produit attendu.
PF : 158 - 159 °C
R.M.N. ¹H (DMSO D₆), δ (ppm) : 11,2 (s, 1 H) ; 8,65 (d, 1 H) ; 8,4 (s, 1 H) ; 7,9 (s, 1H) ; 7,55 (m, 4H) ; 7,35 (m, 1H) ; 7,2 (m, 1 H) ; 7,05 (m, 1 H) ; 6,9 (d, 1H) ; 6,8 (d, 1H) ; 5,9 (s, 2H) ; 2,8 (q, 2H) ; 1,25 (t, 3H).
LC-MS: 431 ([M+H]+, *R*t= 1,08 min.).

### Exemple 13 (Composé N°13)

### N-(2-tert-Butyl-imidazo[1,2-a]pyridin-7-yl)-5-fluoro-1-[(3-fluorophényl)méthyl-1H-indole-2- carboxamide

Le composé N° 13 a été préparé selon un procédé similaire à celui décrit à l'étape 10.2 en faisant réagir 0,1 g (0,26 mmole) de *N*-[2-amino-pyridin-4-yl]-5-fluoro-1-[(3-fluorophényl)méthyl]-1*H*-indole-2-carboxamide préparé selon le protocole décrit à l'étape 10.1 avec 0,096 g (0,53 mmole) de 1-bromo-3,3-diméthyl-butan-2-one dans 4 mL d'acétonitrile. On obtient ainsi 0,116 g du produit attendu.
PF : 327 - 328 °C
R.M.N. ¹H (DMSO D₆), δ (ppm) : 11,2 (s, 1H) ; 8,7 (d, 1H) ; 8,45 (s, 1H) ; 8,0 (s, 1H) ; 7,7 (m, 4H) ; 7,35 (m, 1H) ; 7,25 (m, 1 H) ; 7,1 (m, 1H) ; 6,9 (m, 2H) ; 5,9 (s, 2H) ; 1,4 (s, 9H).
LC-MS: 459 ([M+H]+, *R*t= 1,14 min.).

### Exemple 14 (Composé N°14)

### N-(2,3-Diméthyl-imidazo[1,2-a]pyridin-7-yl)-5-trifluorométhyl-1-[(3-fluorophényl)méthyl]-1H-indole-2-carboxamide

### 14.1 2,3-Diméthyl-imidazo[1,2-a]pyridin-7-carboxylate de méthyle (Composé N° (IIIb)

A une suspension de 5 g (32,86 mmoles) de 2-aminopyridine-4-carboxylate de méthyle et de 5,52 g (65,72 mmoles) d'hydrogénocarbonate de sodium dans 250 ml d'éthanol, on ajoute 12,4 g (82,15 mmoles) de 3-bromo-2-butanone. Le mélange réactionnel est agité à reflux pendant 2 heures. On rajoute ensuite au mélange réactionnel 12,4 g (82,15 mmoles) de 3-bromo-2-butanone supplémentaire et on continue l'agitation à reflux pendant 15 heures.
Après retour à température ambiante, le solvant est évaporé. Le résidu est dilué avec 50 mL d'eau puis basifié par ajout de NaHCO₃ saturé jusqu'à pH 8-9. Le précipité formé est filtré, rincé plusieurs fois à l'eau puis séché.
On obtient 4 g de produit attendu sous la forme d'un solide beige.
RMN (DMSO-D₆) δ ppm : 8,3 (d, 1 H) ; 8,05 (s, 1 H) ; 7,3 (dd, 1H) ; 3,9(s, 3H) ; 2,5 (s, 3H) ; 2,35 (s, 3H).

### 14.2 Acide 2,3-diméthyl-imidazo[1,2-a]pyridin-7-carboxylique

A une solution de 1,85 g (9,06 mmoles) de 2,3-diméthyl-imidazo[1,2-a]pyridine-7-carboxylate de méthyle obtenu selon le protocole décrit à l'étape 14.1 dans 50 mL de méthanol, on ajoute une solution de 0,77 g (13,59 mmoles) d'hydroxyde de potassium dans 1,5 mL d'eau. On porte le mélange réactionnel à 50°C pendant 18 heures. Après retour à température ambiante, la solution est refroidie à 0°C et le pH du milieu réactionnel est ajusté à pH 5 par ajout goutte à goutte de HCl 6N. Le précipité qui se forme est filtré, rincé à l'eau puis à l'éther pour donner quantitativement l'acide attendu. LC-MS: 191 ([M+H]+, *R*t= 0,44 min.).

### 14.3 N-[2,3-Diméthyl-imidazo[1,2-a]pyridin-7-yl]carbamate de tert-butyle

A une suspension de 1,7 g (8,94 mmoles) d'acide 2,3-diméthyl-imidazo[1,2-*a*]pyridin-7-carboxylique, obtenu selon le protocole décrit dans l'étape 14.2, dans 29 ml de tert-butanol, on ajoute 3,19 g (11,62 mmoles) de diphénylphosphorylazide et 3,11 mL (22,34 mmoles) de triéthylamine. Après 4 heures de chauffage à 90°C et 12 heures à 40°C, le milieu réactionnel est concentré à sec puis dilué avec 20 ml d'eau. Le précipité obtenu est filtré, rincé plusieurs fois à l'eau puis séché. Une purification par chromatographie flash sur gel de silice en éluant avec un gradient de 1 à 5 % de méthanol dans le dichlorométhane conduit à 1,15 g de produit attendu sous la forme d'un solide jaune.
RMN (DMSO-D₆) δ ppm : 7,8 (d, 1H) ; 7,5-7,2 (m, 2H) ; 2,4 (2s, 6H) ; 1,6 (s, 9H).

### 14.4 Chlorhydrate de 2,3-diméthyl-imidazo[1,2-a]pyridin-7-yl-amine

A une solution de 1,15 g (4,40 mmoles) de 2,3-diméthyl-imidazo[1,2-a]pyridin-7-yl)-carbamate de tert-butyle obtenu selon le protocole décrit à l'étape 14.3 dans 15 ml de dioxane, on ajoute 15,4 ml (61,61 mmoles) d'une solution de HCl 4N dans le dioxane. On porte le mélange réactionnel à 55°C pendant 4 heures. Après retour à température ambiante, le mélange réactionnel est dilué à l'éther diéthylique sous agitation. Le précipité formé est filtré, rincé à l'éther puis séché sous pression réduite. On obtient 0,9 g du chlorhydrate de 2,3-diméthyl-imidazo[1,2-a]pyridin-7-yl-amine attendu.
RMN (DMSO-D₆) δ ppm : 13,2 (s, 1 H) ; 8,2 (d, 1 H) ; 6,8 (d, 1 H) ; 6,55 (s, 1 H) ; 2,3 (s, 3H) ; 2,25 (s, 3H).

### 14.5 N-(2,3-Diméthyl-imidazo[1,2-a]pyridin-7-yl)-5-trifluorométhyl-1-[(3-fluorophényl)méthyl]-1H-indole-2-carboxamide (composé N° 14)

A une solution, agitée à 20°C sous atmosphère inerte, de 1 g (2,96 mmoles) de l'acide 5-trifluorométhyl-1-[(3-fluorophényl)méthyl]-1*H*-indole-2-carboxylique
(WO2006072736 dans 70 mL de DMF, sont ajoutés, 0,74 g (3,56 mmoles) de dicyclohexylcarbodiimide (DCC) et 0,48 g (3,56 mmoles) de 1-hydroxy-benzotriazole hydrate (HOBT). Le mélange réactionnel est agité à température ambiante pendant 30 minutes. On ajoute ensuite au mélange réactionnel, 0,7 g (4,45 mmoles) de 2,3-diméthyl-imidazo[1,2-a]pyridin-7-ylamine, obtenu selon le protocole décrit à l'étape 14.4, et 100 mg de diméthylaminopyridine (DMAP). Après 12 heures d'agitation à 50°C, le mélange réactionnel est concentré à sec. Le résidu obtenu est repris avec un mélange de 50 mL d'eau et de 50 mL d'acétate d'éthyle. On décante et on extrait la phase aqueuse par 200 mL d'acétate d'éthyle. Les phases organiques rassemblées sont séchées sur sulfate de sodium puis concentrées sous pression réduite. Le produit est isolé après purifications successives par chromatographie sur colonne de silice en éluant avec un mélange de dichlorométhane et de méthanol. On obtient ainsi 50 mg du produit attendu.
PF = 280 - 281°C
R.M.N. ¹H (DMSO-D₆), δ ppm : 10,75 (s, 1H) ; 8,25 (s, 1H) ; 8,15 (d, 1H) ; 7,95 (s, 1H) ; 7,8 (d, 1H) ; 7,6 (m, 2H) ; 7,3 (m, 2H) ; 7,1 (m, 1H) ; 6,9 (m, 2H) ; 5,95 (s, 2H) ; 2,4 (s, 3H) ; 2,3 (s, 3H).
LC-MS: 481 ([M+H]+, *R*t= 1,18 min.).

### Exemple 15 (Composé N°15)

### N-(2-Ethyl-imidazo[1,2-a]pyridin-7-yl)-5-trifluorométhyl-1-[(3-fluorophényl)méthyl]-1H-pyrrolo[2,3-b]pyridine-2- carboxamide

### 15.1 5-trifluorométhyl-1-[(3-fluorophényl)méthyl]-1H-pyrrolo[2,3-b]pyridine-2-carboxamide

A une suspension, agitée à 20°C, de 1,5 g (4,43 mmoles) d'acide 5-trifluorométhyl-1-[(3-fluorophényl)méthyl]-1*H*-pyrrolo[2,3-b]pyridine-2-carboxylique (WO2008/093024), dans 40 mL de toluène sec, sont ajoutés 3,2 mL (44,34 mmoles) de chlorure de thionyle. Le mélange réactionnel est agité 2 heures à reflux puis est concentré sous pression réduite. Le produit résultant est repris par 20 mL de dichlorométhane et on verse cette solution, goutte à goutte, sur une solution de 5,81 mL (44,34 mmoles) d'ammoniaque à 30% dans l'eau. Le mélange réactionnel est agité 14 heures à 20°C. Après ce temps, on recueille, par filtration, un solide que l'on triture dans 50 mL d'éther diisopropylique. On recueille après filtration et séchage sous pression réduite 1,5 g de produit attendu.
PF : 203 - 204 °C
R.M.N. ¹H (DMSO-D₆), δ ppm : 8,78 (d, 1H) ; 8,65 (d, 1H) ; 8,29 (pic élargi, 1H) ; 7,62 (pic élargi, 1 H) ; 7,4 (s, 1 H) ; 7,32 (m, 1 H) ; 7,05 (m, 1 H) ; 6,92 (m, 2H) ; 6,0 (s, 2H). LC-MS: 338 ([M+H]+, *R*t= 1,20 min.).

### 15.2 N-[2-Aminopyridin-4-yl]-5-trifluorométhyl-1-[(3-fluorophényl)méthyl]-1H-pyrrolo[2,3-b]pyridine-2-carboxamide

Dans un tube à pression de 10 mL spécifique pour microonde, sont introduits 0,056 g (0,30 mmole) d'iodure de cuivre, 0,2 g (0,59 mmole) de 5-trifluorométhyl-1-[(3-fluorophényl)méthyl]-1*H*-pyrrolo[2,3-b]pyridine-2-carboxamide préparé selon le protocole décrit à l'étape 15.1, 0,11 g (0,65 mmole) de 2-amino-4-bromopyridine, 0,33 g (2,37 mmoles) de carbonate de potassium et 5 mL de dioxane anhydre. La suspension est dégazée quelques minutes puis on ajoute 0,04 mL (0,33 mmole) de trans-1,2-cyclohexanediamine. Le tube est ensuite scellé et mis à chauffer au micro-onde à 170°C pendant 45 minutes. Après ce temps, la suspension refroidie à température ambiante est versée dans 30 mL d'eau puis extrait avec 2x40 mL de dichlororométhane. Les phases organiques rassemblées sont séchées sur sulfate de magnésium et concentrées sous pression réduite. Le brut réactionnel est ensuite purifié par chromatographie sur colonne de gel de silice en éluant avec un mélange de dichlorométhane et de méthanol. On obtient ainsi 0,22 g du produit attendu sous la forme d'un solide blanc.
PF:197-198°C
R.M.N. ¹H (DMSO-D₆), δ ppm : 10,6 (s, 1 H) ; 8,85 (s, 1 H) ; 8,75 (s, 1 H) ; 7,85 (d, 1H) ; 7,65 (s, 1 H) ; 7,35 (m, 1 H) ; 7,1 (m, 2H) ; 6,9 (m, 2H) ; 6,8 (m, 1H) ; 5,95 (s, 2H) ; 5,85 (s, 2H).
LC-MS: 430 ([M+H]+, *R*t= 1,08 min.).

### 15.3 N-(2-Ethyl-imidazo[1,2-a]pyridin-7-yl)-5-trifluorométhyl-1-[(3-fluorophényl)méthyl]-1H-pyrrolo[2,3-b]pyridine-2-carboxamide (composé N° 15)

Dans un tube à pression de 10 mL spécifique pour microonde, sont introduits 0,125 g (0,29 mmole) de *N*-[2-aminopyridin-4-yl]-5-trifluorométhyl-1-[(3-fluorophényl)méthyl]-1*H*-pyrrolo[2,3-b]pyridine-2-carboxamide préparé selon le protocole décrit à l'étape 15.2, 0,088 g (0,58 mmole) de 1-bromo-butan-2-one et 4 mL d'acétonitrile. Le tube est ensuite scellé et mis à chauffer au micro-onde à 150°C pendant deux cycles de 1 h. Après ce temps, le mélange réactionnel est concentré sous pression réduite. Le brut réactionnel est ensuite purifié par chromatographie sur colonne de gel de silice en éluant avec un mélange de dichlorométhane et de méthanol. On obtient ainsi 0,076 g du produit attendu sous la forme d'un solide jaune.
PF : 264 - 265 °C
R.M.N. ¹H (DMSO-D₆), δ ppm : 10,8 (s, 1H) ; 8,85 (s, 1 H) ; 8,8 (s, 1 H) ; 8,45 (d, 1H) ; 7,95 (s, 1 H) ; 7,65 (m, 2H) ; 7,35 (m, 1 H) ; 7, 2 (d, 1 H) ; 7,1 (m, 1 H) ; 6,95 (m, 2H) ; 6,0 (s, 2H) ; 2,7 (q, 2H) ; 1,3 (t, 3H).
LC-MS: 482 ([M+H]+, *R*t= 4,75 min.).

### Exemple 16 (Composé N°16)

### N-(2,3-Diméthyl-imidazo[1,2-a]pyridin-7-yl)-5-trifluorométhyl-1-[(3-fluorophényl)méthyl]--1H-pyrrolo[2,3-b]pyridine-2-carboxamide

Le composé N° 16 a été préparé selon un procédé similaire à celui décrit à l'étape 15.3 en faisant réagir 0,12 g (0,28 mmole) de *N*-[2-aminopyridin-4-yl]-5-trifluorométhyl-1-[(3-fluorophényl)méthyl]-1*H*-pyrrolo[2,3-b]pyridine-2-carboxamide préparé selon le protocole décrit à l'étape 15.2 avec 0,084 g (0,56 mmole) de 3-bromo-butan-2-one dans 5 mL d'acétonitrile. On obtient 45 mg du produit attendu.
PF = 287 - 288 °C
R.M.N. ¹H (DMSO-D₆), δ ppm : 10,8 (s, 1 H) ; 8,85 (s, 1 H) ; 8,8 (s, 1 H) ; 8,2 (d, 1 H) ; 8,0 (s, 1H) ; 7,65 (m, 1 H) ; 7,35 (m, 1 H) ; 7, 28 (m, 1 H) ; 7,1 (m, 1 H) ; 6,95 (m, 2H) ; 6,0 (s, 2H) ; 2,4 (s, 3H) ; 2,3 (s, 3H).
LC-MS: 482 ([M+H]+, *R*t= 1,13 min.).

### Exemple 17 (Composé N°17)

### N-[2-(hydroxyméthyl)-imidazo[1,2-a]pyridin-7-yl]-5-trifluorométhyl-1-[(3-fluorophényl)méthyl]-1H-indole-2-carboxamide

### 17.1 5-trifluorométhyl-1-[(3-fluorophényl)méthyl]-1H-indole-2-carboxamide

A une suspension, agitée à 20°C, de 8 g (23,72 mmoles) d'acide 5-trifluorométhyl-1-(3-fluorobenzyl)-1*H* indole-2-carboxylique (WO2006072736), dans 150 mL de toluène sec, sont ajoutés 17,3 mL (237,2 mmoles) de chlorure de thionyle. Le mélange réactionnel est agité 2 heures à reflux puis est concentré sous pression réduite. Le produit résultant est repris par 25 mL de dichlorométhane et on verse cette solution, goutte à goutte, sur une solution de 9,32 mL d'ammoniaque à 30% dans l'eau. Le mélange réactionnel est agité 14 heures à 20°C. Après ce temps, on recueille, par filtration, un solide que l'on triture dans 50 mL de pentane. On recueille après filtration et séchage sous pression réduite 5,87 g de produit attendu.
R.M.N. ¹H (DMSO-D₆), δ ppm : 8,28 (pic élargi, 1H) ; 8,13 (s, 1H) ; 7,77 (d, 1H) ; 7,6 (m, 2H) ; 7,41 (s, 1 H) ; 7,32 (m, 1 H) ; 7,05 (m, 1 H) ; 6,9 (m, 2H) ; 6 (s, 2H).

### 17.2 N-[2-Aminopyridin-4-yl]-5-trifluorométhyl-1-[(3-fluorophényl)méthyl]-1H-indole-2-carboxamide

Dans un tube à pression de 10 mL spécifique pour microonde, sont introduits 0,056 g (0,30 mmole) d'iodure de cuivre, 0,2 g (0,59 mmoles) de 5-trifluorométhyl-1-[(3-fluorophényl)méthyl]-1*H*-indole-2-carboxamide préparé selon le protocole décrit à l'étape 17.1, 0,12 g (0,71 mmole) de 2-amino-4-bromopyridine, 0,33 g (2,38 mmoles) de carbonate de potassium et 4 mL de dioxane anhydre. La suspension est dégazée quelques minutes puis on ajoute 0,04 mL (0,3 mmole) de trans-1,2-cyclohexanediamine. Le tube est ensuite scellé et mis à chauffer au micro-onde à 170°C pendant 45 minutes. Après ce temps, la suspension refroidie à température ambiante est versée dans 30 mL d'eau puis extrait avec 2x40 mL de dichlororométhane. Les phases organiques rassemblées sont séchées sur sulfate de magnésium et concentrées sous pression réduite. Le brut réactionnel est ensuite purifié par chromatographie sur colonne de gel de silice en éluant avec un mélange de dichlorométhane et de méthanol. On obtient ainsi 0,186 g du produit attendu.
PF : 225 - 226 °C
R.M.N. ¹H (DMSO-D₆), δ ppm : 10,6 (s, 1H) ; 8,25 (s, 1H) ; 7,8 (m, 2H) ; 7,6 (m, 2H) ; 7,35 (m, 1 H) ; 7,1 (m, 2H) ; 6,9 (m, 2H) ; 6,8 (d, 1 H) ; 5,9 (s, 2H) ; 5,85 (s, 2H). LC-MS: 429 ([M+H]+, *R*t= 4,55 min.).

### 17.3 N-[2-(Ethyloxycarbonyl)-imidazo[1,2-a]pyridin-7-yl]-5-trifluorométhyl-1-[(3-fluorophényl)méthyl]-1H-indole-2-carboxamide

Le composé est préparé selon un procédé similaire à celui décrit à l'étape 15.3 en faisant réagir 0,23 g (0,54 mmole) de *N*-[2-Aminopyridin-4.-yl]-5-trifluorométhyl-1-[(3-fluorophényl)méthyl]-1*H*-indole-2-carboxamide préparé selon le protocole décrit à l'étape 17.2 avec 0,21 g (1,07 mmole) de 3-bromo-2-oxo-propionate d'éthyle dans 4 mL d'acétonitrile. On obtient 192 mg du produit attendu.
PF = 282 - 283 °C
R.M.N. ¹H (DMSO D₆), δ (ppm) : 10,9 (s, 1H) ; 8,55 (d, 1H) ; 8,45 (s, 1H) ; 8,25 (s, 1H) ; 8,1 (s, 1H) ; 7,85 (d, 1H) ; 7,65 (s, 1H) ; 7,6 (d, 1 H) ; 7,35 (m, 2H) ; 7,1 (m, 1H) ; 6,95 (d, 2H) ; 6,0 (s, 2H) ; 4,3 (q, 2H) ; 1,3 (t, 3H).
LC-MS: 525 ([M+H]+, *R*t= 4,83 min.).

### 17.4 N-[2-(Hydroxyméthyl)-imidazo[1,2-a]pyridin-7-yl]-5-trifluorométhyl-1-[(3-fluorophényl)méthyl]-1H-indole-2-carboxamide (composé N° 17)

A 0,47 mL (0,47 mmole) d'une solution molaire de LiAlH4, dilué dans 1 mL de THF anhydre, refroidie à -5°C, est ajoutée goutte à goutte, à l'aide d'une seringue, une suspension de 190 mg (0,36 mmole) de *N*-[2-(éthyloxycarbonyl)-imidazo[1,2-a]pyridin-7-yl]-5-trifluorométhyl-1-[(3-fluorophényl)méthyl]-1*H*-indole-2-carboxamide préparé selon le protocole décrit à l'étape 17.3, dans 4 mL de THF anhydre. Après 3 heures d'agitation à température ambiante, le milieu réactionnel est lentement versé dans un mélange (v/v) d'eau et de soude concentré puis extrait avec 3x100 mL de dichlorométhane. Les phases organiques rassemblées sont séchées sur sulfate de magnésium puis concentrées sous pression réduite. Le brut réactionnel obtenu est purifié par chromatographie sur colonne de silice en éluant avec un mélange de dichlorométhane et de méthanol. On obtient ainsi 30 mg du produit attendu.
PF = 254 - 255 °C
R.M.N. ¹H (DMSO-D₆), δ ppm : 10,8 (s, 1H) ; 8,49 (d, 1H) ; 8,25 (s, 1H) ; 8,0 (s, 1H) ; 7,8 (d, 1 H) ; 7,7 (s, 2H) ; 7,6 (m, 2H) ; 7,3 (m, 1 H) ; 7,2 (m, 1 H) ; 7,05 (m, 1 H) ; 6,95 (d, 2H) ; 5,95 (s, 2H) ; 5,1 (m, 1 H) ; 4,55 (d, 2H).
LC-MS: 483 ([M+H]+, *R*t= 1,29 min.).

### Exemple 18 (Composé N°18)

### N-[2-(Hydroxyméthyl)-imidazo[1,2-a]pyridin-7-yl]-5-trifluorométhyl-1-[(3-fluorophényl)méthyl]-1H-pyrrolo[2,3-b]pyridine-2-carboxamide (composé 18)

### 18.1 N-[2-(Ethyloxycarbonyl)-imidazo[1,2-a]pyridin-7-yl]-5-trifluorométhyl-1-[(3-fluorophényl)méthyl]-1H-pyrrolo[2,3-b]pyridine-2-carboxamide

Le composé est préparé selon un procédé similaire à celui décrit à l'étape 15.3 en faisant réagir 0,197 g (0,46 mmole) de *N*-[2-aminopyridin-4-yl]-5-trifluorométhyl-1-[(3-fluorophényl)méthyl]-1*H*-pyrrolo[2,3-b]pyridine-2-carboxamide préparé selon le protocole décrit à l'étape 15.2 avec 0,179 g (0,92 mmole) de 3-bromo-2-oxo-propionate d'éthyle dans 5 mL d'acétonitrile. On obtient 130 mg du produit attendu.
R.M.N. ¹H (DMSO-D₆), δ ppm : 10,9 (s, 1 H) ; 8,9 (s, 1 H) ; 8,8 (s, 1 H) ; 8,5 (m, 2H) ; 8,1 (s, 1 H) ; 7,7 (s, 1 H) ; 7,35 (m, 2H) ; 7,1 (m, 1 H) ; 6,95 (m, 2H) ; 6,0 (s, 2H) ; 4,3 (q, 2H) ; 1,3 (t, 3H).

### 18.2 N-[2-(Hydroxyméthyl)-imidazo[1,2-a]pyridin-7-yl]-5-trifluorométhyl-1-[(3-fluorophényl)méthyl]-1H-pyrrolo[2,3-b]pyridine-2-carboxamide (composé N° 18)

Le composé N° 18 a été préparé selon un procédé similaire à celui décrit à l'étape 17.4 en faisant réagir 0,13 g (0,25 mmole) de *N*-[2-(Ethyloxycarbonyl)-imidazo[1,2-*a*]pyridin-7-yl]-5-trifluorométhyl-1-[(3-fluorophényl)méthyl]-1*H*-pyrrolo[2,3-b]pyridine-2-carboxamide préparé selon le protocole décrit à l'étape 18.1 avec 0,32 mL d'une solution molaire de LiAlH4 dans 4 mL de tetrahydrofurane. On obtient 22 mg du produit attendu.
PF = 238 - 240 °C
R.M.N. ¹H (DMSO-D₆), δ ppm : 10,8 (s, 1H) ; 8,85 (s, 1H) ; 8,8 (s, 1H) ; 8,45 (d, 1H) ; 8,0 (s, 1 H) ; 7,7 (s, 1 H) ; 7,65 (s, 1 H) ; 7,35 (m, 1 H) ; 7,2 (m, 1 H) ; 7,05 (m, 1 H) ; 6,95 (m, 2H) ; 6,0 (s, 2H) ; 5,15 (t, 1 H) ; 4,55 (d, 2H).
LC-MS: 484 ([M+H]+, *R*t= 1,13 min.).

### Exemple 19 (Composé N°19)

### N-(2-Méthyl-imidazo[1,2-a]pyridin-7-yl)-5-fluoro-1-[(3-fluorophényl)méthyl]-1H-indole-2-carboxamide

Le composé N° 19 a été préparé selon un procédé similaire à celui décrit à l'étape 10.2 en faisant réagir 0,12 g (0,32 mmole) de *N*-[2-amino-pyridin-4-yl]-5-fluoro-1-[(3-fluorophényl)méthyl]-1*H*-indole-2-carboxamide préparé selon le protocole décrit à l'étape 10.1 avec 0,059 g (0,63 mmole) de 1-chloro-propan-2-one dans 4 mL d'acétonitrile. On obtient ainsi 50 mg du produit attendu.
PF : 267 - 268 °C
R.M.N. ¹H (DMSO D₆), δ (ppm) : 10,6 (s, 1H) ; 8,4 (d, 1H) ; 7,95 (s, 1H) ; 7,6 (m, 3H) ; 7,45 (s, 1H) ; 7,35 (m, 1H) ; 7,2 (m, 2H) ; 7,05 (m, 1H) ; 6,9 (m, 2H) ; 5,9 (s, 2H) ; 2,3 (s, 3H).
LC-MS: 417 ([M+H]+, *R*t= 1,2 min.).

### Exemple 20 (Composé N°20)

### N-(2-Méthyl-pyrazolo[1,5-a]pyrimidin-6-yl)-5-fluoro-1-[(3-fluorophényl)méthyl]-1H-indole-2- carboxamide

On mélange, dans un tube à pression sous atmosphère inerte à 20°C, 80 mg (0,42 mmole) d'iodure de cuivre, 0,4 g (1,40 mmole) de 5-fluoro-1-[(3-fluorophényl)méthyl]-1*H*-indole-2-carboxamide, obtenu selon le protocole décrit à l'étape 1.2, 0,32 g (1,54 mmole) de 6-bromo-2-méthyl-pyrazolo[1,5-a]pyrimidine (WO2006128693), 0,38 g (2,79 mmoles) de carbonate de potassium et 10 mL de dioxane. La suspension est dégazée quelques minutes puis on ajoute 0,053 g (0,46 mmole) de trans-1,2-cyclohexanediamine et on ferme le tube. Le mélange réactionnel est agité 15 heures à 110 °C. Après ce temps, la suspension refroidie à température ambiante est versée dans 50 mL d'eau et 50 mL d'acétate d'éthyle. On extrait la phase aqueuse avec 2x30 mL d'acétate d'éthyle. Les phases organiques rassemblées sont séchées sur sulfate de magnésium et concentrées sous pression réduite. Le produit résultant est trituré dans un mélange bouillant de 5 mL de dichlorométhane et de 20 mL d'éther isopropylique puis filtré à chaud. Le solide obtenu est purifié par chromatographie sur colonne de silice en éluant avec un mélange de de n-heptane et d'acétate d'éthyle. On recueille après séchage 0,23 g du produit attendu sous la forme d'un solide blanc.
PF : 267 - 268 °C
R.M.N. ¹H (DMSO-D₆), δ ppm : 10,8 (s, 1H) ; 9,4 (s, 1 H) ; 8,7 (s, 1 H) ; 7,65 (m, 2H) ; 7,5 (s, 1 H) ; 7,35 (m, 1 H) ; 7,2 (m, 1 H) ; 7,05 (m, 1H) ; 6,95 (m, 2H) ; 6,55 (s, 1 H) ; 5,9 (s, 2H) ; 2,4 (s, 3H).
LC-MS: 418 ([M+H]+, *R*t= 1,26 min.).

### Exemple 21 (Composé N°21)

### N-[2-(Ethyloxycarbonyl)-imidazo[1,2-b]pyridazin-6-yl]-5-fluoro-1-[(3-fluorophényl)méthyl]-1H-indole-2- carboxamide

### 21.1 6-bromo-imidazo[1,2-b]pyridazine-2-carboxylate d'éthyle

A une solution de 2,0 g (11,49 mmoles) de 3-amino-5-bromopyridazine dans 200 mL d'éthanol absolu est ajouté lentement 2,73 g (1,77 mmole) de 3-bromo-2-oxo-propionate d'éthyle. Le mélange réactionnel est porté à reflux pendant 18 heures. Après ce temps, le mélange réactionnel est refroidi à température ambiante, le précipité formé est filtré, rincé avec un minimum d'éthanol puis séché à l'étuve sous pression réduite. On obtient 1,33 g du produit attendu.
R.M.N. ¹H (DMSO-D₆), δ ppm : 8,9 (s, 1H); 8,2 (d, 1H) ; 7,55 (d, 1H) ; 4,3 (q, 2H) ; 1,3 (t, 3H).
LC-MS: 270 ([M+H]+, *R*t= 0,88 min.).

### 21.2 N-[2-(Ethyloxycarbonyl)-imidazo[1,2-b]pyridazin-6-yl]-5-fluoro-1-[(3-fluorophényl)méthyl]-1H-indole-2- carboxamide (composé N° 21)

Le composé N° 21 a été préparé selon un procédé similaire à celui décrit à l'exemple 20 en faisant réagir 0,4 g (1,40 mmole) de 5-fluoro-1-[(3-fluorophényl)méthyl]-1*H-*indole-2-carboxamide préparé selon le protocole décrit à l'étape 1.2 avec 0,41 g (1,54 mmole) de 6-bromo-imidazo[1,2-b]pyridazine-2-carboxylate d'éthyle préparé selon le protocole décrit à l'étape 21.1, en présence de 80 mg (0,42 mmole) d'iodure de cuivre, de 0,38 g (2,79 mmole) de carbonate de potassium et de 53 mg (0,46 mmole) de trans-1,2-cyclohexanediamine dans 10 mL de dioxane. On obtient ainsi 20 mg du produit attendu.
PF : 267 - 268 °C
R.M.N. ¹H (DMSO-D₆), δ ppm : 11,6 (s, 1H) ; 8,7 (s, 1H) ; 8,2 (d, 1H) ; 7,9 (d, 1H) ; 7,65 (m, 3H) ; 7,35 (m, 1 H) ; 7,25 (m, 1 H) ; 7,05 (m, 1 H) ; 6,9 (m, 2H) ; 5,9 (s, 2H) ; 4,35 (q, 2H) ; 1,3 (t, 3H).
LC-MS: 476 ([M+H]+, *R*t= 1,26 min.).

### Exemple 22 (Composé N°22)

### N-[2-(Ethyloxycarbonyl)-imidazo[1,2-a]pyridin-6-yl]-5-fluoro-1-[(3-fluorophényl)méthyl]-1H-indole-2-carboxamide

Le composé N° 22 a été préparé selon un procédé similaire à celui décrit à l'étape 4.3 en faisant réagir 0,1 g (0,26 mmole) de *N*-[6-aminopyridin-3-yl]-5-fluoro-1-[(3-fluorophényl)méthyl]-1*H*-indole-2-carboxamide préparé selon le protocole décrit à l'étape 4.2 avec 0,105 g (0,53 mmole) de 3-bromo-2-oxo-propionate d'ethyle dans 4 mL d'acétonitrile. On obtient 0,05 g du produit attendu sous la forme d'un solide blanc.
PF = 242 - 245 °C
R.M.N. ¹H (DMSO D₆), δ (ppm) : 10,8 (s, 1H) ; 10,55 (s, 1H) ; 8,8 (s, 1H) ; 7,8 (m, 2H) ; 7,65 (m, 2H) ; 7,55 (s, 1 H) ; 7,35 (m, 1 H) ; 7,2 (m, 1H) ; 7,05 (m, 1 H) ; 6,9 (m, 2H) ; 5,9 (s, 2H) ; 4,4 (q, 2H) ; 1,35 (t, 3H).
LC-MS: 475 ([M+H]+, *R*t= 1,15 min.).

### Exemple 23 (Composé N°23)

### N-(2-Méthyl-imidazo[1,2-a]pyridin-6-yl)-5-fluoro-1-[(3-fluorophényl)méthyl]-1H-indole-2-carboxamide

Dans un tube à pression de 10 mL spécifique pour microonde, sont introduits 0,085 g (0,22 mmole) de *N*-[6-aminopyridin-3-yl]-5-fluoro-1-[(3-fluorophényl)méthyl]-1*H*-indole-2-carboxamide préparé selon le protocole décrit à l'étape 4.2 avec 0,043 g (0,45 mmole) de 1-chloro-propan-2-one dans 6 mL d'acétonitrile. Le tube est ensuite scellé et mis à chauffer au micro-onde à 150°C pendant deux cycles de 1 h. Après ce temps, le mélange réactionnel est concentré à sec. Le brut réactionnel est ensuite purifié par chromatographie sur colonne de gel de silice en éluant avec un mélange de dichlorométhane et de méthanol. On obtient ainsi 0,058 g du produit attendu sous la forme d'un solide blanc.
PF : 200 - 203 °C
R.M.N. ¹H (DMSO-D₆), δ ppm : 10,5 (s, 1H) ; 9,2 (s, 1H) ; 7,8 (s, 1H) ; 7,6 (m, 2H) ; 7,45 (m, 2H) ; 7,35 (m, 2H) ; 7,2 (m, 1 H) ; 7,1 (m, 1 H) ; 6, 9 (m, 2H) ; 5,9 (s, 2H) ; 2,3 (s, 3H).
LC-MS: 417 ([M+H]+, *R*t= 1,05 min.).

### Exemple 24 (Composé N°24)

### N-([1,2,4]triazolo[1,5-a]pyridin-6-yl)-5-fluoro-1-[(3-fluorophényl)méthyl]-1H-indole-2-carboxamide

Le composé N° 24 a été préparé selon un procédé similaire à celui décrit à l'exemple 20 en faisant réagir 0,5 g (1,75 mmole) de 5-fluoro-1-[(3-fluorophényl)méthyl]-1*H-*indole-2-carboxamide préparé selon le protocole décrit à l'étape 1.2 avec 0,35 g (1,75 mmole) de 6-bromo-[1,2,4]triazolo[1,5-a]pyridine, en présence de 99 mg (0,52 mmole) d'iodure de cuivre, de 0,48 g (3,49 mmole) de carbonate de potassium et de 66 mg (0,58 mmole) de trans-1,2-cyclohexanediamine dans 15 mL de dioxane. On obtient ainsi 393 mg du produit attendu.
PF : 227 - 229 °C
R.M.N. ¹H (DMSO-D₆), δ ppm : 10,8 (s, 1H) ; 9,55 (s, 1H) ; 8,5 (s, 1H) ; 7,9 (s, 2H) ; 7,60 (m, 2H) ; 7,5 (s, 1 H) ; 7,35 (m, 1 H) ; 7,20 (m, 1H) ; 7,05 (m, 1 H) ; 6,9 (m, 2H) ; 5,9 (s, 2H)
LC-MS: 404 ([M+H]+, *R*t= 1,16 min.).

### Le tableau 1 qui suit illustre les structures chimiques et les propriétés physiques de quelques composés de formule générale (I) selon l'invention.

Dans ce tableau :
- les composés de formule générale (I) sont définis avec n égal à 1 et W = O
- la colonne « PF (°C)» renseigne les points de fusion des produits en degrés Celsius (°C) ;
- les composés sont sous forme de base libre.

**Tableau 1**

| | | | | |
|---|---|---|---|---|
| 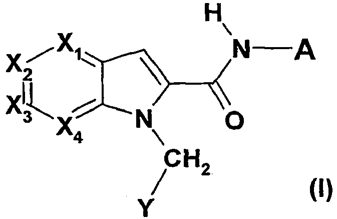 | | | | |

| **N°** | **X₁, X₂, X₃, X₄** | **Y** | **A** | **PF (°C)** |
|---|---|---|---|---|
| 1 | CH, C-F, CH, CH | 3-fluorophényl | 2,3-diméthyl-imidazo[1,2-*a*]pyridin-7-yl | 251 - 253 |
| 2 | CH, C-F, CH, CH | 3-fluorophényl | 2,3-diméthyl-imidazo[1,2-*a*]pyridin-6-yl | 220-242 |
| 3 | CH, C-F, CH, CH | 3-fluorophényl | 2-hydroxyméthyl-imidazo[1,2-*a*]pyridin-6-yl | 247 - 249 |
| 4 | CH, C-F, CH, CH | 3-fluorophényl | 3-méthyl-2-phényl-imidazo[1,2-*a*]pyridin-6-yl | 258 - 259 |
| 5 | CH, C-F, CH, CH | 3-fluorophényl | 2-éthyl-imidazo[1,2-*a*]pyridin-6-yl | 278-279 |
| 6 | CH, C-F, CH, CH | 3-fluorophényl | 2-thién-2-yl-imidazo[1,2-*a*]pyridin-6-yl | 316 - 318 |
| 7 | CH, C-F, CH, CH | 3-fluorophényl | 2-tert-Butyl-imidazo[1,2-*a*]pyridin-6-yl | 190-192 |
| 8 | CH, C-F, CH, CH | 3-fluorophényl | 2-(méthyloxyméthyl)-imidazo[1,2-*a*]pyridin-7-yl | 215-216 |
| 9 | CH, C-F, CH, CH | 3-fluorophényl | 2-(hydroxyméthyl)-imidazo[1,2-*a*]pyridin-7-yl | 244-245 |
| 10 | CH, C-F, CH, CH | 3-fluorophényl | 2-méthyl-3-phényl-imidazo[1,2-*a*]pyridin-7-yl | 305-307 |
| 11 | CH, C-F, CH, CH | 3-fluorophényl | 2-thién-2-yl-imidazo[1,2-*a*]pyridin-7-yl | 259-260 |
| 12 | CH, C-F, CH, CH | 3-fluorophényl | 2-éthyl-imidazo[1,2-*a*]pyridin-7-yl | 158-159 |
| 13 | CH, C-F, CH, CH | 3-fluorophényl | 2-tert-Butyl-imidazo[1,2-*a*]pyridin-7-yl | 327-328 |
| 14 | CH, C-CF₃, CH, CH | 3-fluorophényl | 2,3-diméthyl-imidazo[1,2-*a*]pyridin-7-yl | 280-281 |
| 15 | CH, C-CF₃, CH, N | 3-fluorophényl | 2-éthyl-imidazo[1,2-*a*]pyridin-7-yl | 264-265 |
| 16 | CH, C-CF₃, CH, N | 3-fluorophényl | 2,3-diméthyl-imidazo[1,2-*a*]pyridin-7-yl | 287-288 |
| 17 | CH, C-CF₃, CH, CH | 3-fluorophényl | 2-(hydroxyméthyl)-imidazo[1,2-*a*]pyridin-7-yl | 254-255 |
| 18 | CH, C-CF₃, CH, N | 3-fluorophényl | 2-(hydroxyméthyl)-imidazo[1,2-*a*]pyridin-7-yl | 238-240 |
| 19 | CH, C-F, CH, CH | 3-fluorophényl | 2-méthyl-imidazo[1,2-*a*]pyridin-7-yl | 267-268 |
| 20 | CH, C-F, CH, CH | 3-fluorophényl | 2-Méthyl-pyrazolo[1,5-*a*]pyrimidin-6-yl | 267-268 |
| 21 | CH, C-F, CH, CH | 3-fluorophényl | 2-Ethyloxycarbonyl-imidazo[1,2-b]pyridazin-6-yl | 267-268 |
| 22 | CH, C-F, CH, CH | 3-fluorophényl | 2-(éthyloxycarbonyl)-imidazo[1,2-*a*]pyridin-6-yl | 242-245 |
| 23 | CH, C-F, CH, CH | 3-fluorophényl | 2-méthyl-imidazo[1,2-*a*]pyridin-6-yl | 200-203 |
| 24 | CH, C-F, CH, CH | 3-fluorophényl | [1,2,4]triazolo[1,5-a]pyridin-6-yl | 227-229 |

Les composés selon l'invention ont fait l'objet d'essais pharmacologiques *in vitro* et *in vivo* qui ont mis en évidence leur intérêt comme substances à activités thérapeutiques. Ces composés présentent une activité antagoniste ou agoniste vis-à-vis des récepteurs TRPV1 (ou VR1).

### Test d'inhibition du courant induit par la capsaïcine sur les DRG de rat

- Culture primaire de cellules de ganglions de racine dorsale (DRG) de rat :
   Les neurones du DRG expriment naturellement le récepteur TRPV1.
   Les cultures primaires de DRG de rats nouveaux nés sont préparées à partir de ratons de 1 jour. Brièvement, après dissection, les ganglions sont trypsinés et les cellules dissociées mécaniquement par trituration ménagée. Les cellules sont re-suspendues dans un milieu de culture basal Eagle contenant 10 % de sérum de veau foetal, 25 mM KCI, 2 mM glutamine, 100 µg/mL gentamicine et 50 ng/mL de NGF, puis déposées sur des lamelles de verre recouvertes de laminine (0,25 x 10⁶ cellules par lamelle) qui sont ensuite placées dans des boîtes 12 puits Corning. Les cellules sont incubées à 37°C en atmosphère humidifiée contenant 5% de CO₂ et 95% d'air. De la cytosine β-D-arabinoside (1 µM) est ajoutée 48 h après la mise en culture, pour prévenir le développement des cellules non neuronales. Les lamelles sont transférées dans les chambres expérimentales pour les études de patch-clamp après 7-10 jours de culture.
- Electrophysiologie :
   Les chambres de mesure (volume 800 µl) contenant la préparation cellulaire sont placées sur la platine d'un microscope inversé (Olympus IMT2) équipé d'optiques Hoffman (Modulation Contrast, New York) et observées au grossissement de 400X. Les chambres sont continuellement perfusées par gravité (2,5 mL/min) à l'aide d'un distributeur de solutions acceptant 8 entrées et dont la sortie unique, constituée par un tube de polyéthylène (ouverture 500µm) est placée à moins de 3 mm de la cellule étudiée. La configuration "cellule entière" de la technique de patch-clamp a été utilisée. Les pipettes en verre borosilicaté (résistance 5-10 MOhms) sont approchées de la cellule grâce à un micromanipulateur piézoélectrique 3D (Burleigh, PC1000). Les courants globaux (potentiel de membrane fixé à -60 mV) sont enregistrés avec un amplificateur Axopatch 1 D (Axon Instruments, Foster city, Californie), connecté à un PC piloté par les logiciels de Pclamp8 (Axon Instrument). Les traces de courant sont enregistrées sur papier et simultanément digitalisées (fréquence d'échantillonnage 15 à 25 Hz) et acquises sur le disque dur du PC.
L'application d'une solution de capsaïcine 300 nM provoque sur les cellules de DRG (voltage fixé à -70 mV) un courant cationique entrant. Afin de minimiser la désensibilisation des récepteurs, l'intervalle d'une minute minimum entre deux applications de capsaïcine est respecté. Après une période contrôle (stabilisation de la réponse capsaïcine seule), les composés de l'invention à tester sont appliqués seuls à une concentration donnée (concentration de 10 nM ou de 1 nM) pendant une durée de 4 à 5 minutes, au cours desquelles plusieurs tests « capsaïcine + composé » sont réalisés (obtention de l'inhibition maximale). Les résultats sont exprimés en pourcentage d'inhibition de la réponse capsaïcine contrôle.
Dans le cas des composés antagonistes VR1, les pourcentages d'inhibition de la réponse capsaïcine (1µM) sont compris entre 20% et 100% pour les composés les plus actifs de l'invention testés à des concentrations de 0,1 à 100 nM. Ce sont donc des antagonistes efficaces des récepteurs de type TRPV1. Le tableau 2 donne quelques exemples de pourcentage d'inhibition obtenu avec les composés de l'invention.

**Tableau 2**

| **N° composé** | **% inhibition en patch DRG** |
|---|---|
| **1** | 81,5% (1 nM) |
| **20** | 80% (100nM) |

Les composés de l'invention peuvent donc être utilisés pour la préparation de médicaments, notamment pour la préparation d'un médicament destiné à prévenir ou à traiter les pathologies dans lesquelles les récepteurs de type TRPV1 sont impliqués.

Les composés de l'invention peuvent être utiles pour prévenir ou traiter les pathologies dans lesquelles les récepteurs de type TRPV1 sont impliqués.

Ainsi, l'invention a pour objet des médicaments qui comprennent au moins un composé de formule (I), ou un sel pharmaceutiquement acceptable, ou encore un hydrate ou un solvate dudit composé.
Ces médicaments pourraient trouver leur emploi en thérapeutique, notamment dans la prévention et/ou le traitement de la douleur et de l'inflammation, de la douleur chronique, neuropathique (traumatique, diabétique, métabolique, infectieuse, toxique, induite par un traitement anticancéreux ou iatrogène), (ostéo-) arthritique, rhumatismale, des fibromyalgies, de la douleur du dos, de la douleur liée au cancer, de la névralgie faciale, des céphalées, de la migraine, de la douleur dentaire, de la brûlure, du coup de soleil, de la morsure ou de la piqûre, de la névralgie post-herpétique, de la douleur musculaire, de la compression nerveuse (centrale et/ou périphérique), des traumatismes de la moelle et/ou du cerveau, de l'ischémie (de la moelle et/ou du cerveau), de la neurodégénérescence, des accidents vasculaires hémorragiques (de la moelle et/ou du cerveau), de la douleur post-stroke.
Les composés de l'invention pourraient également être utilisés pour prévenir et/ou traiter les désordres métaboliques tels que le diabète.
Les composés de l'invention pourraient également être utilisés pour prévenir et/ou traiter les désordres urologiques tels que l'hyperactivité de la vessie, l'hyperéflexie vésicale, l'instabilité vésicale, l'incontinence, la miction d'urgence, l'incontinence urinaire, la cystite, la colique néphrétique, l'hypersensibilité pelvienne et la douleur pelvienne.
Les composés de l'invention pourraient être utiles pour prévenir et/ou traiter les désordres gynécologiques comme la vulvodynie, les douleurs liées aux salpingites, aux dysménorrhées.
On pourrait également utiliser ces produits pour prévenir et/ou traiter les désordres gastro-intestinaux tels que le désordre du réflexe gastro-esophagique, l'ulcère de l'estomac, l'ulcère du duodénum, la dyspepsie fonctionnelle, la colite, l'IBS, la maladie de Crohn, la pancréatite, l'oesophagite, la colique hépatique.
De même, les produits de la présente invention pourraient être utiles dans la prévention et/ou le traitement des désordres respiratoires tels que l'asthme, la toux, la maladie pulmonaire obstructive chronique (COPD), la bronchoconstriction et les désordres inflammatoires de la sphère respiratoire.
Ces produits pourraient également être utilisés pour prévenir et/ou traiter le psoriasis, le pruritis, les irritations dermiques, des yeux ou des muqueuses, l'herpès, le zona. Les composés de l'invention pourraient également être utilisés pour traiter la dépression.
Les composés de l'invention pourraient aussi être utilisés pour traiter les maladies du système nerveux central telles que la sclérose en plaques.
Les composés de l'invention pourraient aussi être utilisés pour traiter les cancers.

Selon un autre de ses aspects, la présente invention concerne des compositions pharmaceutiques comprenant, en tant que principe actif, au moins un composé selon l'invention. Ces compositions pharmaceutiques contiennent une dose efficace d'au moins un composé selon l'invention, ou un sel pharmaceutiquement acceptable, un hydrate ou solvate dudit composé, ainsi qu'au moins un excipient pharmaceutiquement acceptable.
Lesdits excipients sont choisis selon la forme pharmaceutique et le mode d'administration souhaité, parmi les excipients habituels qui sont connus de l'homme du métier.

Les compositions pharmaceutiques de la présente invention peuvent être administrées par voie orale, sublinguale, sous-cutanée, intramusculaire, intra-veineuse, topique, locale, intratrachéale, intranasale, transdermique ou rectale. Ces compositions peuvent être administrées sous forme unitaire, en mélange avec des excipients pharmaceutiques classiques. Elles sont destinées à être administrées aux animaux et aux êtres humains pour la prophylaxie ou le traitement des troubles ou des maladies citées ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Lesdites formes unitaires sont dosées pour permettre une administration journalière de 0,001 à 30 mg de principe actif par kg de poids corporel, selon la forme galénique.
Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

## Revendications

1. Composé répondant à la formule générale (I) 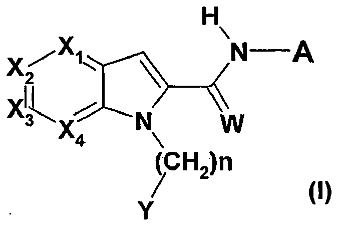 dans laquelle :
X₁, X₂, X₃, et X₄ représentent, indépendamment l'un de l'autre, un atome d'azote ou un groupe C-R₁ ;
étant entendu que quand l'un des X₁, X₂, X₃, et X₄ représente un atome d'azote, les autres correspondent à un groupe C-R₁ ;
W représente un atome d'oxygène ou de soufre ;
n est égal à 0, 1, 2 ou 3 ;
Y représente un aryle ou un hétéroaryle éventuellement substitué par un ou plusieurs groupes choisis parmi un atome d'halogène, un groupe C₁-C₆-alkyle, C₃-C₇-cycloalkyle, C₃-C₇-cycloalkyl-C₁-C₃-alkylène, C₁-C₆-fluoroalkyle, hydroxy, C₁-C₆-alcoxy, C₃-C₇-cycloalkyloxy, C₃-C₇-cycloalkyl-C₁-C₆-alkylène-O-, C₁-C₆-fluoroalcoxy, cyano, C(O) NR₃R₄, nitro, NR₃R₄, C₁-C₆-thioalkyle, thiol, -S(O)-C₁-C₆-alkyle, -S(O)₂-C₁-C₆-alkyle, SO₂NR₃R₄, NR₅C(O)R₆, NR₅SO₂R₇, C(O)NR₃R₄, OC(O)NR₃R₄, -Si-(C₁-C₆-alkyl)₃, -SF₅, aryle-C₁-C₅-alkylène ou aryle, hétéroaryl-C₁-C₅-alkylène ou hétéroaryle ; les groupes C₁-C₆-alkyle, C₃-C₇-cycloalkyle, C₃-C₇-cycloalkyl-C₁-C₃-alkylène, C₁-C₆-fluoroalkyle, C₁-C₆-alcoxy, C₃-C₇-cycloalkyloxy, C₃-C₇-cycloalkyl-C₁-C₆-alkylène-O- pouvant être substitués par un groupe hydroxy, C₁-C₆-alcoxy ou NR₃R₄ ; les groupes aryle et hétéroaryle étant éventuellement substitués par un ou plusieurs substituants R₈ identiques ou différents l'un de l'autre ;
A représente un hétéroaryle bicyclique de formule : 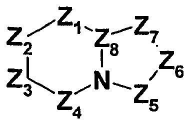 où
Z₁, Z₂, Z₃, et Z₄ représentent, indépendamment l'un de l'autre, un atome de carbone, un atome d'azote ou un groupe C-R₂ₐ ;
Z₅, Z₆ et Z₇ représentent, indépendamment l'un de l'autre, un atome d'azote ou un groupe C-R_{2b} ;
Z₈ représente un atome de carbone ;
trois, au maximum, des Z₁, Z₂, Z₃, Z₄, Z₅, Z₆ et Z₇ représentant un atome d'azote ;
l'un des Z₁, Z₂, Z₃ et Z₄, correspondant à un atome de carbone, étant lié à l'atome d'azote de l'amide ou du thioamide de formule (I) ;
R₁ est choisi parmi un atome d'hydrogène, un atome d'halogène, C₁-C₆-alkyle, C₃-C₇-cycloalkyle, C₃-C₇-cycloalkyl-C₁-C₃-alkylène, C₁-C₆-fluoroalkyle, aryloxy-C₁-C₆-alkyle, hétéroaryloxy-C₁-C₆-alkyle, aryl-C₁-C₃-alkylènoxy-C₁-C₆-alkyle, hétéroaryl-C₁-C₃-alkylènoxy-C₁-C₆-alkyle, arylthio-C₁-C₆-alkyle, hétéroarylthio-C₁-C₆-alkyle, aryl-C₁-C₃-alkylène-thio-C₁-C₆-alkyle, hétéroaryl-C₁-C₃-alkylène-thio-C₁-C₆-alkyle, C₁-C₆-alcoxy, C₃-C₇-cycloalkyloxy, C₃-C₇-cycloalkyl-C₁-C₃-alkylènoxy, C₁-C₆-fluoroalcoxy, cyano, C(O)NR₃R₄, nitro, NR₃R₄, C₁-C₆-thioalkyle, C₃-C₁-cycloalkylthio, C₃-C₇-cycloalkyl-C₁-C₃-alkylène-thio, -S(O)-C₁-C₆-alkyle, -S(O)-C₃-C₇-cycloalkyle, -S(O)-C₁-C₃-alkylène-C₃-C₇-cycloalkyle, C₁-C₆-alkyle-S(O)₂-, C₁-C₆-fluoroalkyle-S(O)₂-, C₃-C₇-cycloalkyl-S(O)₂-, C₃-C₇-cycloalkyl-C₁-C₃-alkylène-S(O)₂-, SO₂NR₃R₄, (C₁-C₆-alkyl)₃-Si-, -SF₅, NR₅C(O)R₆, NR₅SO₂R₇, C(O)NR₃R₄, OC(O)NR₃R₄, aryle, hétéroaryle, aryl-C₁-C₅-alkylène, hétéroaryl-C₁-C₅-alkylène, aryloxy, arylthio, hétéroaryloxy ou hétéroarylthio ; les groupes hétéroaryle ou aryle étant éventuellement substitués par un ou plusieurs substituants R₈, identiques ou différents l'un de l'autre ;
R₂ₐ représente un atome d'hydrogène, un atome d'halogène, un groupe C₁-C₆-alkyle, C₃-C₇-cycloalkyle, C₃-C₇-cycloalkyl-C₁-C₃-alkylène, C₁-C₆-fluoroalkyle, C₁-C₆-alcoxy, C₃-C₇-cycloalkyloxy, C₃-C₇-cycloalkyl-C₁-C₃-alkylène-O-, hydroxy, thiol, C₁-C₆-fluoroalcoxy ;
R_{2b} représente un atome d'hydrogène, un atome d'halogène, un groupe C₁-C₆-alkyle, C₃-C₇-cycloalkyle, C₃-C₇-cycloalkyl-C₁-C₃-alkylène, C₁-C₆-fluoroalkyle, C₁-C₆-alcoxy, hydroxy, thiol, oxo, thio, C₃-C₇-cycloalkyloxy, C₁-C₆-fluoroalcoxy, C₃-C₇-cycloalkyl-C₁-C₃-alkylénoxy, C₁-C₆-alcoxy-C₁-C₃-alkylène, C₃-C₇-cycloalkyloxy-C₁-C₃-alkylène, C₃-C₇-cycloalkyl-C₁-C₃-alkylénoxy-C₁-C₃-alkylène, C₁-C₆-alkyl-C(O)-O-C₁-C₃-alkylène, C₁-C₆-alkyl-C(O)-O-, C₃-C₇-cycloalkyl-C(O)-O-C₁-C₃-alkylène, C₃-C₇-cycloalkyl-C(O)-O-, C₁-C₆-fluoroalkyl-C(O)-O-C₁-C₃-alkylène, C₁-C₆-fluoroalkyl-C(O)-O-, C(O)NR₃R₄, C(O)O-C₁-C₆-alkyle, cyano, CHO, CO₂H, -C(O)-C₁-C₆-alkyle, -C(O)-C₃-C₇-cycloalkyle, phényle ou thiényle ; les groupes C₁-C₆-alkyle, C₃-C₇-cycloalkyle, C₃-C₇-cycloalkyl-C₁-C₃-alkylène, C₁-C₆-fluoroalkyle, C₁-C₆-alcoxy, C₃-C₇-cycloalkyloxy, C₁-C₆-fluoroalcoxy, C₃-C₇-cycloalkyl-C₁-C₃-alkylénoxy, C₁-C₆-alcoxy-C₁-C₃-alkylène, C₃-C₇-cycloalkyloxy-C₁-C₃-alkylène, C₃-C₇-cycloalkyl-C₁-C₃-alkylénoxy-C₁-C₃-alkylène pouvant être substitués par un groupe hydroxy, C₁-C₆-alcoxy ou NR₃R₄ ;
R₃ et R₄ représentent, indépendamment l'un de l'autre, un atome d'hydrogène ou un groupe C₁-C₆-alkyle, C₃-C₇-cycloalkyle, C₃-C₇-cycloalkyl-C₁-C₃-alkylène, aryle-C₁-C₅-alkylène ou aryle, ou R₃ et R₄ forment ensemble, avec l'atome d'azote qui les porte, un groupe azétidine, pyrrolidine, pipéridine, azépine, morpholine, thiomorpholine, pipérazine, homopipérazine; le groupe NR₃R₄ étant éventuellement substitué par un groupe C₁-C₆-alkyle, C₃-C₇-cycloalkyle, C₃-C₇-cycloalkyl-C₁-C₃-alkyléne, aryle-C₁-C₆-alkylène, aryle, hétéroaryle, aryle-S(O)₂-, C₁-C₆-alkyle-S(O)₂-, C₁-C₆-fluoroalkyle-S(O)₂, C₃-C₇-cycloalkyl-S(O)₂-, C₃-C₇-cycloalkyl-C₁-C₃-alkylène-S(O)₂-, aryle-C(O)-, C₁-C₆-alkyle-C(O)-, C₃-C₇-cycloalkyl-C(O)-, C₃-C₇-cycloalkyl-C₁-C₃-alkylène-C(O)-, C₁-C₆-fluoroalkyle-C(O)-, hydroxy, C₁-C₆-alkyloxy, C₃-C₇-cycloalkyloxy, C₃-C₇-cycloalkyl-C₁-C₃-alkylénoxy, C₁-C₆-fluoroalkyle, aryloxy-C₁-C₆-alkylène, aryloxy, hétéroaryloxy-C₁-C₆-alkylène, hétéroaryloxy ;
et R₆ représentent, indépendamment l'un de l'autre, un atome d'hydrogène, un groupe C₁-C₆-alkyle, C₃-C₇-cycloalkyle, C₃-C₇-cycloalkyl-C₁-C₃-alkylène, aryle-C₁-C₆-alkylène ou aryle ; le groupe aryle étant éventuellement substitué par un ou plusieurs substituants choisis parmi un atome d'halogène, un groupe C₁-C₆-alkyle, C₃-C₇-cycloalkyle, C₃-C₇-cycloalkyl-C₁-C₃-alkylène, C₁-C₆-fluoroalkyle, C₁-C₆-alcoxy, C₃-C₇-cycloalkyloxy, C₃-C₇-cycloalkyl-C₁-C₃-alkylénoxy, C₁-C₆-fluoroalcoxy, nitro ou cyano ; ou R₅ et R₆ forment ensemble un lactame de 4 à 7 chaînons comprenant l'atome d'azote et le groupe C(O) qui les portent ;
R₇ représente un groupe C₁-C₆-alkyle, C₃-C₇-cycloalkyle, C₃-C₇-cycloalkyl-C₁-C₃-alkylène, aryle-C₁-C₆-alkylène ou aryle ; le groupe aryle étant éventuellement substitué par un ou plusieurs substituants choisis parmi un atome d'halogène, un groupe C₁-C₆-alkyle, C₃-C₇-cycloalkyle, C₃-C₇-cycloalkyl-C₁-C₃-alkylène, C₁-C₆-fluoroalkyle, C₁-C₆-alcoxy, C₃-C₇-cycloalkyloxy, C₃-C₇-cycloalkyl-C₁-C₃-alkylénoxy, C₁-C₆-fluoroalcoxy, nitro ou cyano ;
ou R₅ et R₇ forment ensemble un sultame de 4 à 7 chaînons comprenant l'atome d'azote et le groupe S(O)₂ qui les portent ;
R₈ représente un atome d'halogène, un groupe C₁-C₆-alkyle, C₃-C₇-cycloalkyle, C₃-C₇-cycloalkyl-C₁-C₃-alkylène, C₁-C₆-fluoroalkyle, C₁-C₆-alcoxy, C₃-C₇-cycloalkyloxy, C₃-C₇-cycloalkyl-C₁-C₃-alkylénoxy, C₁-C₆-fluoroalcoxy, nitro, cyano, NR₃R₄, -C(O)-C₁-C₆-alkyle, -C(O)-C₃-C₇-cycloalkyle ; les groupes C₁-C₆-alkyle, C₃-C₇-cycloalkyle, C₃-C₇-cycloalkyl-C₁-C₃-alkylène, C₁-C₆-fluoroalkyle, C₁-C₆-alcoxy, C₃-C₇-cycloalkyloxy, C₃-C₇-cycloalkyl-C₁-C₃-alkylénoxy, pouvant être substitués par un groupe OH, C₁-C₆-alcoxy ou NR₃R₄ ;
le ou les atomes de soufre du composé de formule générale (I) pouvant être sous forme oxydée ;
le ou les atomes d'azote du composé de formule générale (I) pouvant être sous forme oxydée ;
à l'état de base ou de sel d'addition à un acide, ainsi qu'à l'état d'hydrate ou de solvate.

2. Composé de formule (I) selon la revendication 1, **caractérisé en ce que**
X₁, X₂, X₃, et X₄ représentent, indépendamment l'un de l'autre un groupe C-R₁ ;
R₁ étant tel que défini dans la formule générale (I) selon la revendication 1 ;
à l'état de base ou de sel d'addition à un acide, ainsi qu'à l'état d'hydrate ou de solvate.

3. Composé de formule (I) selon la revendication 1, **caractérisé en ce que**
X₁, X₂, X₃ représentent un groupe C-R₁ ; X₄ représente un atome d'azote ;
R₁ étant tel que défini dans la formule générale (I) selon la revendication 1 ;
à l'état de base ou de sel d'addition à un acide, ainsi qu'à l'état d'hydrate ou de solvate.

4. Composé de formule (I) selon l'une quelconque des revendications 1 à 3, **caractérisé en ce que** R₁ est choisi parmi un atome d'hydrogène, un atome d'halogène ou un groupe C₁-C₆-fluoroalkyle ;
à l'état de base ou de sel d'addition à un acide, ainsi qu'à l'état d'hydrate ou de solvate.

5. Composé de formule (I) selon l'une quelconque des revendications 1 à 4, **caractérisé en ce que** n est égal à 1 ;
à l'état de base ou de sel d'addition à un acide, ainsi qu'à l'état d'hydrate ou de solvate.

6. Composé de formule (1) selon l'une quelconque des revendications 1 à 5, **caractérisé en ce que** Y représente un aryle éventuellement substitué par un ou plusieurs atomes d'halogène ;
à l'état de base ou de sel d'addition à un acide, ainsi qu'à l'état d'hydrate ou de solvate.

7. Composé de formule (I) selon l'une quelconque des revendications 1 à 6, **caractérisé en ce que** W représente un atome d'oxygène ;
à l'état de base ou de sel d'addition à un acide, ainsi qu'à l'état d'hydrate ou de solvate.

8. Composé de formule (I) selon l'une quelconque des revendications 1 à 7, **caractérisé en ce que**
A représente un hétéroaryle bicyclique de formule : 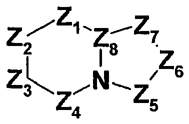 où
Z₁, Z₂, Z₃, et Z₄ représentent, indépendamment l'un de l'autre, un atome de carbone, un atome d'azote ;
Z₅, Z₆ et Z₇ représentent, indépendamment l'un de l'autre, un atome d'azote ou un groupe C-R_{2b} ;
Z₈ représente un atome de carbone ;
deux, au maximum, des Z₁, Z₂, Z₃, Z₄, Z₅, Z₆ et Z₇ représentant un atome d'azote ;
l'un des Z₁, Z₂, Z₃ et Z₄, correspondant à un atome de carbone, étant lié à l'atome d'azote de l'amide ou du thioamide de formule (I) ;
R_{2b} représente un atome d'hydrogène, un groupe C₁-C₆-alkyle, C(O)O-C₁-C₆-alkyle, phényle ou thiényle ; les groupes C₁-C₆-alkyle pouvant être substitués par un groupe hydroxy ou C₁-C₆-alcoxy ;
à l'état de base ou de sel d'addition à un acide, ainsi qu'à l'état d'hydrate ou de solvate.

9. Composé de formule (I) selon l'une quelconque des revendications 1 à 8, **caractérisé en ce que**
A représente le groupe 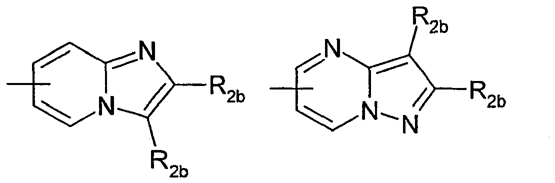 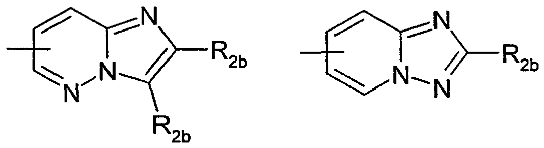
R_{2b} représente un atome d'hydrogène, un groupe C₁-C₆-alkyle, C(O)O-C₁-C₆-alkyle, phényle ou thiényle ; les groupes C₁-C₆-alkyle pouvant être substitués par un groupe hydroxy ou C₁-C₆-alcoxy ;
à l'état de base ou de sel d'addition à un acide, ainsi qu'à l'état d'hydrate ou de solvate.

10. Composé de formule (I) selon l'une quelconque des revendications 1 à 9, **caractérisé en ce que**
A représente le groupe 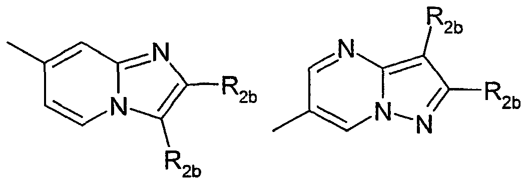
R_{2b} représente un atome d'hydrogène, un groupe C₁-C₆-alkyle, phényle ou thiényle ; les groupes C₁-C₆-alkyle pouvant être substitués par un groupe hydroxy ou C₁-C₆-alcoxy ;
à l'état de base ou de sel d'addition à un acide, ainsi qu'à l'état d'hydrate ou de solvate.

11. Composé de formule (I) selon l'une quelconque des revendications 1 à 8, **caractérisé en ce que**
X₁, X₂, X₃, et X₄ représentent, indépendamment l'un de l'autre un groupe C-R₁ ; ou bien X₁, X₂, X₃ représentent un groupe C-R₁ ; X₄ représente un atome d'azote ;
R₁ est choisi parmi un atome d'hydrogène, un atome d'halogène ou un groupe C₁-C₆-fluoroalkyle ;
n est égal à 1 ;
Y représente un aryle éventuellement substitué par un ou plusieurs atomes d'halogène ;
W représente un atome d'oxygène ;
A représente un hétéroaryle bicyclique de formule : 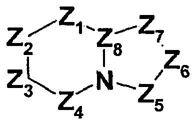 où
Z₁, Z₂, Z₃, et Z₄ représentent, indépendamment l'un de l'autre, un atome de carbone, un atome d'azote ;
Z₅, Z₆ et Z₇ représentent, indépendamment l'un de l'autre, un atome d'azote ou un groupe C-R_{2b} ;
Z₈ représente un atome de carbone ;
deux, au maximum, des Z₁, Z₂, Z₃, Z₄, Z₅, Z₆ et Z₇ représentant un atome d'azote ;
l'un des Z₁, Z₂, Z₃ et Z₄, correspondant à un atome de carbone, étant lié à l'atome d'azote de l'amide ou du thioamide de formule (I) ;
R_{2b} représente un atome d'hydrogène, un groupe C₁-C₆-alkyle, C(O)O-C₁-C₆-alkyle, phényle ou thiényle ; les groupes C₁-C₆-alkyle pouvant être substitués par un groupe hydroxy ou C₁-C₆-alcoxy ;
à l'état de base ou de sel d'addition à un acide, ainsi qu'à l'état d'hydrate ou de solvate.

12. Composé de formule (I) selon la revendication 1, choisi parmi
*N*-(2,3-Diméthyl-imidazo[1,2-*a*]pyridin-7-yl)-5-fluoro-1-[(3-fluorophényl)méthyl]-1*H-*indole-2- carboxamide ;
*N*-(2,3-Diméthyl-imidazo[1,2-*a*]pyridin-6-yl)-5-fluoro-1-[(3-fluorophényl)méthyl]-1*H-*indole-2-carboxamide ;
*N*-[2-(Hydroxyméthyl)-imidazo[1,2-*a*]pyridin-6-yl]-5-fluoro-1-[(3-fluorophényl)méthyl]-1*H*-indole-2-carboxamide ;
*N*-(3-Méthyl-2-phényl-imidazo[1,2-*a*]pyridin-6-yl)-5-fluoro-1-[(3-fluorophényl)méthyl]-1*H-*indole-2-carboxamide ;
*N*-(2-Ethyl-imidazo[1,2-*a*]pyridin-6-yl)-5-fluoro-1-[(3-fluorophényl)méthyl-1*H*-indole-2-carboxamide ;
*N*-[2-(Thién-2-yl)-imidazo[1,2-*a*]pyridin-6-yl]-5-fluoro-1-[(3-fluorophényl)méthyl]-1*H-*indole-2-carboxamide ;
*N*-(2-*tert*-Butyl-imidazo[1,2-*a*]pyridin-6-yl)-5-fluoro-1-[(3-fluorophényl)méthyl]-1*H*-indole-2-carboxamide ;
*N*-(2-Méthoxyméthyl-imidazo[1,2-*a*]pyridin-7-yl)-5-fluoro-1-[(3-fluorophényl)méthyl]-1*H-*indole-2- carboxamide ;
*N*-[2-(Hydroxyméthyl)-imidazo[1,2-*a*]pyridin-7-yl]-5-fluoro-1-[(3-fluorophényl)méthyl]-1*H*-indole-2- carboxamide ;
*N*-(2-Méthyl-3-phényl-imidazo[1,2-*a*]pyridin-7-yl)-5-fluoro-1-[(3-fluorophényl)méthyl]-1*H-*indole-2- carboxamide ;
*N*-[2-(Thién-2-yl)-imidazo[1,2-*a*]pyridin-7-yl]-5-fluoro-1-[(3-fluorophényl)méthyl]-1*H-*indole-2- carboxamide ;
*N*-(2-Ethyl-imidazo[1,2-*a*]pyridin-7-yl)-5-fluoro-1-[(3-fluorophényl)méthyl-1*H*-indole-2-carboxamide ;
*N*-(2-*tert*-Butyl-imidazo[1,2-*a*]pyridin-7-yl)-5-fluoro-1-[(3-fluorophényl)méthyl]-1*H*-indole-2- carboxamide ;
*N*-(2,3-Diméthyl-imidazo[1,2-*a*]pyridin-7-yl)-5-trifluorométhyl-1-[(3-fluorophényl)méthyl]-1*H*-indole-2- carboxamide ;
*N*-(2-Ethyl-imidazo[1,2-*a*]pyridin-7-yl)-5-trifluorométhyl-1-[(3-fluorophényl)méthyl]-1*H-*pyrrolo[2,3-*b*]pyridine-2- carboxamide ;
*N*-(2,3-Diméthyl-imidazo[1,2-*a*]pyridin-7-yl)-5-trifluorométhyl-1-[(3-fluorophényl)méthyl]-*1*H-pyrrolo[2,3-*b*]pyridine-2-carboxamide ;
*N*-[2-(Hydroxyméthyl)-imidazo[1,2-*a*]pyridin-7-yl]-5-trifluorométhyl-1-[(3-fluorophényl)méthyl]-1*H*-indole-2-carboxamide ;
*N*-[2-(Hydroxyméthyl)-imidazo[1,2-*a*]pyridin-7-yl]-5-trifluorométhyl-1-[(3-fluorophényl)méthyl]-1*H*-pyrrolo[2,3-*b*]pyridine-2-carboxamide ;
*N*-(2-Méthyl-imidazo[1,2-a]pyridin-7-yl)-5-fluoro-1-[(3-fluorophényl)méthyl]-1*H*-indole-2-carboxamide ;
*N*-(2-Méthyl-pyrazolo[1,5-*a*]pyrimidin-6-yl)-5-fluoro-1-[(3-fluorophényl)méthyl]-1*H-*indole-2- carboxamide ;
*N*-[2-(Ethyloxycarbonyl)-imidazo[1,2-*b*]pyridazin-6-yl]-5-fluoro-1-[(3-fluorophényl)méthyl]-1*H*-indole-2-carboxamide ;
*N*-[(2-(Ethyloxycarbonyl)-imidazo[1,2-*a*]pyridin-6-yl)-5-fluoro-1-[(3-fluorophényl)méthyl]-*1*H-indole-2-carboxamide ;
*N*-(2-Méthyl-imidazo[1,2-*a*]pyridin-6-yl)-5-fluoro-1-[(3-fluorophényl)méthyl]-1*H*-indole-2-carboxamide ;
*N*-([1,2,4]triazolo[1,5-*a*]pyridin-6-yl)-5-fluoro-1-[(3-fluorophényl)méthyl]-1*H*-indole-2-carboxamide.

13. Procédé de préparation d'un composé de formule (I) selon l'une quelconque des revendications 1 à 12, **caractérisé en ce que** l'on fait réagir un composé de formule générale (II) 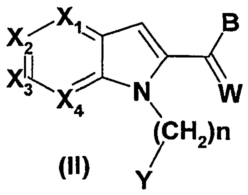 dans laquelle X₁, X₂, X₃, X₄, n, Y et W sont tels que définis dans la formule générale (I) selon la revendication 1,
avec composé de formule générale (III), 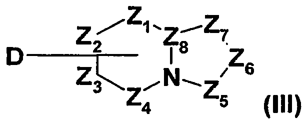 dans laquelle Z₁, Z₂, Z₃, Z₄, Z₅, Z₆, Z₇ et Z₈ sont tels que définis dans la formule générale (I) selon la revendication 1 :
- lorsque B représente un groupe NH₂- et D représente un groupe partant,
soit en présence d'un sel de cuivre en quantité catalytique, d'un ligand du cuivre en quantité catalytique et d'une base, dans un solvant ; soit en présence d'une quantité catalytique d'un dérivé du palladium, d'une quantité catalytique d'un ligand du palladium et d'une base au reflux d'un solvant ;
- lorsque B représente un groupe hydroxyle et D représente un groupe NH₂, en présence d'un agent de couplage dans un solvant ;
- lorsque B est un atome de chlore et D représente un groupe NH₂, dans un solvant.

14. Procédé de préparation d'un composé de formule (I) selon l'une quelconque des revendications 1 à 12, dans laquelle W représente un atome d'oxygène et A représente un groupe : 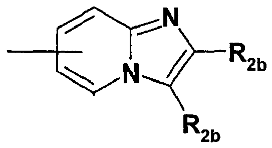 **caractérisé en ce que** l'on fait réagir un composé de formule générale (V) 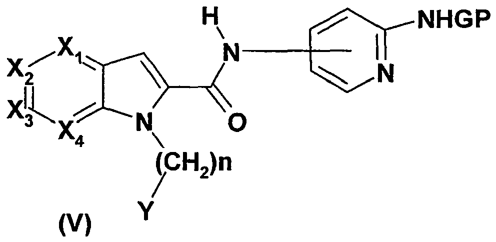 dans laquelle X₁, X₂, X₃, X₄, n et Y sont tels que définis dans la formule générale (I) selon la revendication 1 et GP représente un atome d'hydrogène,
avec un composé de formule générale (IV), 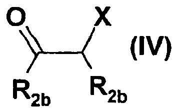 dans laquelle X représente un groupe partant et R_{2b} est tel que défini dans la formule générale (I) selon la revendication 1.

15. Composé de formule (IIIa), (IIIb), (IIIc), (IIId), (IIIe) ou (IIIf) :

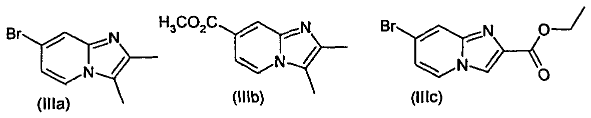

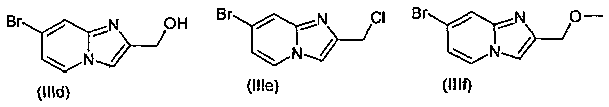

16. Médicament, **caractérisé en ce qu'**il comprend un composé de formule (I) selon l'une quelconque des revendications 1 à 12, ou un sel d'addition de ce composé à un acide pharmaceutiquement acceptable, ou encore un hydrate ou un solvate du composé de formule (I).

17. Composition pharmaceutique, **caractérisée en ce qu'**elle comprend un composé de formule (I) selon l'une quelconque des revendications 1 à 12, ou un sel pharmaceutiquement acceptable, un hydrate ou un solvate de ce composé, ainsi qu'au moins un excipient pharmaceutiquement acceptable.

18. Utilisation d'un composé de formule (I) selon l'une quelconque des revendications 1 à 12 pour la préparation d'un médicament destiné à la prévention et au traitement des pathologies dans lesquelles les récepteurs de type TRPV1 sont impliqués.

19. Utilisation d'un composé de formule (I) selon l'une quelconque des revendications 1 à 12 pour la préparation d'un médicament destiné à prévenir ou à traiter la douleur, l'inflammation, les désordres métaboliques, les désordres urologiques, les désordres gynécologiques, les désordres gastro-intestinaux, les désordres respiratoires, le psoriasis, le pruritis, les irritations dermiques, des yeux ou des muqueuses, l'herpès, le zona, la sclérose en plaques, la dépression, les cancers.

## Patentansprüche

1. Verbindung der allgemeinen Formel (I) 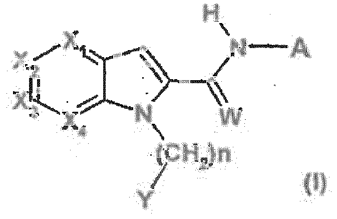 worin:
X₁, X₂, X₃ und X₄ unabhängig voneinander für ein Stickstoffatom oder eine Gruppe C-R₁ stehen;
mit der Maßgabe, dass dann, wenn eine der Variablen X₁, X₂, X₃ und X₄ für ein Stickstoffatom steht, die anderen Variablen einer Gruppe C-R₁ entsprechen;
W für ein Sauerstoff- oder Schwefelatom steht;
n für 0, 1, 2 oder 3 steht;
Y für ein Aryl oder ein Heteroaryl steht, das gegebenenfalls durch eine oder mehrere aus einem Halogenatom oder einer C₁-C₆-Alkyl-, C₃-C₇-Cyclo-alkyl-, C₃-C₇-Cycloalkyl-C₁-C₃-alkylen-, C₁-C₆-Fluoralkyl-, Hydroxy-, C₁-C₆-Alkoxy-, C₃-C₇-Cycloalkyloxy-, C₃-C₇-Cycloalkyl-C₁-C₆-alkylen-O-, C₁-C₆-Fluoralkoxy-, Cyano-, C(O)NR₃R₄-_{,} Nitro-, NR₃R₄-, C₁-C₆-Thioalkyl-, Thiol-, -S(O)-C₁-C₆-Alkyl-, -S(O)₂-C₁-C₆-Alkyl-, SO₂NR₃R₄-, NR₅C(O)R₆-, NR₆SO₂R₇-, C(O)NR₃R₄-, OC(O)NR₃R₄-, -Si(C₁-C₆-Alkyl)₃-, -SF₅-, Aryl-C₁-C₅-alkylen- oder Aryl-, Heteroaryl-C₁-C₅-alkylen- oder Hetroarylgruppe ausgewählte Gruppen substituiert ist; wobei die C₁-C₆-Alkyl-, C₃-C₇-Cycloalkyl-, C₃-C₇-Cycloalkyl-C₁-C₃-alkylen-, C₁-C₆-Fluoroalkyl-, C₁-C₆-Alkoxy-, C₃-C₇-Cycloalkyloxy- und C₃-C₇-Cycloalkyl-C₁-C₆-alkylen-o-Gruppen durch eine Hydroxy-, C₁-C₆-Alkoxy- oder
NR₃R₉-Gruppe substituiert sein können; wobei die Aryl- und Heteroarylgruppen gegebenenfalls durch einen oder mehrere Substituenten R₈, die gleich oder voneinander verschieden sind, substituiert sind;
A für ein bicyclisches Heteroaryl der Formel: 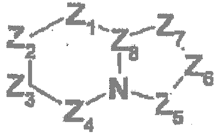 steht, wobei
Z₁, Z₂, Z₃ und Z₄ unabhängig voneinander für ein Kohlenstoffatom, ein Stickstoffatom oder eine Gruppe C-R₂ₐ stehen;
Z₅, Z₆ und Z₇ unabhängig voneinander für ein Stickstoffatom oder eine Gruppe C-R_{2b} stehen;
Z₈ für ein Kohlenstoffatom steht;
höchstens drei der Variablen Z₁, Z₂, Z₃, Z₄, Z₅, Z₆ und Z₇ für ein Stickstoffatom stehen;
wobei eine der Variablen Z₁, Z₂, Z₃ und Z₄, die einem Kohlenstoffatom entspricht, an das Stickstoffatom des Amids bzw. Thioamids der Formel (I) gebunden ist;
R₁ aus einem Wasserstoffatom, einem Halogenatom und einer C₁-C₆-Alkyl-, C₃-C₇-Cycloalkyl-, C₃-C₇-Cycloalkyl-C₁-C₃-alkylen-, C₁-C₆-Fluoralkyl-, Aryloxy-C₁-C₆-alkyl-, Heteroaryloxy-C₁-C₆-alkyl-, Aryl-C₁-C₃-alkylenoxy-C₁-C₆-alkyl-, Heteroaryl-C₁-C₃-alkylenoxy-C₁-C₆-alkyl-, Arylthio-C₁-C₆-alkyl-, Heteroarylthio-C₁-C₆-alkyl-, Aryl-C₁-C₃-alkylenthio-C₁-C₆-alkyl-, Heteroaryl-C₁-C₃-alkylenthio-C₁-C₆-alkyl, C₁-C₆-Alkoxy-, C₃-C₇-Cycloalkyloxy-, C₃-C₇-Cycloalkyl-C₁-C₃-alkylenoxy-, C₁-C₆-Fluoralkoxy-, Cyano-, C(O)NR₃R₄-, Nitro-, NR₃R₄-, C₁-C₆-Thioalkyl-, C₃-C₇-Cycloalkylthio-, C₃-C₇-Cycloalkyl-C₁-C₃-alkylenthio-, -S(O)-C₁-C₆-Alkyl-, - S(O)-C₃-C₇-Cycloalkyl-, -S(O)-C₁-C₃-Alkylen-C₃-C₇-cycloalkyl-, C₁-C₆-Alkyl-S(O)₂-, C₁-C₆-Fluoroalkyl-S(O)₂-, C₃-C₇-Cycloalkyl-S(O)₂-, C₃-C₇-Cycloalkyl-C₁-C₃-alkylen-S(O)₂-, SO₂NR₃R₄-, (C₁-C₆-Alkyl)₃-Si-, - SF₅-, NR₅C(O)R₆-, NR₅SO₂R₇-, C(O)NR₃R₄-, OC(O)NR₃R₄-, Aryl-, Heteroaryl-, Aryl-C₁-C₅-alkylen-, Heteroaryl-C₁-C₅-alkylen-, Aryloxy-, Arylthio-, Heteroaryloxy- oder Heteroarylthiogruppe ausgewählt ist; wobei die Heteroaryl- oder Arylgruppen gegebenenfalls durch einen oder mehrere Substituenten R₈, die gleich oder voneinander verschieden sind, substituiert sind;
R₂ₐ für ein Wasserstoffatom, ein Halogenatom oder eine C₁-C₆-Alkyl-, C₃-C₇-Cycloalkyl-, C₃-C₇-Cycloalkyl-C₁-C₃-alkylen-, C₁-C₆-Fluoralkyl-, C₁-C₆-Alkoxy-, C₃-C₇-Cycloalkyloxy-, C₃-C₇-Cycloalkyl-C₁-C₃-alkylen-O-, Hydroxy-, Thiol- oder C₁-C₆-Fluoralkoxygruppe steht;
R_{2b} für ein Wasserstoffatom, ein Halogenatom oder eine C₁-C₆-Alkyl-, C₃-C₇-Cycloalkyl-, C₃-C₇-Cycloalkyl-C₁-C₃-alkylen-, C₁-C₆-Fluoralkyl-, C₁-C₆-Alkoxy-, Hydroxy-, Thiol-, Oxo-, Thio-, C₃-C₇-Cycloalkyloxy-, C₁-C₆-Fluoralkoxy-, C₃-C₇-Cycloalkyl-C₁-C₃-alkylenoxy-, C₁-C₆-Alkoxy-C₁-C₃-alkylen-, C₃-C₇-Cycloalkyloxy-C₁-C₃-alkylen-, C₃-C₇-Cycloalkyl-C₁-C₃-alkylenoxy-C₁-C₃-alkylen-, C₁-C₆-Alkyl-C(O)-O-C₁-C₃-alkylen-, C₁-C₆-Alkyl-C(O)-O-, C₃-C₇-Cycloalkyl-C(O)-O-C₁-C₃-alkylen-, C₃-C₇-Cycloalkyl-C(O)-O-, C₁-C₆-Fluoralkyl-C(O)-O-C₁-C₃-alkylen-, C₁-C₆-Fluoralkyl-C(O)-O-, C(O)NR₃R₄-, C(O)O-C₁-C₆-Alkyl-, Cyano-, CHO-, CO₂H-, -C(O)-C₁-C₆-Alkyl-, -C(O)-C₃-C₇-Cycloalkyl-, Phenyl- oder Thienylgruppe steht; wobei die C₁-C₆-Alkyl-, C₃-C₇-Cycloalkyl-, C₃-C₇-Cycloalkyl-C₁-C₃-alkylen-, C₁-C₆-Fluoralkyl-, C₁-C₆-Alkoxy-, C₃-C₇-Cycloalkyl-oxy-, C₁-C₆-Fluoralkoxy-, C₃-C₇-Cycloalkyl-C₁-C₃-alkylenoxy-, C₁-C₆-Alkoxy-C₁-C₃-alkylen-, C₃-C₇-Cycloalkyloxy-C₁-C₃-alkylen- und C₃-C₇-Cycloalkyl-C₁-C₃-alkylenoxy-C₁-C₃-alkylengruppen durch eine Hydroxy-, C₁-C₆-Alkoxy- oder NR₃R₄-Gruppe substituiert sein können;
R₃ und R₄ unabhängig voneinander für ein Wasserstoffatom oder eine C₁-C₆-Alkyl-, C₃-C₇-Cycloalkyl-, C₃-C₇-Cycloalkyl-C₁-C₃-alkylen-, Aryl-C₁-C₅-alkylen-oder Arylgruppe stehen oder R₃ und R₄ zusammen mit dem Stickstoffatom, das sie trägt, eine Azetidin-, Pyrrolidin-, Piperidin-, Azepin-, Morpholin-, Thiomorpholin-, Piperazin- oder Homopiperazingruppe bilden; wobei die Gruppe NR₃R₄ gegebenenfalls durch eine C₁-C₆-Alkyl-, C₃-C₇-Cycloalkyl-, C₃-C₇-Cycloalkyl-C₁-C₃-alkylen-, Aryl-C₁-C₅-alkylen-, Aryl-, Heteroaryl-, Aryl-S(O)₂-, C₁-C₆-Alkyl-S(O)₂-, C₁-C₆-Fluoralkyl-S(O)₂-, C₃-C₇-Cycloalkyl-S(O)₂-, C₃-C₇-Cycloalkyl-C₁-C₃-alkylen-S(O)₂-, Aryl-C(O)-, C₁-C₆-Alkyl-C(O)-, C₃-C₇-Cycloalkyl-C(O)-, C₃-C₇-Cycloalkyl-C₁-C₃-alkylen-C(O)-, C₁-C₆-Fluoralkyl-C(O)-, Hydroxy-, C₁-C₆-Alkyloxy-, C₃-C₇-Cycloalkyloxy-, C₃-C₇-Cycloalkyl-C₁-C₃-alkylenoxy-, C₁-C₆-Fluoralkyl-, Aryloxy-C₁-C₆-alkylen-, Aryloxy-, Heteroaryloxy-C₁-C₆-alkylen- oder Heteroaryloxygruppe substituiert ist;
R₅ und R₆ unabhängig voneinander für ein Wasserstoffatom oder eine C₁-C₆-Alkyl-, C₃-C₇-Cycloalkyl-, C₃-C₇-Cycloalkyl-C₁-C₃-alkylen-, Aryl-C₁-C₆-alkylen- oder Arylgruppe stehen; wobei die Arylgruppe gegebenenfalls durch einen oder mehrere aus einem Halogenatom und einer C₁-C₆-Alkyl-, C₃-C₇-Cycloalkyl-, C₃-C₇-Cycloalkyl-C₁-C₃-alkylen-, C₁-C₆-Fluoralkyl-, C₁-C₆-Alkoxy-, C₃-C₇-Cycloalkyloxy-, C₃-C₇-Cycloalkyl-C₁-C₃-alkylenoxy-, C₁-C₆-Fluoralkoxy-, Nitro- oder Cyanogruppe ausgewählte Substituenten substituiert ist;
oder R₅ und R₆ zusammen ein 4- bis 7-gliedriges Lactam bilden, das das Stickstoffatom und die C(O)-Gruppe, die sie tragen, umfasst;
R₇ für eine C₁-C₆-Alkyl-, C₃-C₇-Cycloalkyl-, C₃-C₇-Cycloalkyl-C₁-C₃-alkylen-, Aryl-C₁-C₆-alkylen- oder Arylgruppe steht; wobei die Arylgruppe gegebenenfalls durch einen oder mehrere aus einem Halogenatom und einer C₁-C₆-Alkyl-, C₃-C₇-Cycloalkyl-, C₃-C₇-Cycloalkyl-C₁-C₃-alkylen-, C₁-C₆-Fluoralkyl-, C₁-C₆-Alkoxy-, C₃-C₇-Cycloalkyloxy-, C₃-C₇-Cycloalkyl-C₁-C₃-alkylenoxy-, C₁-C₆-Fluoralkoxy-, Nitro- oder Cyanogruppe ausgewählte Substituenten substituiert sind;
oder R₅ und R₇ zusammen ein 4- bis 7-gliedriges Sultam bilden, das das Stickstoffatom und die S(O)₂-Gruppe, die sie tragen, umfasst;
R₈ für ein Halogenatom oder eine C₁-C₆-Alkyl-, C₃-C₇-Cycloalkyl-, C₃-C₇-Cycloalkyl-C₁-C₃-alkylen-, C₁-C₆-Fluoralkyl-, C₁-C₆-Alkoxy-, C₃-C₇-Cycloalkyloxy-, C₃-C₇-Cycloalkyl-C₁-C₃-alkylenoxy-, C₁-C₆-Fluoralkoxy-, Nitro-, Cyano-, NR₃R₄-, -C(O)-C₁-C₆-Alkyl- oder C(O)-C3-C7-Cycloalkylgruppe steht; wobei die C₁-C₆-Alkyl-, C₃-C₇-Cycloalkyl-, C₃-C₇-Cycloalkyl-C₁-C₃-alkylen-, C₁-C₆-Fluoralkyl-, C₁-C₆-Alkoxy-, C₃-C₇-Cycloalkyloxy- und C₃-C₇-Cycloalkyl-C₁-C₃-alkylenoxygruppen durch eine Hydroxy-, C₁-C₆-Alkoxy- oder NR₃R₄-Gruppe substituiert sein können;
wobei das Schwefelatom bzw. die Schwefelatome der Verbindungen der allgemeinen Formel (I) in oxidierter Form vorliegen kann bzw. können;
wobei das Stickstoffatom bzw. die Stickstoffatome der Verbindungen der allgemeinen Formel (I) in oxidierter Form vorliegen kann bzw. können;
in Basen- oder Säureadditionssalzform sowie in Hydrat- oder Solvatform.

2. Verbindung der Formel (I) nach Anspruch 1, **dadurch gekennzeichnet, dass**
X₁, X₂, X₃ und X₄ unabhängig voneinander für eine Gruppe C-R₁ stehen;
wobei R₁ wie in der allgemeinen Formel (I) nach Anspruch 1 definiert ist;
in Basen- oder Säureadditionssalzform sowie in Hydrat- oder Solvatform.

3. Verbindung der Formel (I) nach Anspruch 1, **dadurch gekennzeichnet, dass**
X₁, X₂ und X₃ für eine Gruppe C-R₁ stehen; X₄ für ein Stickstoffatom steht;
wobei R₁ wie in der allgemeinen Formel (I) nach Anspruch 1 definiert ist;
in Basen- oder Säureadditionssalzform sowie in Hydrat- oder Solvatform.

4. Verbindung der Formel (I) nach einem der Ansprüche 1 bis 3, **dadurch gekennzeichnet, dass** R₁ aus einem Wasserstoffatom, einem Halogenatom oder einer C₁-C₆-Fluoralkylgruppe ausgewählt ist;
in Basen- oder Säureadditionssalzform sowie in Hydrat- oder Solvatform.

5. Verbindung der Formel (I) nach einem der Ansprüche 1 bis 4, **dadurch gekennzeichnet, dass** n gleich 1 ist;
in Basen- oder Säureadditionssalzform sowie in Hydrat- oder Solvatform.

6. Verbindung der Formel (I) nach einem der Ansprüche 1 bis 5, **dadurch gekennzeichnet, dass** Y für ein Aryl, das gegebenenfalls durch ein oder mehrere Halogenatome substituiert ist, steht;
in Basen- oder Säureadditionssalzform sowie in Hydrat- oder Solvatform.

7. Verbindung der Formel (I) nach einem der Ansprüche 1 bis 6, **dadurch gekennzeichnet, dass** W für ein Sauerstoffatom steht;
in Basen- oder Säureadditionssalzform sowie in Hydrat- oder Solvatform.

8. Verbindung der Formel (I) nach einem der Ansprüche 1 bis 7, **dadurch gekennzeichnet, dass** A für ein bicyclisches Heteroaryl der Formel: 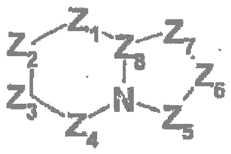 steht, wobei
Z₁, Z₂, Z₃ und Z₄ unabhängig voneinander für ein Kohlenstoffatom oder ein Stickstoffatom stehen;
Z₅, Z₆ und Z₇ unabhängig voneinander für ein Stickstoffatom oder eine Gruppe C-R_{2b} stehen;
Z₈ für ein Kohlenstoffatom steht;
höchstens zwei der Variablen Z₁, Z₂, Z₃, Z₄, Z₅, Z₆ und Z₇ für ein Stickstoffatom stehen;
wobei eine der Variablen Z₁, Z₂, Z₃ und Z₄, die einem Kohlenstoffatom entspricht, an das Stickstoffatom des Amids bzw. Thioamids der Formel (I) gebunden ist;
R_{2b} für ein Wasserstoffatom oder eine C₁-C₆-Alkyl-, -C(O)O-C₁-C₆-Alkyl-, Phenyl- oder Thienylgruppe steht; wobei die C₁-C₆-Alkylgruppen durch eine Hydroxy- oder C₁-C₆-Alkoxygruppe substituiert sein können;
in Basen- oder Säureadditionssalzform sowie in Hydrat- oder Solvatform.

9. Verbindung der Formel (I) nach einem der Ansprüche 1 bis 8, **dadurch gekennzeichnet, dass**
A für die Gruppe 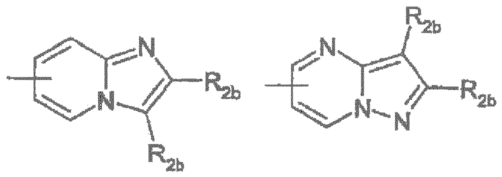 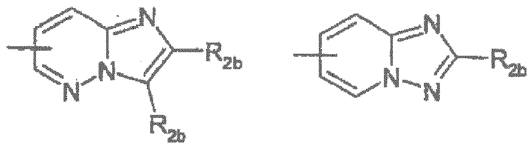 steht,
R_{2b} für ein Wasserstoffatom oder eine C₁-C₆-Alkyl-, -C (O) O-C₁-C₆-Alkyl-, Phenyl- oder Thienylgruppe steht; wobei die C₁-C₆-Alkylgruppen durch eine Hydroxy- oder C₁-C₆-Alkoxygruppe substituiert sein können;
in Basen- oder Säureadditionssalzform sowie in Hydrat- oder Solvatform.

10. Verbindung der Formel (I) nach einem der Ansprüche 1 bis 9, **dadurch gekennzeichnet, dass**
A für die Gruppe 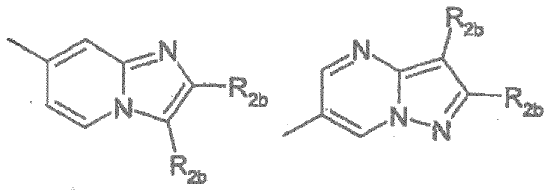 steht,
R_{2b} für ein Wasserstoffatom oder eine C₁-C₆-Alkyl-, Phenyl- oder Thienylgruppe steht; wobei die C₁-C₆-Alkylgruppen durch eine Hydroxy- oder C₁-C₆-Alkoxygruppe substituiert sein können;
in Basen- oder Säureadditionssalzform sowie in Hydrat- oder Solvatform.

11. Verbindung der Formel (I) nach einem der Ansprüche 1 bis 8, **dadurch gekennzeichnet, dass**
X₁, X₂, X₃ und X₄ unabhängig voneinander für eine Gruppe C-R₁ stehen; oder auch X₁, X₂ und X₃ für eine Gruppe C-R₁ stehen; X₄ für ein Stickstoffatom steht;
R₁ aus einem Wasserstoffatom, einem Halogenatom oder einer C₁-C₆-Fluoralkylgruppe ausgewählt ist;
n gleich 1 ist;
Y für ein Aryl, das gegebenenfalls durch ein oder mehrere Halogenatome substituiert ist, steht;
W für ein Sauerstoffatom steht;
A für ein bicyclisches Heteroaryl der Formel: 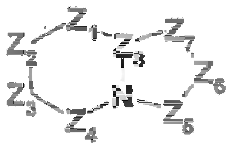 steht, wobei
Z₁, Z₂, Z₃ und Z₄ unabhängig voneinander für ein Kohlenstoffatom oder ein Stickstoffatom stehen;
Z₅, Z₆ und Z₇ unabhängig voneinander für ein Stickstoffatom oder eine Gruppe C-R_{2b} stehen;
Z₈ für ein Kohlenstoffatom steht;
höchstens zwei der Variablen Z₁, Z₂, Z₃, Z₄, Z₅, Z₆ und Z₇ für ein Stickstoffatom stehen;
wobei eine der Variablen Z₁, Z₂, Z₃ und Z₄, die einem Kohlenstoffatom entspricht, an das Stickstoffatom des Amids bzw. Thioamids der Formel (I) gebunden ist;
R_{2b} für ein Wasserstoffatom oder eine C₁-C₆-Alkyl-, -C(O)O-C₁-C₆-Alkyl-, Phenyl- oder Thienylgruppe steht; wobei die C₁-C₆-Alkylgruppen durch eine Hydroxy- oder C₁-C₆-Alkoxygruppe substituiert sein können;
in Basen- oder Säureadditionssalzform sowie in Hydrat- oder Solvatform.

12. Verbindung der Formel (I) nach Anspruch 1, ausgewählt aus
*N*-(2,3-Dimethylimidazo[1,2-*a*]pyridin-7-yl)-5-fluor-1-[(3-fluorphenyl)methyl]-1*H*-indol-2-carboxamid;
*N*-(2,3-Dimethylimidazo[1,2-*a*]pyridin-6-yl)-5-fluor-1-[(3-fluorphenyl)methyl]-1*H*-indol-2-carboxamid;
*N*-[2-(Hydroxymethyl)imidazo[1,2-*a*]pyridin-6-yl]-5-fluor-1-[(3-fluorphenyl)methyl]-1*H*-indol-2-carboxamid;
*N*-(3-Methyl-2-phenylimidazo[1,2-*a*]pyridin-6-yl)-5-fluor-1-[(3-fluorphenyl)methyl]-1*H*-indol-2-carboxamid;
*N*-(2-Ethylimidazo[1,2-*a*]pyridin-6-yl)-5-fluor-1-[(3-fluorphenyl)methyl-1*H*-indol-2-carboxamid;
*N*-[2-(Thien-2-yl)imidazo[1,2-*a*]pyridin-6-yl]-5-fluor-1-[(3-fluorphenyl)methyl]-1*H*-indol-2-carboxamid;
*N*-(2-tert.-Butylimidazo[1,2-*a*]pyridin-6-yl)-5-fluor-1-[(3-fluorphenyl)methyl]-1*H*-indol-2-carboxamid;
*N*-(2-Methoxymethylimidazo[1,2-*a*]pyridin-7-yl)-5-fluor-1-[(3-fluorphenyl)methyl]-1*H*-indol-2-carboxamid;
*N*-[2-(Hydroxymethyl)imidazo[1,2-*a*]pyridin-7-yl]-5-fluor-1-[(3-fluorphenyl)methyl]-1*H*-indol-2-carboxamid;
*N*-(2-Methyl-3-phenylimidazo[1,2-*a*]pyridin-7-yl)-5-fluor-1-[(3-fluorphenyl)methyl]-1*H*-indol-2-carboxamid;
*N*-[2-(Thien-2-yl)imidazo[1,2-*a*]pyridin-7-yl]-5-fluor-1-[(3-fluorphenyl)methyl]-1*H*-indol-2-carboxamid;
*N*-(2-Ethylimidazo[1,2-*a*]pyridin-7-yl)-5-fluor-1-[(3-fluorphenyl)methyl-1*H*-indol-2-carboxamid;
*N*-(2-*tert*.-Butylimidazo[1,2-*a*]pyridin-7-yl)-5-fluor-1-[(3-fluorphenyl)methyl]-1*H*-indol-2-carboxamid;
*N*-(2,3-Dimethylimidazo[1,2-*a*]pyridin-7-yl)-5-trifluormethyl-1-[(3-fluorphenyl)methyl]-1*H*-indol-2-carboxamid;
*N*-(2-Ethylimidazo[1,2-*a*]pyridin-7-yl)-5-trifluormethyl-1-[(3-fluorphenyl)methyl]-1*H-*pyrrolo[2,3-b]pyridin-2-carboxamid;
*N*-(2,3-Dimethylimidazo[1,2-*a*]pyridin-7-yl)-5-trifluormethyl-1-[(3-fluorphenyl)methyl]-1*H-*pyrrolo[2,3-b]pyridin-2-carboxamid;
*N*-[2-(Hydroxymethyl)imidazo[1,2-*a*]pyridin-7-yl]-5-trifluormethyl-1-[(3-fluorphenyl)methyl]-1*H*-indol-2-carboxamid;
*N*-[2-(Hydroxymethyl)imidazo[1,2-*a*]pyridin-7-yl]-5-trifluormethyl-1-[(3-fluorphenyl)methyl]-1*H-*pyrrolo[2,3-b]pyridin-2-carboxamid;
*N*-(2-Methylimidazo[1,2-*a*]pyridin-7-yl)-5-fluor-1-[(3-fluorphenyl)methyl]-1*H*-indol-2-carboxamid;
*N*-(2-Methylpyrazolo[1,5-*a*]pyrimidin-6-yl)-5-fluor-1-[(3-fluorphenyl)methyl]-1*H*-indol-2-carboxamid;
*N*-[2-(Ethyloxycarbonyl)imidazo[1,2-*b*]pyridazin-5-yl]-5-fluor-1-[(3-fluorphenyl)methyl]-1*H*-indol-2-carboxamid;
*N*-[(2-(Ethyloxycarbonyl)imidazo[1,2-*a*]pyridin-6-yl)-5-fluor-1-[(3-fluorphenyl)methyl]-1*H*-indol-2-carboxamid;
*N*-(2-Methylimidazo[1,2-*a*]pyridin-6-yl)-5-fluor-1-[(3-fluorphenyl)methyl]-1*H*-indol-2-carboxamid;
*N*-([1,2,4]Triazolo[1,5-*a*]pyridin-6-yl)-5-fluor-1-[(3-fluorphenyl)methyl]-1*H*-indol-2-carboxamid.

13. Verfahren zur Herstellung einer Verbindung der Formel (I) nach einem der Ansprüche 1 bis 12, **dadurch gekennzeichnet, dass** man eine Verbindung der allgemeinen Formel (II) 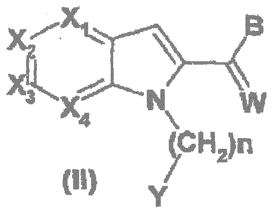 worin X₁, X₂, X₃, X₄, n, Y und W wie in der allgemeinen Formel (I) nach Anspruch 1 definiert sind,
mit einer Verbindung der allgemeinen Formel (III) 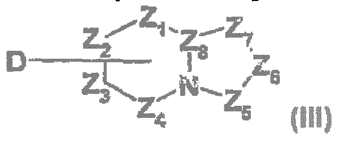 worin Z₁, Z₂, Z₃, Z₄, Z₅, Z₆, Z₇ und Z₈ wie in der allgemeinen Formel (I) nach Anspruch 1 definiert sind, umsetzt, und zwar dann,
- wenn B für eine NH₂-Gruppe steht und D für eine Abgangsgruppe steht,
entweder in Gegenwart eines Kupfersalzes in katalytisch wirksamer Menge, eines Kupferliganden in katalytisch wirksamer Menge und einer Base in einem Lösungsmittel oder in Gegenwart einer katalytisch wirksamen Menge eines Palladiumderivats, einer katalytisch wirksamen Menge eines Palladiumliganden und einer Base unter Rückfluss eines Lösungsmittels;
- wenn B für eine Hydroxylgruppe steht und D für eine NH₂-Gruppe steht, in Gegenwart eines Kupplungsmittels in einem Lösungsmittel;
- wenn B für ein Chloratom steht und D für eine NH₂-Gruppe steht, in einem Lösungsmittel.

14. Verfahren zur Herstellung einer Verbindung der Formel (I) nach einem der Ansprüche 1 bis 12, worin W für ein Sauerstoffatom steht und A für eine Gruppe: 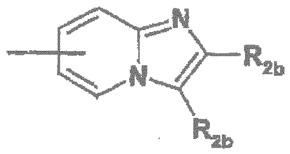 steht, **dadurch gekennzeichnet, dass** man eine Verbindung der allgemeinen Formel (V) 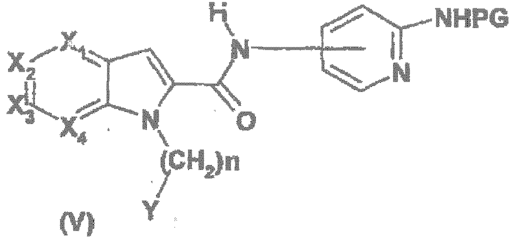 worin X₁, X₂, X₃, X₄, n und Y wie in der allgemeinen Formel (I) nach Anspruch 1 definiert sind und PG für ein Wasserstoffatom steht,
mit einer Verbindung der allgemeinen Formel (IV) 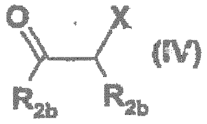 worin X für eine Abgangsgruppe steht und R_{2b} wie in der allgemeinen Formel (I) nach Anspruch 1 definiert ist,
umsetzt.

15. Verbindung der Formel (IIIa), (IIIb), (IIIc), (IIId), (IIIe) oder (IIIf):

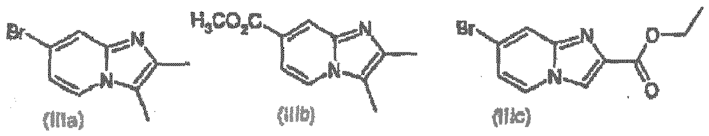

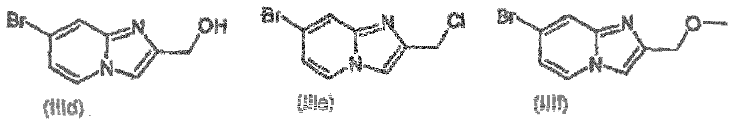

16. Arzneimittel, **dadurch gekennzeichnet, dass** es eine Verbindung der Formel (I) nach einem der Ansprüche 1 bis 12 oder ein Additionssalz dieser Verbindung mit einer pharmazeutisch unbedenklichen Säure oder auch ein Hydrat oder ein Solvat der Verbindung der Formel (I) umfasst.

17. Pharmazeutische Zusammensetzung, **dadurch gekennzeichnet, dass** sie eine Verbindung der Formel (I) nach einem der Ansprüche 1 bis 12 oder ein pharmazeutisch unbedenkliches Salz oder auch ein Hydrat oder ein Solvat dieser Verbindung sowie mindestens einen pharmazeutisch unbedenklichen Hilfsstoff umfasst.

18. Verwendung einer Verbindung der Formel (I) nach einem der Ansprüche 1 bis 12 zur Herstellung eines Arzneimittels zur Prävention oder Behandlung von Pathologien, an denen Rezeptoren vom TRPV1-Typ beteiligt sind.

19. Verwendung einer Zusammensetzung der Formel (I) nach einem der Ansprüche 1 bis 12 zur Herstellung eines Arzneimittels zur Prävention oder Behandlung von Schmerzen, Entzündungen, Stoffwechselstörungen, urologischen Störungen, gynäkologischen Störungen, gastrointestinalen Störungen, respiratorischen Störungen, Psoriasis, Pruritis, Haut-, Augen- oder Schleimhautreizungen, Herpes, Zona, Multipler Sklerose, Depression und Krebserkrankungen.

## Claims

1. Compound corresponding to the general formula (I) 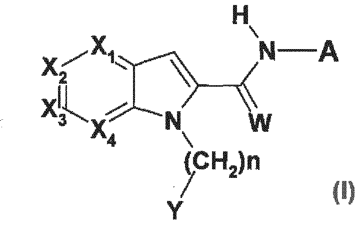 in which:
X₁, X₂, X₃ and X₄ represent, independently of each other, a nitrogen atom or a group C-R₁;
it being understood that when one from among X₁, X₂, X₃ and X₄ represents a nitrogen atom, the others correspond to a group C-R₁;
W represents an oxygen or sulfur atom;
n is equal to 0, 1, 2 or 3;
Y represents an aryl or a heteroaryl optionally substituted with one or more groups chosen from a halogen atom and a group C₁-C₆-alkyl, C₃-C₇-cycloalkyl, C₃-C₇-cycloalkyl-C₁-C₃-alkylene, C₁-C₆-fluoroalkyl, hydroxyl, C₁-C₆-alkoxy, C₃-C₇-cycloalkyloxy, C₃-C₇-cycloalkyl-C₁-C₆-alkylene-O-, C₁-C₆-fluoroalkoxy, cyano, C(O)NR₃R₄, nitro, NR₃R₄, C₁-C₆-thioalkyl, thiol, -S(O)-C₁-C₆-alkyl, -S(O)₂-C₁-C₆-alkyl, SO₂NR₃R₄, NR₅C(O)R₆, NR₅SO₂R₇, C(O)NR₃R₄, OC(O)NR₃R₄, -Si-(C₁-C₆-alkyl)₃, -SF₅, aryl-C₁-C₅-alkylene or aryl, heteroaryl-C₁-C₅-alkylene or heteroaryl; the groups C₁-C₆-alkyl, C₃-C₇-cycloalkyl, C₃-C₇-cycloalkyl-C₁-C₃-alkylene, C₁-C₆-fluoroalkyl, C₁-C₆-alkoxy, C₃-C₇-cycloalkyloxy and C₃-C₇-cycloalkyl-C₁-C₆-alkylene-O- possibly being substituted with a hydroxyl, C₁-C₆-alkoxy or NR₃R₄ group; the aryl and heteroaryl groups being optionally substituted with one or more substituents R₈, which may be identical to or different from each other;
A represents a bicyclic heteroaryl of formula: 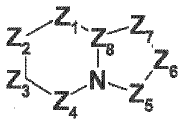 in which
Z₁, Z₂, Z₃ and Z₄ represent, independently of each other, a carbon atom, a nitrogen atom or a group C-R₂ₐ;
Z₅, Z₆ and Z₇ represent, independently of each other, a nitrogen atom or a group C-R_{2b};
Z₈ represents a carbon atom;
three, at most, from among Z₁, Z₂, Z₃, Z₄, Z₅, Z₆ and Z₇ represent a nitrogen atom;
one from among Z₁, Z₂, Z₃ and Z₄, corresponding to a carbon atom, being bonded to the nitrogen atom of the amide or thioamide of formula (I);
R₁ is chosen from a hydrogen atom, a halogen atom, C₁-C₆-alkyl, C₃-C₇-cycloalkyl, C₃-C₇-cycloalkyl-C₁-C₃-alkylene, C₁-C₆-fluoroalkyl, aryloxy-C₁-C₆-alkyl, heteroaryloxy-C₁-C₆-alkyl, aryl-C₁₋C₃-alkylenoxy-C₁-C₆-alkyl, heteroaryl-C₁-C₃-alkylenoxy-C₁-C₆-alkyl, arylthio-C₁-C₆-alkyl, heteroarylthio-C₁-C₆-alkyl, aryl-C₁-C₃-alkylenethio-C₁-C₆-alkyl, heteroaryl-C₁-C₃-alkylenethio-C₁-C₆-alkyl, C₁-C₆-alkoxy, C₃-C₇-cycloalkyloxy, C₃-C₇-cycloalkyl-C₁-C₃-alkylenoxy, C₁-C₆-fluoroalkoxy, cyano, C(O)NR₃R₄, nitro, NR₃R₄, C₁-C₆-thioalkyl, C₃-C₇-cycloalkylthio, C₃-C₇-cycloalkyl-C₁-C₃-alkylenethio, - S(O)-C₁-C₆-alkyl, -S(O)-C₃-C₇-cycloalkyl, -S(O)-C₁-C₃-alkylene-C₃-C₇-cycloalkyl, C₁-C₆-alkyl-S(O)₂-, C₁-C₆-fluoroalkyl-S(O)₂-, C₃-C₇-cycloalkyl-S(O)₂-, C₃-C₇-cycloalkyl-C₁-C₃-alkylene-S(O)₂-, SO₂NR₃R₄, (C₁-C₆-alkyl)₃-Si-, -SF₅, NR₅C(O)R₆, NR₅SO₂R₇, C(O)NR₃R₄, OC(O)NR₃R₄, aryl, heteroaryl, aryl-C₁-C₅-alkylene, heteroaryl-C₁-C₅-alkylene, aryloxy, arylthio, heteroaryloxy or heteroarylthio; the heteroaryl or aryl groups being optionally substituted with one or more substituents R₈, which may be identical to or different from each other;
R₂ₐ represents a hydrogen atom, a halogen atom or a group C₁-C₆-alkyl, C₃-C₇-cycloalkyl, C₃-C₇-cycloalkyl-C₁-C₃-alkylene, C₁-C₆-fluoroalkyl, C₁-C₆-alkoxy, C₃-C₇-cycloalkyloxy, C₃-C₇-cycloalkyl-C₁-C₃-alkylene-O-, hydroxyl, thiol or C₁-C₆-fluoroalkoxy;
R_{2b} represents a hydrogen atom, a halogen atom or a group C₁-C₆-alkyl, C₃-C₇-cycloalkyl, C₃-C₇-cycloalkyl-C₁-C₃-alkylene, C₁-C₆-fluoroalkyl, C₁-C₆-alkoxy, hydroxyl, thiol, oxo, thio, C₃-C₇-cycloalkyloxy, C₁-C₆-fluoroalkoxy, C₃-C₇-cycloalkyl-C₁-C₃-alkylenoxy, C₁-C₆-alkoxy-C₁-C₃-alkylene, C₃-C₇-cycloalkyloxy-C₁-C₃-alkylene, C₃-C₇-cycloalkyl-C₁-C₃-alkylenoxy-C₁-C₃-alkylene, C₁-C₆-alkyl-C(O)-O-C₁-C₃-alkylene, C₁-C₆-alkyl-C(O)-O-, C₃-C₇-cycloalkyl-C(O) -O-C₁-C₃-alkylene, C₃-C₇-cycloalkyl-C(O)-O-, C₁-C₆-fluoroalkyl-C(O)-O-C₁-C₃-alkylene, C₁-C₆-fluoroalkyl-C(O)-O-, C(O)NR₃R₄, C(O)O-C₁-C₆-alkyl, cyano, CHO, CO₂H, -C(O)-C₁-C₆-alkyl, -C(O)-C₃-C₇-cycloalkyl, phenyl or thienyl; the groups C₁-C₆-alkyl, C₃-C₇-cycloalkyl, C₃-C₇-cycloalkyl-C₁-C₃-alkylene, C₁-C₆-fluoroalkyl, C₁-C₆-alkoxy, C₃-C₇-cycloalkyloxy, C₁-C₆-fluoroalkoxy, C₃-C₇-cycloalkyl-C₁-C₃-alkylenoxy, C₁-C₆-alkoxy-C₁-C₃-alkylene, C₃-C₇-cycloalkyloxy-C₁-C₃-alkylene and C₃-C₇-cycloalkyl-C₁-C₃-alkylenoxy-C₁-C₃-alkylene possibly being substituted with a hydroxyl, C₁-C₆-alkoxy or NR₃R₄ group;
R₃ and R₄ represent, independently of each other, a hydrogen atom or a group C₁-C₆-alkyl, C₃-C₇-cycloalkyl, C₃-C₇-cycloalkyl-C₁-C₃-alkylene, aryl-C₁-C₅-alkylene or aryl, or R₃ and R₄ together form, with the nitrogen atom that bears them, an azetidine, pyrrolidine, piperidine, azepine, morpholine, thiomorpholine, piperazine or homopiperazine group; the group NR₃R₄ being optionally substituted with a group C₁-C₆-alkyl, C₃-C₇-cycloalkyl, C₃-C₇-cycloalkyl-C₁-C₃-alkylene, aryl-C₁-C₆-alkylene, aryl, heteroaryl, aryl-S(O)₂-, C₁-C₆-alkyl-S(O)₂-, C₁-C₆-fluoroalkyl-S(O)₂, C₃-C₇-cycloalkyl-S(O)₂-, C₃-C₇-cycloalkyl-C₁-C₃-alkylene-S(O)₂-, aryl-C(O)-, C₁-C₆-alkyl-C(O)-, C₃-C₇-cycloalkyl-C(O)-, C₃-C₇-cycloalkyl-C₁-C₃-alkylene-C(O)-, C₁-C₆-fluoroalkyl-C(O)-, hydroxyl, C₁-C₆-alkyloxy, C₃-C₇-cycloalkyloxy, C₃-C₇-cycloalkyl-C₁-C₃-alkylenoxy, C₁-C₆-fluoroalkyl, aryloxy-C₁-C₆-alkylene, aryloxy, heteroaryloxy-C₁-C₆-alkylene or heteroaryloxy;
R₅ and R₆ represent, independently of each other, a hydrogen atom or a group C₁-C₆-alkyl, C₃-C₇-cycloalkyl, C₃-C₇-cycloalkyl-C₁-C₃-alkylene, aryl-C₁-C₆-alkylene or aryl; the aryl group being optionally substituted with one or more substituents chosen from a halogen atom and a group C₁-C₆-alkyl, C₃-C₇-cycloalkyl, C₃-C₇-cycloalkyl-C₁-C₃-alkylene, C₁-C₆-fluoroalkyl, C₁-C₆-alkoxy, C₃-C₇-cycloalkyloxy, C₃-C₇-cycloalkyl-C₁-C₃-alkylenoxy, C₁-C₆-fluoroalkoxy, nitro or cyano;
or R₅ and R₆ together form a 4- to 7-membered lactam comprising the nitrogen atom and the C(O) group that bear them;
R₇ represents a group C₁-C₆-alkyl, C₃-C₇-cycloalkyl, C₃-C₇-cycloalkyl-C₁-C₃-alkylene, aryl-C₁-C₆-alkylene or aryl; the aryl group being optionally substituted with one or more substituents chosen from a halogen atom and a group C₁-C₆-alkyl, C₃-C₇-cycloalkyl, C₃-C₇-cycloalkyl-C₁-C₃-alkylene, C₁-C₆-fluoroalkyl, C₁-C₆-alkoxy, C₃-C₇-cycloalkyloxy, C₃-C₇-cycloalkyl-C₁-C₃-alkylenoxy, C₁-C₆-fluoroalkoxy, nitro or cyano;
or R₅ and R₇ together form a 4- to 7-membered sultam comprising the nitrogen atom and the S(O)₂ group that bear them;
R₈ represents a halogen atom or a group C₁-C₆-alkyl, C₃-C₇-cycloalkyl, C₃-C₇-cycloalkyl-C₁-C₃-alkylene, C₁-C₆-fluoroalkyl, C₁-C₆-alkoxy, C₃-C₇-cycloalkyloxy, C₃-C₇-cycloalkyl-C₁-C₃-alkylenoxy, C₁-C₆-fluoroalkoxy, nitro, cyano, NR₃R₄, -C(O)-C₁-C₆-alkyl or -C(O)-C₃-C₇-cycloalkyl; the groups C₁-C₆-alkyl, C₃-C₇-cycloalkyl, C₃-C₇-cycloalkyl-C₁-C₃-alkylene, C₁-C₆-fluoroalkyl, C₁-C₆-alkoxy, C₃-C₇-cycloalkyloxy and C₃-C₇-cycloalkyl-C₁-C₃-alkylenoxy possibly being substituted with a group OH, C₁-C₆-alkoxy or NR₃R₄;
the sulfur atom(s) of the compound of general formula (I) possibly being in oxidized form;
the nitrogen atom(s) of the compound of general formula (I) possibly being in oxidized form;
in the form of the base or of an acid-addition salt, and also in the form of hydrate or solvate.

2. Compound of formula (I) according to Claim 1, **characterized in that**
X₁, X₂, X₃ and X₄ represent, independently of each other, a group C-R₁;
R₁ being as defined in the general formula (I) according to Claim 1;
in the form of the base or of an acid-addition salt, and also in the form of hydrate or solvate.

3. Compound of formula (I) according to Claim 1, **characterized in that**
X₁, X₂ and X₃ represent a group C-R₁; X₄ represents a nitrogen atom;
R₁ being as defined in the general formula (I) according to Claim 1;
in the form of the base or of an acid-addition salt, and also in the form of hydrate or solvate.

4. Compound of formula (I) according to any one of Claims 1 to 3, **characterized in that** R₁ is chosen from a hydrogen atom, a halogen atom and a group C₁-C₆-fluoroalkyl;
in the form of the base or of an acid-addition salt, and also in the form of hydrate or solvate.

5. Compound of formula (I) according to any one of Claims 1 to 4, **characterized in that** n is equal to 1; in the form of the base or of an acid-addition salt, and also in the form of hydrate or solvate.

6. Compound of formula (I) according to any one of Claims 1 to 5, **characterized in that** Y represents an aryl optionally substituted with one or more halogen atoms;
in the form of the base or of an acid-addition salt, and also in the form of hydrate or solvate.

7. Compound of formula (I) according to any one of Claims 1 to 6, **characterized in that** W represents an oxygen atom;
in the form of the base or of an acid-addition salt, and also in the form of hydrate or solvate.

8. Compound of formula (I) according to any one of Claims 1 to 7, **characterized in that**
A represents a bicyclic heteroaryl of formula: 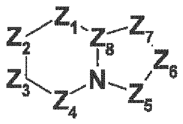 in which
Z₁, Z₂, Z₃ and Z₄ represent, independently of each other, a carbon atom or a nitrogen atom;
Z₅, Z₆ and Z₇ represent, independently of each other, a nitrogen atom or a group C-R_{2b};
Z₈ represents a carbon atom;
two, at most, from among Z₁, Z₂, Z₃, Z₄, Z₅, Z₆ and Z₇ represent a nitrogen atom;
one from among Z₁, Z₂, Z₃ and Z₄, corresponding to a carbon atom, being bonded to the nitrogen atom of the amide or thioamide of formula (I);
R_{2b} represents a hydrogen atom or a group C₁-C₆-alkyl, C(O)O-C₁-C₆-alkyl, phenyl or thienyl; the groups C₁-C₆-alkyl possibly being substituted with a hydroxyl or C₁-C₆-alkoxy group;
in the form of the base or of an acid-addition salt, and also in the form of hydrate or solvate.

9. Compound of formula (I) according to any one of Claims 1 to 8, **characterized in that**
A represents the group 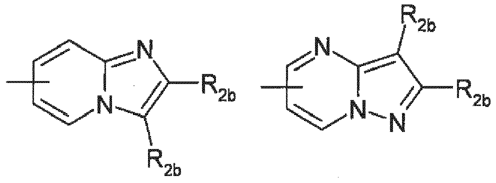 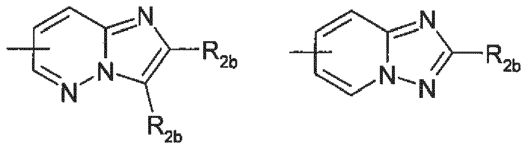 R_{2b} represents a hydrogen atom or a group C₁-C₆-alkyl, C(O)O-C₁-C₆-alkyl, phenyl or thienyl; the groups C₁-C₆-alkyl possibly being substituted with a hydroxyl or C₁-C₆-alkoxy group;
in the form of the base or of an acid-addition salt, and also in the form of hydrate or solvate.

10. Compound of formula (I) according to any one of Claims 1 to 9, **characterized in that**
A represents the group 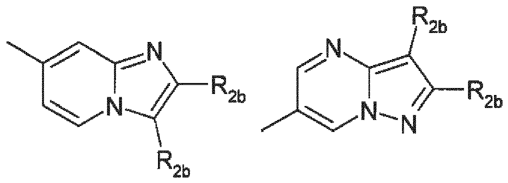 R_{2b} represents a hydrogen atom or a group C₁-C₆-alkyl, phenyl or thienyl; the groups C₁-C₆-alkyl possibly being substituted with a hydroxyl or C₁-C₆-alkoxy group;
in the form of the base or of an acid-addition salt, and also in the form of hydrate or solvate.

11. Compound of formula (I) according to any one of Claims 1 to 8, **characterized in that**
X₁, X₂, X₃ and X₄ represent, independently of each other, a group C-R₁; or alternatively X₁, X₂ and X₃ represent a group C-R₁; X₄ represents a nitrogen atom;
R₁ is chosen from a hydrogen atom, a halogen atom or a group C₁-C₆-fluoroalkyl;
n is equal to 1;
Y represents an aryl optionally substituted with one or more halogen atoms;
W represents an oxygen atom;
A represents a bicyclic heteroaryl of formula: 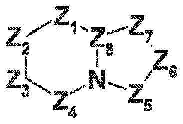 in which
Z₁, Z₂, Z₃ and Z₄ represent, independently of each other, a carbon atom or a nitrogen atom;
Z₅, Z₆ and Z₇ represent, independently of each other, a nitrogen atom or a group C-R_{2b};
Z₈ represents a carbon atom;
two, at most, from among Z₁, Z₂, Z₃, Z₄, Z₅, Z₆ and Z₇ represent a nitrogen atom;
one from among Z₁, Z₂, Z₃ and Z₄, corresponding to a carbon atom, being bonded to the nitrogen atom of the amide or thioamide of formula (I) ;
R_{2b} represents a hydrogen atom or a group C₁-C₆-alkyl, C(O)O-C₁-C₆-alkyl, phenyl or thienyl; the groups C₁-C₆-alkyl possibly being substituted with a hydroxyl or C₁-C₆-alkoxy group;
in the form of the base or of an acid-addition salt, and also in the form of hydrate or solvate.

12. Compound of formula (I) according to Claim 1, chosen from:
*N*-(2,3-Dimethylimidazo[1,2-*a*]pyrid-7-yl)-5-fluoro-1-[(3-fluorophenyl)methyl]-1*H*-indole-2-carboxamide;
*N*-(2,3-Dimethylimidazo[1,2-*a*]pyrid-6-yl)-5-fluoro-1-[(3-fluorophenyl)methyl]-1*H*-indole-2-carboxamide;
*N*-[2-(Hydroxymethyl)imidazo[1,2-*a*]pyrid-6-yl]-5-fluoro-1-[(3-fluorophenyl)methyl]-1*H*-indole-2-carboxamide;
*N*-(3-Methyl-2-phenylimidazo[1,2-*a*]pyrid-6-yl)-5-fluoro-1-[(3-fluorophenyl)methyl]-1*H*-indole-2-carboxamide;
*N*-(2-Ethylimidazo[1,2-*a*]pyrid-6-yl)-5-fluoro-1-[(3-fluorophenyl)methyl-1*H*-indole-2-carboxamide;
*N*-[2-(Thien-2-yl)imidazo[1,2-*a*]pyrid-6-yl]-5-fluoro-1-[(3-fluorophenyl)methyl]-1*H*-indole-2-carboxamide;
*N*-(2-tert-Butylimidazo[1,2-*a*]pyrid-6-yl)-5-fluoro-1-[(3-fluorophenyl)methyl]-1*H*-indole-2-carboxamide;
*N*-(2-Methoxymethylimidazo[1,2-*a*]pyrid-7-yl)-5-fluoro-1-[(3-fluorophenyl)methyl]-1*H*-indole-2-carboxamide;
*N*-[2-(Hydroxymethyl)imidazo[1,2-*a*]pyrid-7-yl]-5-fluoro-1-[(3-fluorophenyl)methyl]-1*H*-indole-2-carboxamide;
*N*-(2-Methyl-3-phenylimidazo[1,2-*a*]pyrid-7-yl)-5-fluoro-1-[(3-fluorophenyl)methyl]-1*H*-indole-2-carboxamide;
*N*-[2-(Thien-2-yl)imidazo[1,2-*a*]pyrid-7-yl]-5-fluoro-1-[(3-fluorophenyl)methyl]-1*H*-indole-2-carboxamide;
*N*-(2-Ethylimidazo[1,2-*a*]pyrid-7-yl)-5-fluoro-1-[(3-fluorophenyl)methyl-1*H*-indole-2-carboxamide;
*N*-(2-*tert*-Butylimidazo[1,2-*a*]pyrid-7-yl)-5-fluoro-1-[(3-fluorophenyl)methyl]-1*H*-indole-2-carboxamide;
*N*-(2,3-Dimethylimidazo[1,2-*a*]pyrid-7-yl)-5-trifluoromethyl-1-[(3-fluorophenyl)methyl]-1*H*-indole-2-carboxamide;
*N*-(2-Ethylimidazo[1,2-*a*]pyrid-7-yl)-5-trifluoromethyl-1-[(3-fluorophenyl)methyl]-1*H*-pyrrolo[2,3-b]pyridine-2-carboxamide;
*N*-(2,3-Dimethylimidazo[1,2-*a*]pyrid-7-yl)-5-trifluoromethyl-1-[(3-fluorophenyl)methyl]-1*H-*pyrrolo[2,3-b]pyridine-2-carboxamide;
*N*-[2-(Hydroxymethyl) imidazo[1,2-*a*]pyrid-7-yl]-5-trifluoromethyl-1-[(3-fluorophenyl)methyl]-1*H*-indole-2-carboxamide;
*N*-[2-(Hydroxymethyl)imidazo[1,2-*a*]pyrid-7-yl]-5-trifluoromethyl-1-[(3-fluorophenyl)methyl]-1*H-*pyrrolo(2,3-b]pyridine-2-carboxamide;
*N*-(2-Methylimidazo[1,2-*a*]pyrid-7-yl)-5-fluoro-1-[(3-fluorophenyl)methyl]-1*H*-indole-2-carboxamide;
*N*-(2-Methylpyrazolo[1,5-*a*]pyrimidin-6-yl)-5-fluoro-1-[(3-fluorophenyl)methyl]-1*H*-indole-2-carboxamide;
*N*-[2-(Ethyloxycarbonyl)imidazo[1,2-*b*]pyridazin-6-yl]-5-fluoro-1-[(3-fluorophenyl)methyl]-2*H*-indole-2-carboxamide;
*N*-[(2-(Ethyloxycarbonyl)imidazo[1,2-*a*]pyrid-6-yl)-5-fluoro-1-[(3-fluorophenyl)methyl]-1*H*-indole-2-carboxamide;
*N*-(2-Methylimidazo[1,2-*a*]pyrid-6-yl)-5-fluoro-1-[(3-fluorophenyl)methyl]-1*H*-indole-2-carboxamide;
*N*-([1,2,4]Triazolo[1,5-a]pyrid-6-yl)-5-fluoro-1-[(3-fluorophenyl)methyl]-1*H*-indole-2-carboxamide.

13. Process for preparing a compound of formula (I) according to any one of Claims 1 to 12, **characterized in that** a compound of general formula (II) 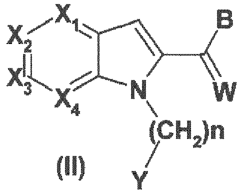 in which X₁, X₂, X₃, X₄, n, Y and W are as defined in the general formula (I) according to Claim 1,
is reacted with a compound of general formula (III), 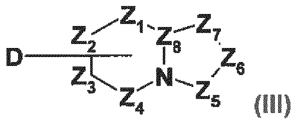 in which Z₁, Z₂, Z₃, Z₄, Z₅, Z₆, Z₇ and Z₈ are as defined in the general formula (I) according to Claim 1:
- when B represents an NH₂ group and D represents a leaving group,
either in the presence of a copper salt in catalytic amount, a copper ligand in catalytic amount and a base, in a solvent; or in the presence of a catalytic amount of a palladium derivative, a catalytic amount of a palladium ligand and a base, in a refluxing solvent;
- when B represents a hydroxyl group and D represents an NH₂ group, in the presence of a coupling agent in a solvent;
- when B is a chlorine atom and D represents an NH₂ group, in a solvent.

14. Process for preparing a compound of formula (I) according to any one of Claims 1 to 12, in which W represents an oxygen atom and A represents a group: 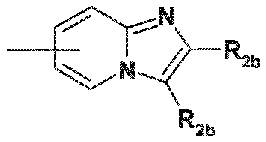 **characterized in that** a compound of general formula (V) 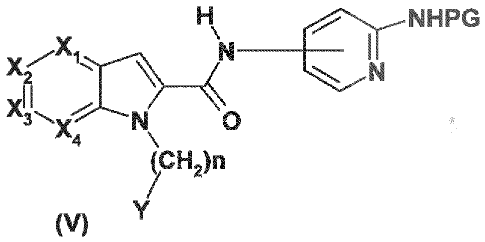 in which X₁, X₂, X₃, X₄, n and Y are as defined in the general formula (I) according to Claim 1 and PG represents a hydrogen atom,
is reacted with a compound of general formula (IV), 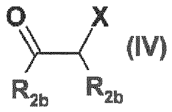 in which X represents a leaving group and R_{2b} is as defined in the general formula (I) according to Claim 1.

15. Compound of formula (IIIa), (IIIb), (IIIc), (IIId), (IIIe) or (IIIf):

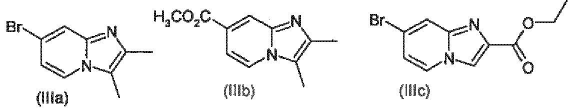

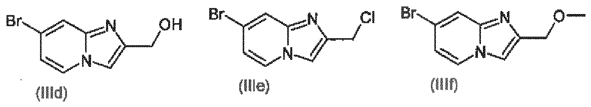

16. Medicament, **characterized in that** it comprises a compound of formula (I) according to any one of Claims 1 to 12, or an addition salt of this compound with a pharmaceutically acceptable acid, or alternatively a hydrate or a solvate of the compound of formula (I).

17. Pharmaceutical composition, **characterized in that** it comprises a compound of formula (I) according to any one of Claims 1 to 12, or a pharmaceutically acceptable salt, a hydrate or a solvate of this compound, and also at least one pharmaceutically acceptable excipient.

18. Use of a compound of formula (I) according to any one of Claims 1 to 12, for the preparation of a medicament for presenting or treating pathologies in which receptors of TRPV1 type are involved.

19. Use of a compound of formula (I) according to any one of Claims 1 to 12, for the preparation of a medicament for preventing or treating pain, inflammation, metabolic disorders, urological disorders, gynaecological disorders, gastrointestinal disorders, respiratory disorders, psoriasis, pruritus, dermal, ocular or mucous irritations, herpes, zona, multiple sclerosis, depression and cancers.
